(19) Europäisches Patentamt
European Patent Office
Office européen des brevets

(11) EP 1 478 357 B1

(12) **EUROPEAN PATENT SPECIFICATION**

(45) Date of publication and mention of the grant of the patent:
**31.01.2007 Bulletin 2007/05**

(21) Application number: **03714744.4**

(22) Date of filing: **18.02.2003**

(51) Int Cl.:
***A61K 31/415*** *(2006.01)* ***C07D 487/04*** *(2006.01)*
***A61P 25/28*** *(2006.01)* ***A61P 31/12*** *(2006.01)*
***A61P 35/00*** *(2006.01)*

(86) International application number:
**PCT/EP2003/001594**

(87) International publication number:
**WO 2003/070236 (28.08.2003 Gazette 2003/35)**

(54) **TRICYCLIC PYRAZOLE DERIVATIVES, PROCESS FOR THEIR PREPARATION AND THEIR USE AS ANTITUMOR AGENTS**

TRIZYKLISCHE PYRAZOLDERIVATE, VERFAHREN ZU IHRER HERSTELLUNG UND IHRE VERWENDUNG ALS ANTITUMOR MITTEL

DERIVES DE PYRAZOLE TRICYCLIQUE, PROCEDES DE LEUR PREPARATION ET LEUR UTILISATION EN TANT QU'AGENTS ANTI-TUMORAUX

(84) Designated Contracting States:
**AT BE BG CH CY CZ DE DK EE ES FI FR GB GR HU IE IT LI LU MC NL PT SE SI SK TR**

(30) Priority: **19.02.2002 US 357918 P**

(43) Date of publication of application:
**24.11.2004 Bulletin 2004/48**

(73) Proprietor: **Pfizer Italia S.r.l.**
**04010 Latina (IT)**

(72) Inventors:
- **D'ALESSIO, Roberto**
  **I-20092 Cinisello Balsamo (IT)**
- **BARGIOTTI, Alberto**
  **I-20146 Milano (IT)**
- **BRASCA, Maria, Gabriella**
  **I-20090 Cusago (IT)**
- **ERMOLI, Antonella**
  **I-20094 Buccinasco (IT)**
- **PEVARELLO, Paolo**
  **I-27100 Pavia (IT)**
- **TIBOLLA, Marcellino**
  **I-20030 Senago (IT)**

(74) Representative: **Modiano, Micaela Nadia et al**
**Modiano Josif Pisanty & Staub Ltd**
**Thierschstrasse 11**
**80538 München (DE)**

(56) References cited:
**EP-A- 0 276 833** **EP-A- 0 276 834**
**WO-A-00/27822** **WO-A-00/59901**
**WO-A-01/87846** **WO-A-99/55335**

- **STRAKOVA I A ET AL: "SYNTHESIS AND REACTIONS OF 1-(2-PYRIDYL)-3-METHYL-4-CHLORO-5-FORMYL-6 ,7-DIHYDROINDAZOLES" CHEMISTRY OF HETEROCYCLIC COMPOUNDS (A TRANSLATION OF KHIMIYA GETEROTSIKLICHESKIKH SOEDINENII), PLENUM PRESS CO., NEW YORK, NY, US, vol. 34, no. 6, 1998, pages 669-673, XP009012872 ISSN: 0009-3122**
- **FRIEDMAN S G ET AL: "PYRAZOLOBENZOTHIAZOLES AND THEIR TRANSFORMATION INTO CYANINE DYES" HIMIA GETEROCIKLICESKIH SOEDINENIJ, LATVIJSKIJ INSTITUT ORGANICESKOGO SINTEZA, RIGA,, LV, no. 3, 1967, pages 481-485, XP009012868 ISSN: 0132-6244**
- **STRAKOVA I A ET AL: "2-AMINO-6-PHENYL-7,8-DIHYDROINDAZOLOÄ4,5- DÜTHIAZOLES" HIMIA GETEROCIKLICESKIH SOEDINENIJ, LATVIJSKIJ INSTITUT ORGANICESKOGO SINTEZA, RIGA,, LV, no. 4, 1996, pages 497-500, XP009012871 ISSN: 0132-6244**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

- STRAKOVA Y ET AL: "REACTION OF 4-CHLORO- AND 4-ETHOXY-1-PHENYL-3-METHYL-5-FORMYL-6,7-DI H YDROINDAZOLES WITH SOME NUCLEOPHILIC REAGE" AKADEMIYA NAUK LATVIISKOI S.S.R. IZVESTIYA. SERIYA KHIMICHESKAYA, IZDEVEJS ZINATNE TURGENEVA, RIGA, LV, no. 2, 1976, pages 234-235, XP009012870 ISSN: 0002-3248
- CAGNOLI BELLAVITA N ET AL: "THIOCYANATION OF AMINOINDAZOLES: NEW CONDENSED HETEROCYCLICS WITH A CENTRAL BENZO RING" ANNALI DI CHIMICA, SOCIETA CHIMICA ITALIANA, ROME, IT, vol. 58, no. 8/9, 1968, pages 823-837, XP009012875 ISSN: 0003-4592
- STRAKOVA I A ET AL: "REACTION OF 1-PHENYL-3-METHYL-4-OXO-4,5,6,7-TETRAHYDRO INDAZOLE FORMYLATION PRODUCTS WITH NUCLEOPHILIC REAGENTS" AKADEMIYA NAUK LATVIISKOI S.S.R. IZVESTIYA. SERIYA KHIMICHESKAYA, IZDEVEJS ZINATNE TURGENEVA, RIGA, LV, no. 5, 1974, pages 610-614, XP009012869 ISSN: 0002-3248
- STRAKOVA I A ET AL: "REACTIONS OF 3-METHYL-1-(2-PYRIDYL)-4-CHLORO-5-FORMYL-6 ,7-DIHYDROINDAZOL" CHEMISTRY OF HETEROCYCLIC COMPOUNDS (A TRANSLATION OF KHIMIYA GETEROTSIKLICHESKIKH SOEDINENII), PLENUM PRESS CO., NEW YORK, NY, US, vol. 34, no. 9, 1998, pages 1036-1040, XP009012873 ISSN: 0009-3122
- MULLOCK E B ET AL: "SYNTHESES OF HETEROCYCLIC COMPOUNDS. PART XXI. OXAZOLES FROM PYROLYSIS OF ARYL AND HETEROCYCLIC AZIDES IN A MIXTURE OF ACETIC AND POLYPHOSPHORIC ACID" JOURNAL OF THE CHEMICAL SOCIETY, SECTION C: ORGANIC CHEMISTRY, CHEMICAL SOCIETY. LETCHWORTH, GB, vol. 15, 1968, pages 1937-1940, XP009012874 ISSN: 0022-4952

**Description**

## BACKGROUND OF THE INVENTION

### Field of the invention

The present invention relates to tricyclic pyrazole derivatives active as kinase inhibitors and, more in particular, it relates to tricyclic pyrazoles and analogues tricyclic heterocyclic derivatives, to a process for their preparation, to pharmaceutical compositions comprising them and to their use for manufacturing therapeutic agents, particularly useful in the treatment of diseases linked to disregulated protein kinases.

### Discussion of background

The malfunctioning of protein kinases (PKs) is the hallmark of numerous diseases. A large share of the oncogenes and proto-oncogenes involved in human cancers code for PKs. The enhanced activities of PKs are also implicated in many non-malignant diseases, such as benign prostate hyperplasia, familial adenomatosis, polyposis, neuro-fibromatosis, psoriasis, vascular smooth cell proliferation associated with atherosclerosis, pulmonary fibrosis, arthritis glomerulonephritis and post-surgical stenosis and restenosis. PKs are also implicated in inflammatory conditions and in the multiplication of viruses and parasites. PKs may also play a major role in the pathogenesis and development of neurodegenerative disorders.
For a general reference to PKs malfunctioning or disregulation see, for instance, Current Opinion in Chemical Biology 1999, 3, 459 - 465.

## SUMMARY OF THE INVENTION

It is an object of the invention to provide compounds which are useful in therapy as agents against a host of diseases caused by and/or associated to a disregulated protein kinase activity.
It is another object to provide compounds which are endowed with multiple protein kinase inhibiting activity.
The present inventors have now discovered that the compounds of the invention, hereinafter shortly referred to as tricyclic pyrazole derivatives, are endowed with multiple protein kinase inhibiting activity and are thus useful in therapy in the treatment of diseases associated with disregulated protein kinases.
More specifically, the compounds of this invention are useful in the treatment of a variety of cancers including, but not limited to: carcinoma such as bladder, breast, colon, kidney, liver, lung, including small cell lung cancer, esophagus, gall-bladder, ovary, pancreas, stomach, cervix, thyroid, prostate, and skin, including squamous cell carcinoma; hematopoietic tumors of lymphoid lineage, including leukemia, acute lymphocitic leukemia, acute lymphoblastic leukemia, B-cell lymphoma, T-cell-lymphoma, Hodgkin's lymphoma, non-Hodgkin's lymphoma, hairy cell lymphoma and Burkett's lymphoma; hematopoietic tumors of myeloid lineage, including acute and chronic myelogenous leukemias, myelodysplastic syndrome and promyelocytic leukemia; tumors of mesenchymal origin, including fibrosarcoma and rhabdomyosarcoma; tumors of the central and peripheral nervous system, including astrocytoma, neuroblastoma, glioma and schwannomas; other tumors, including melanoma, seminoma, teratocarcinoma, osteosarcoma, xeroderma pigmentosum, keratoxanthoma, thyroid follicular cancer and Kaposi's sarcoma.
Due to the key role of PKs in the regulation of cellular proliferation, these compounds are also useful in the treatment of a variety of cell proliferative disorders such as, for instance, benign prostate hyperplasia, familial adenomatosis, polyposis, neuro-fibromatosis, psoriasis, vascular smooth cell proliferation associated with atherosclerosis, pulmonary fibrosis, arthritis glomerulonephritis and post-surgical stenosis and restenosis. The compounds of the invention can be useful in the treatment of Alzheimer's disease, as suggested by the fact that cdk5 is involved in the phosphorylation of tau protein (*J. Biochem.,* 117, 741-749, 1995).
The compounds of the invention are also useful in the treatment and prevention of radiotherapy-induced or chemotherapy-induced alopecia.
The compounds of this invention, as modulators of apoptosis, may also be useful in the treatment of cancer, viral infections, prevention of AIDS development in HIV-infected individuals, autoimmune diseases and neurodegenerative disorders.
The compounds of this invention may be useful in inhibiting tumor angiogenesis and metastasis, as well as in the treatment of organ transplant rejection and host versus graft diseases.
The compounds of the invention are useful as cyclin dependent kinase (cdk) inhibitors and also as inhibitors of other protein kinases such as, for instance, protein kinase C in different isoforms, Met, PAK-4, PAK-5, ZC-1, STLK-2, DDR-2, Aurora 1, Aurora 2, Bub-1, PLK, Chk1, Chk2, HER2, raf1, MEK1, MAPK, EGF-R, PDGF-R, FGF-R, IGF-R, VEGF-R, PI3K, weel kinase, Src, Ab1, Akt, ILK, MK-2, IKK-2, Cdc7, Nek, and thus be effective in the treatment of

diseases associated with other protein kinases.

## DETAILED DESCRIPTION OF THE INVENTION

Several pyrazoles and analogues thereof are known in the art, for instance as synthetic intermediates or even as therapeutic agents.

As an example, carboxamido-pyrazoles possessing cdk inhibitory activity have been described in U.S. Patent No. 6,218,418 to Pevarello et al.

Pyrazoles have been described for use in the treatment of inflammation. U.S. Patent No. 5,134,142 to Matsuo et al describes 1,5-diaryl pyrazoles, and specifically, 1-(4-fluorophenyl)-5-[4-(methylsulfonyl)phenyl]-3-trifluoromethyl pyrazole, as having anti-inflammatory activity.

U.S. patent No. 4,734,430 discloses benzo- and cycloheptadipyrazoles as bronchodilators; U.S. Patent No. 3,940,418 describes tricyclic 4,5-dihydrobenz[g]indazoles as anti-inflammatory agents. In addition, R. Hamilton [*J. Heterocyclic Chem.,* **13**, 545 (1976)] describes tricyclic 4,5-dihydrobenz[g]indazoles as anti-inflammatory agents. U.S. Patent No. 5,134,155 describes fused tricyclic pyrazoles having a saturated ring bridging the pyrazole and a phenyl radical as HMG-CoA reductase inhibitors. European publication EP 477,049, published Mar. 25, 1992, describes [4,5-dihydro-1-phenyl-1H-benz[g]indazol-3-yl]amides as having antipsychotic activity. European publication EP 347,773, published Dec. 27, 1989, describes [4,5-dihydro-1-phenyl-1H-benz[g]indazol-3-yl]propanamides as immunostimulants. M. Hashem et al [*J. Med. Chem.,* **19**, 229 (1976)] describes fused tricyclic pyrazoles, having a saturated ring bridging the pyrazole and a phenyl radical, as antibiotics.

Certain substituted pyrazolyl-benzenesulfonamides have been described in the literature as synthetic intermediates. Specifically, 4-[5-(4-chlorophenyl)-3-phenyl-1*H*-pyrazol-1-yl]benzenesulfonamide has been prepared from a pyrazoline compound as an intermediate for compounds having hypoglycemic activity [R. Soliman et al, *J. Pharm. Sci.,* **76,** 626 (1987)]. 4-[5-[2-(4-Bromophenyl)-2*H*-1,2,3-triazol-4-yl]-3-methyl-1*H*-pyrazol-1-yl]benzenesulfonamide has been prepared from a pyrazoline compound and described as potentially having hypoglycemic activity [H. Mokhtar, *Pak. J. Sci. Ind. Res.,* **31**, 762 (1988)]. Similarly, 4-[4-bromo-5-[2-(4-chlorophenyl)-2*H*-1,2,3-triazol-4-yl]-3-methyl-1*H*-pyrazol-1-yl]benzenesulfonamide has been prepared [H. Mokhtar et al, *Pak. J. Sci. Ind. Res.,* **34**, 9 (1991)].

The phytotoxicity of pyrazole derivatives is described [M. Cocco et al, *Il. Farmaco-Ed. Sci.,* **40**, 272 (1985)], specifically for 1-[4-(aminosulfonyl)phenyl]-5-phenyl-1H-pyrazole-3,4-dicarboxylic acid.

The use of styryl pyrazole esters for antidiabetes drugs is described [H. Mokhtar et al, *Pharmazie,* **33**, 649-651 (1978)]. The use of styryl pyrazole carboxylic acids for antidiabetes drugs is described [R. Soliman et al, *Pharmazie,* **33**, 184-5 (1978)]. The use of 4-[3,4,5-trisubstituted-pyrazol-1-yl]benzenesulfonamides as intermediates for sulfonylurea anti-diabetes agents is described, and specifically, 1-[4-(aminosulfonyl)phenyl]-3-methyl-5-phenyl-1*H*-pyrazole-4-carboxylic acid [R. Soliman et al, *J. Pharm. Sci.,* **72**, 1004 (1983)]. A series of 4-[3-substituted methyl-5-phenyl-1*H*-pyrazol-1-yl]benzenesulfonamides has been prepared as intermediates for anti-diabetes agents, and more specifically, 4-[3-methyl-5-phenyl-1*H*-pyrazol-1-yl]benzenesulfonamide [H. Feid-Allah, *Pharmazie,* **36**, 754 (1981)]. In addition, 1-(4-[aminosulfonyl]phenyl)-5-phenylpyrazole-3-carboxylic acid has been prepared from the above described 4-[3-methyl-5-phenyl-1*H*-pyrazol-1-yl]benzenesulfonamide compound [R. Soliman et al, *J. Pharm. Sci.,* **70**, 602 (1981)].

WO 00/27822 discloses tricyclic pyrazole derivatives, WO 00/59901 discloses dihydroindeno pyrazoles, WO 95/15315 discloses diphenyl pyrazole compounds, WO 95/15317 discloses triphenyl pyrazole compounds, WO 95/15318 discloses tri-substituted pyrazole compounds, and WO 96/09293 discloses benz[g]indazolyl derivatives.

WO 95/15316 discloses substituted pyrazolyl benzenesulfamide derivatives.

Accordingly, the present invention provides the use, for manufacturing a medicament for treating diseases caused by and/or associated with an altered protein kinase activity, by administering to a mammal in need thereof an effective amount of a compound represented by formulae (Ic), (Ie), (If) and (Ig)

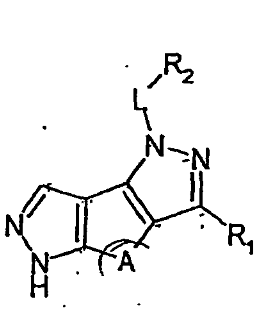

(Ic)

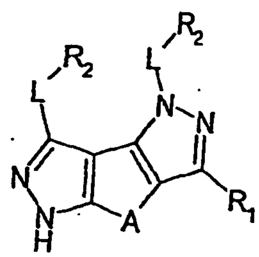

(Ie)

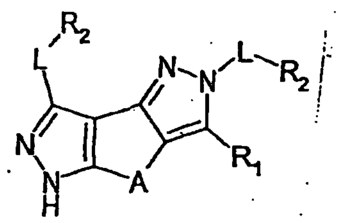

(If)

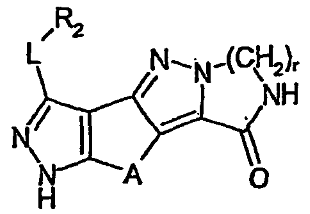

(Ig)

wherein:

$R_1$ in formula (Ic) is selected from the group consisting of hydrido, lower alkyl, perfluorinated lower alkyl, heterocyclyl, CN, $CO_2R'$, $COCF_3$, COR', CONR'R", NR'R", C(=NR')NR'R", $CONHNH_2$, CONHOR', NHCOR',$CH_2NH_2$, and $CH_2NHCOR'$; or

$R_1$ in formulae (Ie) and (If) is selected from the group consisting of CN, $CO_2R'$, COR', CONR'R", NR'R", C(=NOH) NR'R", $CONHNH_2$, CONHOR';

R' and R" in formula (Ic) are selected, each independently, from the group consisting of hydrido, hydroxy, alkyl, hydroxyalkyl, alkenyl, alkynyl, aryl, arylalkyl, heterocyclyl or heterocyclylalkyl; or

R' and R" in formulae (Ie) and (If) are selected, each independently, from the group consisting of hydrido or lower alkyl;

A in formulae.(Ic) is selected from the group consisting of $-CH_2-$; $-CH_2-CH_2-$; -CH=CH-; $(CH_2)-C(CH_3)_2$; or

A in formulae (Ie), (If) and (Ig) is selected from the group consisting of $-CH_2-CH_2-$; -CH=CH-; $(CH_2)-C(CH_3)_2$;

wherein L represents, each independently, a single bond, an alkylidene group or a divalent group selected from NH, NHCO, CONH, NHCONH, $SO_2NH$ and $NHSO_2$; $\mathbf{R_2}$ is, each independently, hydrido, alkyl, 5 to 12 membered mono- or bi-cyclic ring having from 0 to 3 heteroatoms selected from S, O and N, optionally substituted with one or more $-(CH_2)_q-R_3$ groups; or $R_2$ is a group of formula

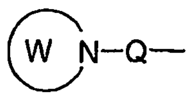

**W** is a 3 to 7 membered ring having one N heteroatom directly linked to Q and from 0 to 2 additional heteroatoms selected from the group consisting of S, SO, $SO_2$, O, N and NR', wherein R' is as above defined;

**Q** is a divalent group selected from CO, $SO_2$ and $(CH_2)_n$;

$\mathbf{R_3}$ is selected, each independently, from the group consisting of alkyl, halogen, $CF_3$, $OCF_3$, $NO_2$, CN, C(=NR') NR'R", OR', SR', OCOR', OCONRR", $COCF_3$, COR', $CO_2R'$, CONR'R", $SO_2R'$, $SO_2NR'R"$, NR'R", NR'COR', NR'COOR', NR'CONR'R", $NR'SO_2R'$, $NR'SO_2NR'R"$, Wherein R' and R" are as above defined;

**n** is, each independently, 0, 1, or 2;

**q** is, each independently, 0 or an integer from 1 to 3;

**r** is an integer from 1 to 3;

or isomers, tautomers, carriers, prodrugs and pharmaceutically acceptable salts thereof

In a preferred embodiment of the use described above, the disease caused by and/or associated with an altered protein kinase activity is selected from the group consisting of cancer, cell proliferative disorders, Alzheimer's disease, viral infections, auto-immune diseases and neurodegenerative disorders.

Specific types of cancer that may be treated include carcinoma, squamous cell carcinoma, hematopoietic tumors of myeloid or lymphoid lineage, tumors of mesenchymal origin, tumors of the central and peripheral nervous system, melanoma, seminoma, teratocarcinoma, osteosarcoma, xeroderma pigmentosum, keratoxanthoma, thyroid follicular cancer and Kaposi's sarcoma.

In another preferred embodiment of the use described above, the cell proliferative disorder is selected from the group consisting of benign prostate hyperplasia, familial adenomatosis polyposis, neuro-fibromatosis, psoriasis, vascular smooth cell proliferation associated with atherosclerosis, pulmonary fibrosis, arthritis glomerulonephritis and post-surgical stenosis and restenosis.

In addition, the use object of the present invention, also provides tumor angiogenesis and metastasis inhibition.

The present invention further provides a compound represented by formulae (Ic), (Ie), (If) and (Ig)

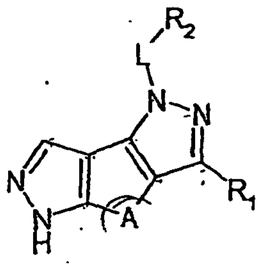

(Ic)

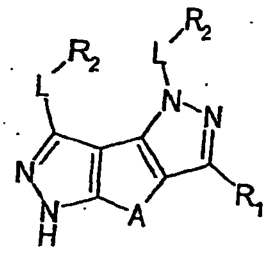

(Ie)

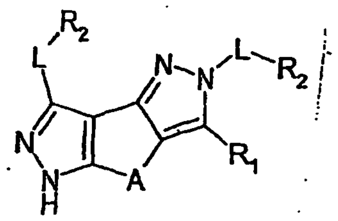

(If)

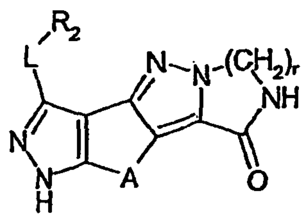

(Ig)

wherein:

$R_1$ in formula (Ic) is selected from the group consisting of perfluorinated lower alkyl, heterocyclyl, CN, $CO_2R'$, $COCF_3$, COR', CONR'R", NR'R", C(=NR')NR'R", $CONHNH_2$, CONHOR', NHCOR',$CH_2NH_2$, and $CH_2NHCOR'$; or
$R_1$ in formulae (Ie) and (If) is selected from the group consisting of CN, $CO_2R'$, COR', CONR'R", NR'R", C(=NOH) NR'R", $CONHNH_2$, CONHOR;
R' and R" in formula (Ic) are selected, each independently, from the group consisting of hydrido, hydroxy, alkyl, hydroxyalkyl, alkenyl, alkynyl, aryl, arylalkyl, heterocyclyl or heterocyclylalkyl; or
R' and R" in formulae (Ie) and (If) are selected, each independently, from the group consisting of hydrido or lower alkyl;
A in formulae (Ic) is selected from the group consisting of $-CH_2-$; $-CH_2-CH_2-$; -CH=CH-; $(CH_2)-C(CH_3)_2$; or
A in formulae (Ie), (If) and (Ig) is selected from the group consisting of $-CH_2-CH_2-$; -CH=CH-; $(CH_2)-C(CH_3)_2$;
wherein L represents, each independently, a single bond, an alkylidene group or a divalent group selected from NH, NHCO, CONH, NHCONH, $SO_2NH$ and $NHSO_2$; **$R_2$** is, each independently, hydrido, alkyl, 5 to 12 membered mono- or bi-cyclic ring having from 0 to 3 heteroatoms selected from S, O and N, optionally substituted with one or more $-(CH_2)_q-R_3$ groups; or $R_2$ is a group of formula

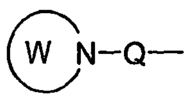

**W** is a 3 to 7 membered ring having one N heteroatom directly linked to Q and from 0 to 2 additional heteroatoms selected from the group consisting of S, SO, $SO_2$, O, N and NR', wherein R' is as above defined;
**Q** is a divalent group selected from CO, $SO_2$ and $(CH_2)_n$;
**$R_3$** is selected, each independently, from the group consisting of alkyl, halogen, $CF_3$, $OCF_3$, $NO_2$, CN, C(=NR') NR'R", OR', SR', OCOR', OCONR'R", $COCF_3$, COR', $CO_2R'$, CONR'R", $SO_2R'$, $SO_2NR'R"$, NR'R", NR'COR', NR'COOR', NR'CONR'R", $NR'SO_2R'$, $NR'SO_2NR'R"$, wherein R' and R" are as above defined;
n is, each independently, 0,1, or 2;
q is, each independently, 0 or an integer from 1 to 3;
r is an integer from 1 to 3;

or isomers, tautomers, carriers, prodrugs, and pharmaceutically acceptable salts thereof.

Unless otherwise specified, when referring to the compounds of formulae (Ic), (Ie), (If) and (Ig) per se as well as to any pharmaceutical composition thereof or to any therapeutic treatment comprising them, the present invention includes all of the hydrates, solvates, complexes and prodrugs of the compounds of this invention. Prodrugs are any

covalently bonded compounds, which release the active parent drug according to formulae (Ic), (Ie), (If) and (Ig) in vivo.

If a chiral center or another form of an isomeric center is present in a compound of the present invention, all forms of such isomer or isomers, including enantiomers and diastereomers, are intended to be covered herein. Compounds containing a chiral center may be used as a racemic mixture, an enantiomerically enriched mixture, or the racemic mixture may be separated using well-known techniques and an individual enantiomer may be used alone. In cases in which compounds have unsaturated carbon-carbon double bonds, both the cis (Z) and trans (E) isomers are within the scope of this invention. In cases wherein compounds may exist in tautomeric forms, such as keto-enol tautomers, each tautomeric form is contemplated as being included within this invention whether existing in equilibrium or predominantly in one form.

The meaning of any substituent at any one occurrence in formulae (Ic), (Ie), (If) and (Ig) or any sub-formula thereof is independent of its meaning, or any other substituents meaning, at any other occurrence, unless specified otherwise.

The term aromatic ring does not need any further clarification as it refers to any ring which can be conventionally defined as aromatic, such a term being widely used in organic chemistry.

Non limiting examples of aromatic rings according to the invention are, for instance, thiophene, furan, furazan, pyrrole, pyrazole, imidazole, thiazole, isothiazole, oxazole or isoxazole.

With the term hydrido it is intended a single hydrogen atom (H); this hydrido radical may be attached, for example, to an oxygen atom to form a hydroxyl radical or two hydrido radicals may be attached to a carbon atom to form a methylene ($-CH_2-$) radical.

With the term lower alkyl group we intend any straight or branched alkyl group with from 1 to 6 carbon atoms such as, for instance, methyl, ethyl, n-propyl, isopropyl, n-butyl, isobutyl, sec-butyl, tert-butyl, n-pentyl, n-hexyl, and the like.

Pefluorinated lower alkyl groups stand for the above lower alkyl groups being further substituted in any of the free positions, at the same or different carbon atom, by more than one fluorine atoms. Non limiting examples of perfluorinated alkyl groups are, for instance, trifluoromethyl, 2,2,2-trifluoroethyl, 1,2-difluoroethyl, 1,1,1,3,3,3-hexafluoropropyl-2-yl, and the like.

Unless otherwise specified in the present description, with the term heterocyclyl we intend any 5 or 6 membered heterocyclic radical with from 1 to 3 heteroatoms selected among N, O and S. If not specifically noted otherwise, the said heterocyclic moieties may comprise saturated, partly unsaturated and fully unsaturated heterocycles; these latter, clearly referable to as aromatic heterocycles, are also conventionally known as heteroaromatic or heteroaryl rings. Non limiting examples of the said heterocycles of the invention are, for instance, thiophene, furan, furazan, pyran, pyrrole, imidazole, pyrazole, thiazole, isothiazole, oxazole, isoxazole, pyridine, pyrazine, pyrimidine, pyridazine, pyrrolidine, pyrroline, imidazolidine, imidazoline, pyrazolidine, pyrazoline, piperidine, piperazine, morpholine, and the like.

With the term hydroxyalkyl we intend any of the above straight or branched lower alkyl radicals having from one to six carbon atoms, any one of which may be substituted with one or more hydroxyl radicals.

With the term halogen atom, optionally referable to as "halo" group, herewith intended are fluorine, chlorine, bromine and iodine atoms.

With the term alkenyl or alkynyl we intend any of the aforementioned lower alkyl groups with from 2 to 6 carbon atoms, bearing a double or triple bond. Non limiting examples of alkenyl or alkynyl groups are thus, for instance, vinyl, allyl, 1-propenyl, isopropenyl, 1-butenyl, 2-butenyl, 3-butenyl, 2-pentenyl, 1-hexenyl, ethynyl, 2-propynyl, 4-pentynyl, and the like.

With the term aryl we intend, unless otherwise specified, any aromatic ring hence including carbocyclic or 5 or 6 membered heterocyclic rings with from 1 to 3 heteroatoms selected among N, O and S. Non limiting examples of aryl groups are thus phenyl, furyl, thienyl, pyrrolyl, imidazolyl, pyrazolyl, thiazolyl, isothiazolyl, oxazolyl, isoxazolyl, pyridyl, pyrazinyl, pyrimidinyl, pyridazinyl, and the like.

With the terms arylalkyl or heterocyclyl-alkyl groups we intend any of the above groups being defined according to the single moieties from which they derive. More particularly, arylalkyl and heterocyclyl-alkyl groups stand for the above alkyl groups further substituted by aryl or heterocyclyl groups, respectively, these latter being as above defined.

Substitutions may occur in any of the free positions of both rings, by replacement of one or more hydrogen atoms, otherwise referred to as hydrido.

When referring to alkylidene, L may represent a saturated divalent hydrocarbon group, with from 1 to 6 carbon atoms such as, for instance, a $-(CH_2)_{1-6}-$ group.

Unless otherwise specified, with the term 5 to 12-membered, either mono- or bi-cyclic ring system, with 0 to 3 heteroatoms among N, O and S, we intend any carbocyclic (e.g. 0 heteroatoms) or heterocyclic (e.g. 1 to 3 heteroatoms) ring, either saturated, partly unsaturated or fully unsaturated (e.g. aromatic) ring system. Unless otherwise defined, within the above bi-cyclic ring systems, each of the two ring units may be fused to each other or otherwise linked through a single bond.

Non limiting examples of the above carbocyclic ring systems include, for instance, cyclopentane, cyclopentene,

cyclohexane, cyclohexene, cyclohexadiene, benzene, naphthalene and biphenylene.

Examples of the above heterocylic ring systems may typically include any of the aforementioned 5 or 6 membered, either saturated, partly unsaturated or fully unsaturated heterocycles (see examples above) which may be further condensed to, or linked through a single bond with, any of the aforementioned mono-cyclic carbocyclic or heterocyclic rings themselves.

Finally, when referring to the W ring, it represents a 3 to 7 membered heterocyclic ring at least containing a N nitrogen atom directly linked to Q, as set forth above. The term "pharmaceutically acceptable salts" embraces salts commonly used to form alkali metal salts and to form addition salts of free acids or free bases. The nature of the salt is not critical, provided that it is pharmaceutically acceptable. Suitable pharmaceutically acceptable acid addition salts of compounds of the present invention may be prepared from an inorganic acid or from an organic acid. Examples of such inorganic acids are hydrochloric, hydrobromic, hydroiodic, nitric, carbonic, sulfuric, and phosphoric acid. Appropriate organic acids may be selected from aliphatic, cycloaliphatic, aromatic, araliphatic, heterocyclic, carboxylic and sulfonic classes of organic acids, examples of which are formic, acetic, trifluoroacetic propionic, succinic, glycolic, gluconic, lactic, malic, tartaric, citric, ascorbic, glucuronic, maleic, fumaric, pyruvic, aspartic, glutamic, benzoic, anthranilic, mesylic, salicyclic, salicyclic, phydroxybenzoic, phenylacetic, mandelic, embonic (pamoic), methanesulfonic, ethanesulfonic, benzenesulfonic, pantothenic, toluenesulfonic, 2-hydroxyethanesulfonic, sulfanilic, stearic, cyclohexylaminosulfonic, algenic, hydroxybutyric, salicyclic, galactaric and galacturonic acid. Suitable pharmaceutically acceptable base addition salts of compounds of the present invention include metallic salts made from aluminum, calcium, lithium, magnesium, potassium, sodium and zinc or organic salts made from N,N'-dibenzylethylenediamine, chloroprocaine, choline, diethanolamine, ethylenediamine, meglumine (N-methyl-glucamine) and procaine. All of these salts may be prepared by conventional means from the corresponding compound of the present invention by reacting, for example, the appropriate acid or base.

A class of compounds of the invention is represented by the derivatives of formula (Ic)

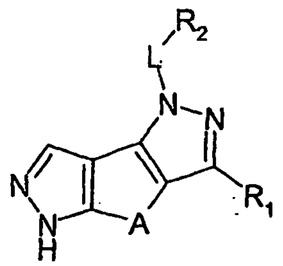

(Ic)

wherein $R_1$, L and $R_2$ are, each independently, as above defined, and A is selected from the group consisting of $-CH_2-$, $-CH_2-CH_2-$, -CH=CH- and $-CH_2-C(CH_3)_2-$. Another class of compounds of the invention is represented by the derivatives of formulae (Ie) or (If)

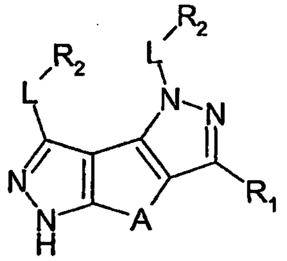

(Ie)

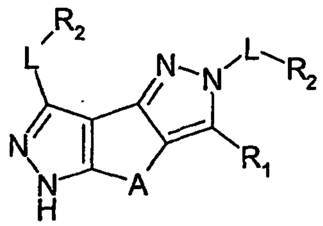

(If)

wherein L and $R_2$ are, each independently and the same or different in each occasion, as above defined; A is selected from the group consisting of $-CH_2-CH_2-$, -CH=CH- and $-CH_2-C(CH_3)_2-$; and $R_1$ is a group selected from NR'R", CN, $CO_2R'$, COR', CONR'R", CONHOR', $CONHNH_2$ and C(=NOH)NR'R", wherein R' and R" are, the same or different, hydrido or alkyl.

Another class of compounds of the invention is represented by the derivatives of formula (Ig)

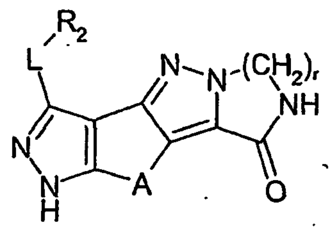

(Ig)

wherein L, $R_2$ and r are as above defined; and A is selected from the group consisting of $-CH_2-CH_2-$, -CH=CH- and $-CH_2-C(CH_3)_2-$.

Still more preferred, in any one of the above classes, are the derivatives wherein L is methylene or a single bond and $R_2$ is hydrido, phenyl or a 5 or 6 membered aromatic heterocycle having 1 or 2 heteroatoms selected among N, O and S, the said phenyl or heterocycle being optionally further substituted as above indicated.

Even more preferred are these latter derivatives of formula (1) wherein $R_2$, being optionally further substituted as above indicated, is selected from the group consisting of hydrido, phenyl, pyridyl, pyridazinyl or pyrimidinyl.

For a general reference to the specific compounds of formula (I) of the invention, and the pharmaceutically acceptable salts thetreof, see the experimental section.

As set forth above, it is a further object of the present invention a process for preparing the compounds of formula (I) and the pharmaceutically acceptable salts thereof The said process can be conveniently described as set forth below according to Schemes I-VI.

## SCHEME I

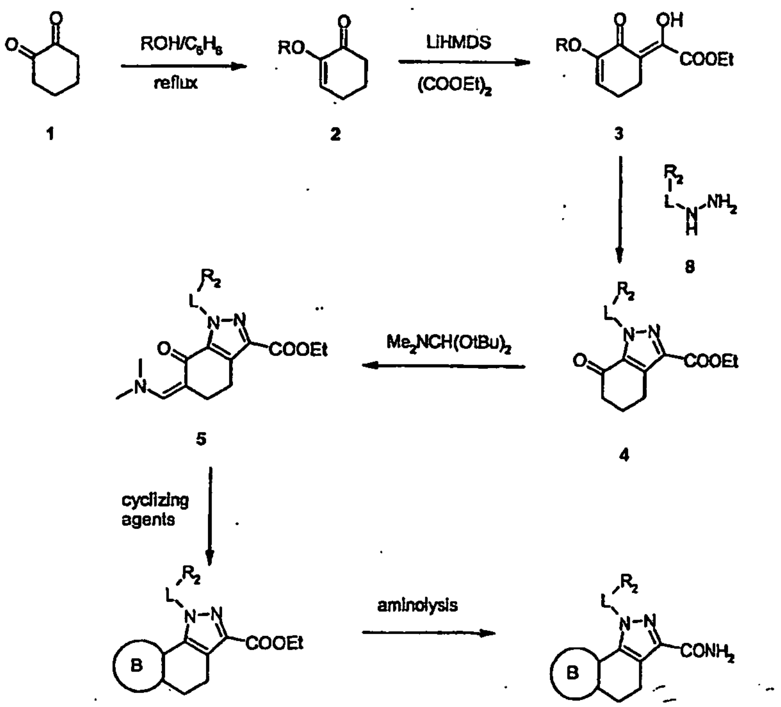


Scheme I describes the synthesis of the pyrazoles of formulae (Ic), (Ie), (If) and (Ig) wherein B is a pyrazole ring with fused heterocycles such as, for instance, substituted pyrimidine and pyrazole derivatives. In step one, 1,2-cyclohexanedione **(1)** was refluxed with alcohols such as methanol or ethanol in benzene to provide the desired enone **(2)**. In step two, enone **(2)** was treated with a base such as lithium bistrimethylsilylamide, followed by condensation with diethyl oxalate to afford 1,3-diketone **(3)**.

In step three, 1,3-diketone was allowed to react with a suitably substituted hydrazine of general formula **(8)** to form pyrazole **(4)**.

In step four, pyrazole was treated with dimethylformamide di-tert-butyl acetal to give enaminone **(5)**. In step

five, enaminone was condensed with cyclizing agents such as hydrazine, guanidine, or thiourea derivatives to afford fused pyrazoles and pyrimidines **(6)**.

In the final step, the ester was converted to amide **(7)** by treatment with ammonium hydroxide in methanol, at a temperature ranging from about 25°C to about 70°C, in a sealed tube.

Hydrazines of general formula **(8)** are commercially available or can be obtained through synthetic procedures well described in the literature. For instance, aryl-hydrazines can be conveniently obtained from the corresponding anilines by diazotization, using sodium nitrite, or an alkyl nitrite, followed by catalytic or chemical reduction as described, for example, in J. Med. Chem., 36, 1529 (1993). In selected cases, aryl halides suitably activated with electron withdrawing groups can be converted to the corresponding arylhydrazines through displacement of the halogen atom with hydrazine or a carbazate, followed by hydrolysis of the protecting group, for instance as reported in J. Het. Chem., 25,1543 (1988) or in Tetrah. Lett., 40 (18), 3543 (1999).

Alkyl-hydrazines can be obtained from alkyl-amines by treatment with hydroxylamine-O-sulfonic acid, for instance as described in JOC, 14, 813 (1949).

**SCHEME II**

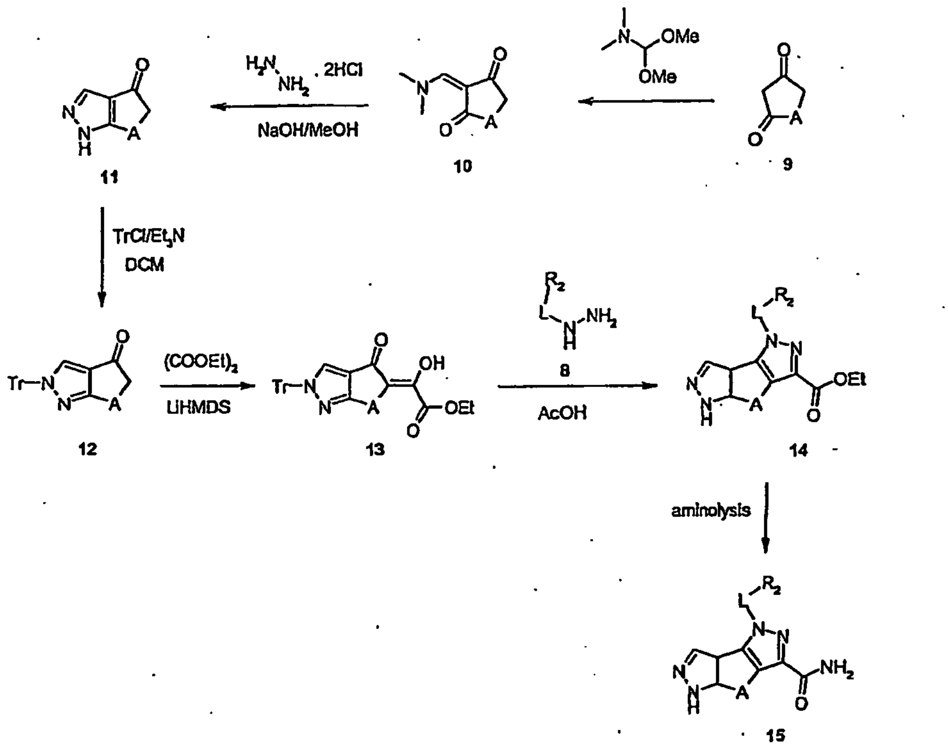


The synthetic pathway reported in Scheme II illustrates a procedure, alternative to Scheme I, for the preparation of derivatives of formulae (Ic), (Ie), (If) and (Ig) wherein A is preferably selected among $-CH_2-$, $-CH_2-CH_2-$, $-CH_2-C(CH_3)_2-$.

In step one, the cyclic diketone **(9)** was condensed with N,N-dimethylformamide dimethyl acetal to obtain the adduct **(10)**, as described in Heterocycles, 32, 41 (1991). In step two, the adduct **(10)** was reacted with hydrazine dihydrochloride to obtain the intermediate **(11)**, that was protected with trityl chloride (step three) to give the intermediate **(12)**. After condensation with oxalyl chloride (step four), the diketoester **(13)** was allowed to react with a suitably substituted hydrazine **(8)** (step five) to form the dipyrazole **(14)**. If a salified form of the.hydrazine **(8)** is used (i.e. hydrochloride), the trityl protecting group is normally lost during the cyclization reaction. Optionally, diluted hydrochloric acid can be added to complete the deprotection, once the cyclization has occurred. In step six, the ester was then converted to the amide **(15)** by treatment with ammonium hydroxide in methanol, at a temperature ranging from about 25°C to about 70°C, in a sealed tube.

The intermediate compound **(11)** wherein A is $-CH_2-$ or $-CH_2-CH_2-$, as well as the intermediate compounds **(12)** and **(13)** wherein A is selected from $-CH_2-$, $-CH_2-CH_2-$ and $-CH_2-C(CH_3)_2-$ are novel and, hence, represent a further object of the present invention.

## SCHEME III

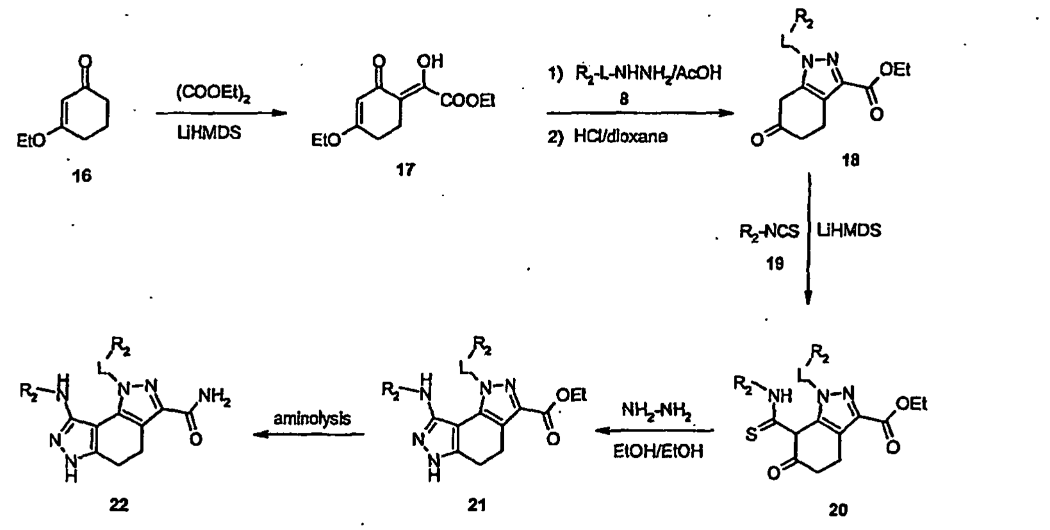

16 17 18 20 21 22

Scheme III illustrates the general synthetic procedure for the preparation of benzodipyrazole derivatives of general formula (I) wherein the pyrazole ring on the left side of the formulae is further substituted by a L-$R_2$ group wherein L is NH.

In step one the commercially available 3-ethoxy-cyclohex-2-enone **(16)** is condensed with diethyl oxalate to afford the diketoester **(17)**, which is then reacted, in step two, with a suitably substituted hydrazine **(8)** to give the pyrazole derivative **(18)**.

In step three the pyrazole **(18)** is treated in the presence of a base, such as lithium bistrimethylsilylamide, with a suitably substituted isothiocyanate **(19)** to afford the intermediate **(20)**, which is then converted to the 3-aminobenzodipyrazole ester of formula **(21)**. In the last step, the ester **(21)** is finally converted to the corresponding amide (22) under standard operative conditions. Isothiocyanates of general formula **(19)** are commercially available or can be obtained through synthetic procedures well described in the literature.

## SCHEME IV

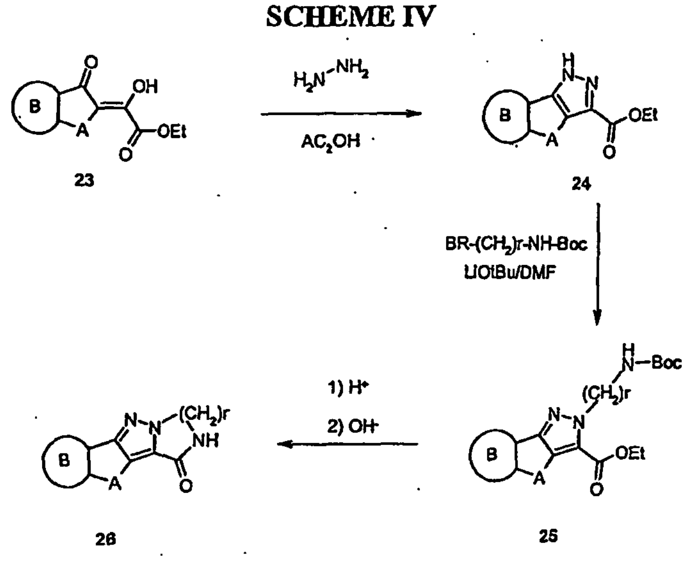

23 24 25 26

Scheme IV describes the general synthetic pathway to obtain compounds of formulae (Ic), (Ie), (If) and (Ig) wherein B in the formulae of the scheme is a pyrazole ring and A is preferably selected from $-CH_2-$, $-CH_2-CH_2-$ or $-CH_2-C(CH_3)_2-$. More generally, scheme IV can also be used to obtain compounds of general formula (I) wherein group L-$R_2$ is linked to Y. In the first step, the intermediate compound **(23)** is reacted with hydrazine to form the pyrazole derivative **(24)**. This is then alkylated, in step two, using an alkyl halide bearing a protected amino group, for instance as tert-butoxy-carbonyl (BOC) amino group. In step three, after removal of the protecting group, the intermediate **(25)** is allowed

to cyclize so to form the final compound **(26)** under standard operative conditions.

## SCHEME V

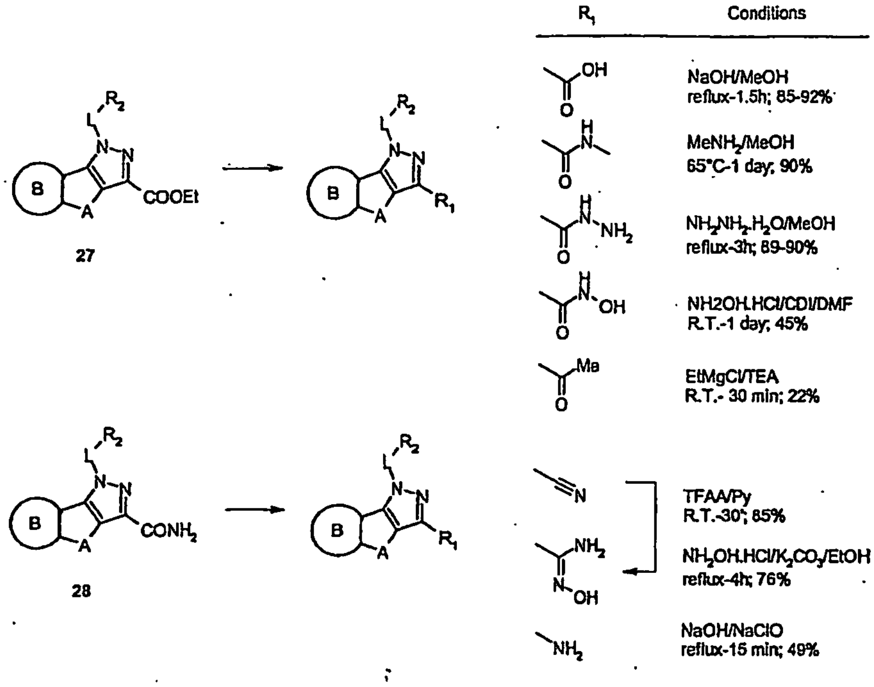


27

28

Scheme V refers to some examples describing the possibility of obtaining compounds of formulae (Ic), (Ie), (If) and (Ig) differently substituted in $R_1$ and wherein B is a pyrazole ring. Preferably, A is selected from the group consisting $-CH_2-$, $-CH_2-CH_2-$, $-CH_2-C(CH_3)_2-$ and -CH=CH-. The ring B in Scheme V is a pyrazole ring. The above reactions of transformation are generally performed by properly reacting the alkoxycarbonyl group of the intermediate **(27)** or the aminocarbonyl group of the intermediate **(28)**, each of which may be suitably protected. The transformations and related experimental conditions shown in scheme V, are readily apparent to one skilled in the art and are thus provided for exemplification purposes only, without limiting the scope of the invention.

## SCHEME VI

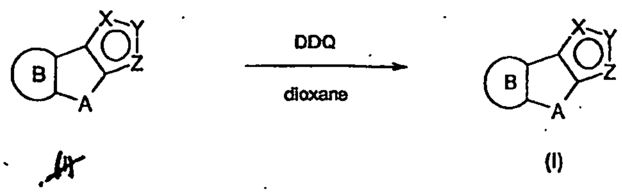


(I)

A = $-CH_2-CH_2-$ A = -CH=CH-

Synthetic scheme VI describes a general procedure for transforming the compounds of in Scheme VI the compounds of formulae (Ic), (Ie), (If) and (Ig) are generically represented by formula (I) and both B and X, Y, Z rings are pyrazole rings and A is $-CH_2-CH_2-$, to the corresponding aromatic counterparts of formulae (Ic), (Ie), (If) and (Ig) wherein A is -CH=CH-. The oxidation of the central ring can be accomplished according to conventional techniques, for instance by using activated quinone derivatives, e.g. 2,3-dichloro-5,6-dicyano-1,4-benzoquinone or, alternatively, palladium on charcoal in a suitable solvent such as decalin, at high temperatures.

When preparing the compounds of according to any variant of the process, which are all to be intended as within the scope of the present invention, optional functional groups within both the starting materials, the reagents or the intermediates thereof, and which could give rise to unwanted side reactions, need to be properly protected according to conventional techniques.

Likewise, the conversion of these latter into the free deprotected compounds may be carried out according to known procedures.

Pharmaceutically acceptable salts of the compounds of formulae (Ic), (Ie), (If) and (Ig) or, alternatively, their free compounds from the salts thereof, my be all obtained according to conventional methods.

Any of the starting material within schemes I to VI and reactants thereof are known, or may be easily prepared according to known methods.

From all of the above, it is also clear to the skilled man that any compound of formulae (Ic), (Ie), (If) and (Ig) of the invention may be prepared by working in analogy to what reported in any one of schemes I to VI and, perhaps, by optionally providing any required modification to the above reactions, on a case by case. The said reactions are however known and conventionally adopted when preparing tricyclyc heterocyclic derivatives of formulae (Ic), (Ie), (If) and (Ig) and substituted compounds thereof.

## PHARMACOLOGY

The compounds of formulae (Ic), (Ie), (If) and (Ig) are active as protein kinase inhibitors and are therefore useful, for instance, to restrict the unregulated proliferation of tumor cells.

In therapy, they may be used in the treatment of various tumors, such as those formerly reported, as well as in the treatment of other cell proliferative disorders such as psoriasis, vascular smooth cell proliferation associated with atherosclerosis and post-surgical stenosis and restenosis and in the treatment of Alzheimer's disease.

The inhibiting activity of putative Cdk/Cyclin inhibitors and the potency of selected compounds was determined through a method of assay based on the use of the SPA technology (Amersham Pharmacia Biotech).

The assay consists of the transfer of radioactivity labelled phosphate moiety by the kinase to a biotinylated substrate. The resulting 33P-labelled biotinylated product is allowed to bind to streptavidin-coated SPA beads (biotin capacity 130 pmol/mg), and light emitted was measured in a scintillation counter.

### Inhibition assay of Cdk2/Cyclin A activity

**Kinase reaction:** 4 μM in house biotinylated histone H1 (Sigma # H-5505) substrate, 10 μM ATP (0.1 microCi $P^{33}\gamma$-ATP), 4.2 ng Cdk2/Cyclin A complex, inhibitor in a final volume of 30 μl buffer (TRIS HCl 10 mM pH 7.5, $MgCl_2$ 10 mM, DTT 7.5 mM + 0.2 mg/ml BSA) were added to each well of a 96 U bottom. After 30 min at r.t. incubation, reaction was stopped by 100 μl PBS + 32 mM EDTA + 0.1% Triton X-100 + 500 μM ATP, containing 1 mg SPA beads. Then a volume of 110 μl is transferred to Optiplate. After 20 min. incubation for substrate capture, 100. μl 5M CsCl were added to allow statification of beads to the top of the plate and let stand 4 hours before radioactivity counting in the Top-Count instrument

**IC50 determination:** inhibitors were tested at different concentrations ranging from 0.0015 to 10 μM. Experimental data were analyzed by the computer program GraphPad Prizm using the four parameter logistic equation:

$$y = bottom+(top-bottom)/(1+10^\wedge((logIC50-x)*slope))$$

where x is the logarithm of the inhibitor concentration, y is the response; y starts at bottom and goes to top with a sigmoid shape.

### Ki calculation:

**Experimental method:** Reaction was carried out in buffer (10 mM Tris, pH 7.5, 10 mM $MgCl_2$, 0.2 mg/ml BSA, 7.5 mM DTT) containing 3.7 nM enzyme, histone and ATP (constant ratio of cold/labeled ATP 1/3000). Reaction was stopped with EDTA and the substrate captured on phosphomembrane (Multiscreen 96 well plates from Millipore). After extensive washing, the multiscreen plates are read on a top counter. Control (time zero) for each ATP and histone concentrations was measured.

**Experimental design:** Reaction velocities are measured at different four ATP, substrate (histone) and inhibitor concentrations. An 80-point concentration matrix was designed around the respective ATP and substrate Km values, and the inhibitor IC50 values (0.3, 1, 3, 9 fold the Km or IC50 values). A preliminary time course experiment in the absence of inhibitor and at the different ATP and substrate concentrations allow the selection of a single endpoint time (10 min) in the linear range of the reaction for the Ki determination experiment.

**Kinetic parameter estimates:** Kinetic parameters were estimated by simultaneous nonlinear least-square regression using [Eq.1] (competitive inhibitor respect to ATP, random mechanism) using the complete data set (80 points):

$$v = \frac{Vm \bullet A \bullet B}{\alpha \bullet Ka \bullet Kb + \alpha \bullet Ka \bullet B + a \bullet Kb \bullet A + A \bullet B + \alpha \bullet \frac{Ka}{Ki} \bullet I \bullet (Kb + \frac{B}{\beta})} \quad \text{[Eq.1]}$$

where A=[ATP], B=[Substrate], I=[inhibitor], Vm= maximum velocity, Ka, Kb, Ki the dissociation constants of ATP, substrate and inhibitor respectively. $\alpha$ and $\beta$ the cooperativity factor between substrate and ATP binding and substrate and inhibitor binding respectively.

In addition the selected compounds have been characterized on a panel of ser/threo kinases strictly related to cell cycle (Cdk2/Cyclin E, Cdk1/cyclin BI, Cdk5/p25, Cdk4/Cyclin D1), and also for specificity on MAPK, PKA, EGFR, IGF1-R, Aurora-2 and Akt.

**Inhibition assay of Cdk2/Cyclin E activity**

**Kinase reaction:** 10 μMin house biotinylated histone H1 (Sigma # H-5505) substrate, 30 μMATP (0.3 microCi $P^{33}\gamma$-ATP), 4 ng GST-Cdk2/Cyclin E complex, inhibitor in a final volume of 30μl buffer (TRIS HCl 10 mM pH 7.5, $MgCl_2$ 10 mM, DTT 7.5 mM + 0.2 mg/ml BSA) were added to each well of a 96 U bottom. After 60 min at r.t. incubation, reaction was stopped by 100 μl PBS + 32 mM EDTA + 0.1% Triton X-100 + 500 μM ATP, containing 1 mg SPA beads. Then a volume of 110 μl is transferred to Optiplate.

After 20 min. incubation for substrate capture, 100 μl 5M CsCl were added to allow statification of beads to the top of the plate and let stand 4 hours before radioactivity counting in the Top-Count instrument

**IC50 determination:** see above

**Inhibition assay of Cdk1/Cyclin B1 activity**

**Kinase reaction:** 4 μM in house biotinylated histone H1 (Sigma # H-5505) substrate, 20 μM ATP (0.2 microCi $P^{33}\gamma$-ATP), 3 ng Cdk1/Cyclin B complex, inhibitor in a final volume of 30 μl buffer (TRIS HCl 10 mM pH 7.5, $MgCl_2$ 10 mM, DTT 7.5 mM + 0.2 mg/ml BSA) were added to each well of a 96 U bottom. After 20 min at r.t. incubation, reaction was stopped by 100 μl PBS + 32 mM EDTA + 0.1% Triton X-100 + 500 μM ATP, containing 1 mg SPA beads. Then a volume of 110 μl is transferred to Optiplate.

After 20 min. incubation for substrate capture, 100 μl 5M CsCl were added to allow statification of beads to the top of the Optiplate and let stand 4 hours before radioactivity counting in the Top-Count instrument.

**IC50 determination:** see above

**Inhibition assay of Cdk5/p25 activity**

The inhibition assay of Cdk5/p25 activity was performed according to the following protocol.

**Kinase reaction:** 10 μM biotinylated histone H1 (Sigma # H-5505) substrate, 30 μM ATP (0.3 microCi $P^{33}\gamma$-ATP), 15 ng CDK5/p25 complex, inhibitor in a final volume of 30 μl buffer (TRIS HCl 10 mM pH 7.5, $MgCl_2$ 10 mM, DTT 7.5 mM + 0.2 mg/ml BSA) were added to each well of a 96 U bottom. After 30 min at r.t. incubation, reaction was stopped by 100 μl PBS + 32 mM EDTA +0.1% Triton X-100 + 500 μM ATP, containing 1 mg SPA beads. Then a volume of 110 μl is transferred to Optiplate.

After 20 min. incubation for substrate capture, 100μl 5M CsCl were added to allow statification of beads to the top of the plate and let stand 4 hours before radioactivity counting in the Top-Count instrument.

**IC50 determination:** see above

**Inhibition assay of Cdk4/Cyclin D1 activity**

**Kinase reaction:** 0,4 uM μM mouse GST-Rb (769-921) (# sc-4112 from Santa Cruz) substrate, 10 μM ATP (0.5 μCi $P^{33}\gamma$-ATP), 100 ng of baculovirus expressed GST-Cdk4/Cyclin D1, suitable concentrations of inhibitor in a final volume of 50 μl buffer (TRIS HCl 10 mM pH 7.5, $MgCl_2$ 10 mM, 7.5 mM DTT+ 0.2mg/ml BSA) were added to each well of a 96 U bottom well plate. After 40 min at 37 °C incubation, reaction was stopped by 20 μl EDTA 120 mM.

**Capture:** 60 μl were transferred from each well to MultiScreen plate, to allow substrate binding to phosphocellulose filter. Plates were then washed 3 times with 150 μl/well PBS $Ca^{++}/Mg^{++}$ free and filtered by MultiScreen filtration system.

**Detection:** filters were allowed to dry at 37°C, then 100 μl/well scintillant were added and $^{33}P$ labeled Rb

fragment was detected by radioactivity counting in the Top-Count instrument.

**IC50 determination:** see above

## Inhibition assay of MAPK activity

**Kinase reaction:** 10 μM in house biotinylated MBP (Sigma # M-1891) substrate, 15 μM ATP (0.15 microCi $P^{33}\gamma$-ATP), 30 ng GST-MAPK (Upstate Biothecnology # 14-173), inhibitor in a final volume of 30 μl buffer (TRIS HCl 10 mM pH 7.5, $MgCl_2$ 10 mM, DTT 7.5 mM + 0.2 mg/ml BSA) were added to each well of a 96 U bottom. After 30 min at r.t. incubation, reaction was stopped by 100 μl PBS + 32 mM EDTA + 0.1% Triton X-100 + 500 μM ATP, containing 1 mg SPA beads. Then a volume of 110 μl is transferred to Optiplate.

After 20 min. incubation for substrate capture, 100 μl 5M CsCl were added to allow statification of beads to the top of the Optiplate and let stand 4 hours before radioactivity counting in the Top-Count instrument.

**IC50 determination:** see above

## Inhibition assay of PKA activity

**Kinase reaction:** 10 μM in house biotinylated histone H1 (Sigma # H-5505) substrate, 10 μM ATP (0.2 microM $P^{33}\gamma$-ATP), 0.45 U PKA (Sigma # 2645), inhibitor in a final volume of 30 μl buffer (TRIS HCl 10 mM pH 7.5, $MgCl_2$ 10 mM, DTT 7.5 mM + 0.2 mg/ml BSA) were added to each well of a 96 U bottom. After 90 min at r.t. incubation, reaction was stopped by 100 μl PBS + 32 mM EDTA + 0.1% Triton X-100 + 500 μM ATP, containing 1 mg SPA beads. Then a volume of 110 μl is transferred to Optiplate. After 20 min. incubation for substrate capture, 100μl 5M CsCl were added to allow statification of beads to the top of the Optiplate and let stand 4 hours before radioactivity counting in the Top-Count instrument.

**IC50 determination:** see above

## Inhibition assay of EGFR activity

**Kinase reaction:** 10 μM in house biotinylated MBP (Sigma # M-1891) substrate, 2 μM ATP (0.04 microCi $P^{33}\gamma$-ATP), 36 ng insect cell expressed GST-EGFR, inhibitor in a final volume of 30 μl buffer (Hepes 50 mM pH 7.5, $MgCl_2$ 3 mM, $MnCl_2$ 3 mM, DTT 1 mM, $NaVO_3$ 3μM + 0.2 mg/ml BSA) were added to each well of a 96 U bottom. After 20 min at r.t incubation, reaction was stopped by 100 μl PBS + 32 mM EDTA + 0.1% Triton X-100 + 500 μM ATP, containing 1 mg SPA beads. Then a volume of 110 μl is transferred to Optiplate.

After 20 min. incubation for substrate capture, 100μl 5M CsCl were added to allow statification of beads to the top of the Optiplate and let stand 4 hours before radioactivity counting in the Top-Count instrument.

**IC50 determination:** see above

## Inhibition assay of IGF1-R activity

The inhibition assay of IGF1-R activity was performed according to the following protocol.

**Kinase reaction:** 10 μM biotinylated MBP (Sigma cat. # M-1891) substrate, 0-20 μM inhibitor, 6 μM ATP, 1 microCi $^{33}P$-ATP, and 22.5 ng GST-IGF1-R (pre-incubated for 30 min at room temperature with cold 60 μM cold ATP) in a final volume of 30 μl buffer (50 mM HEPES pH 7.9, 3 mM $MnCl_2$, 1 mM DTT, 3 μM $NaVO_3$) were added to each well of a 96 U bottom well plate. After incubation for 35 min at room temperature, the reaction was stopped by addition of 100 μl PBS buffer containing 32 mM EDTA, 500 μM cold ATP, 0.1% Triton X100 and 10mg/ml streptavidin coated SPA beads. After 20 min incubation, 110 μL of suspension were withdrawn and transferred into 96-well OPTIPLATEs containing 100 μl of 5M CsCl. After 4 hours, the plates were read for 2 min in a Packard TOP-Count radioactivity reader.

## Inhibition assay of Aurora-2 activity

**Kinase reaction:** 8 μM biotinylated peptide (4 repeats of LRRWSLG), 10 μM ATP (0.5 uCi $P^{33}\gamma$-ATP), 15 ng Aurora2, inhibitor in a final volume of 30 μl buffer (HEPES 50 mM pH 7.0, $MgCl_2$ 10 mM, 1 mM DTT, 0.2 mg/ml BSA, 3μM orthovanadate) were added to each well of a 96 U bottom well plate. After 30 minutes at room temperature incubation, reaction was stopped and biotinylated peptide captured by adding 100 μl of bead suspension.

**Stratification:** 100 μl of CsCl2 5 M were added to each well and let stand 4 hour before radioactivity was counted in the Top-Count instrument.

**IC50 determination:** see above

**Inhibition assay of Cdc7/dbf4 activity**

The inhibition assay of Cdc7/dbf4 activity was performed according to the following protocol.
The Biotin-MCM2 substrate is trans-phosphorylated by the Cdc7/Dbf4 complex in the presence of ATP traced with $\gamma^{33}$-ATP. The phosphorylated Biotin-MCM2 substrate is then captured by Streptavidin-coated SPA beads and the extent of phosphorylation evaluated by β counting.
The inhibition assay of Cdc7/dbf4 activity was performed in 96 wells plate according to the following protocol.
To each well of the plate were added:

- 10 μl substrate (biotinylated MCM2, 6 μM final concentration)
- 10 μl enzyme (Cdc7/Dbf4, 12.5 nM final concentration)
- 10 μl test compound (12 increasing concentrations in the nM to μM range to generate a dose-response curve)
- 10 μl of a mixture of cold ATP (10 μM final concentration) and radioactive ATP (1/2500 molar ratio with cold ATP.) was then used to start the reaction which was allowed to take place at 37°C.

Substrate, enzyme and ATP were diluted in 50 mM HEPES pH 7.9 containing 15 mM $MgCl_2$, 2 mM DTT, 3 μM $NaVO_3$, 2mM glycerophosphate and 0.2mg/ml BSA. The solvent for test compounds also contained 10% DMSO.
After incubation for 20 minutes, the reaction was stopped by adding to each well 100 μl of PBS pH 7.4 containing 50 mM EDTA, 1 mM cold ATP, 0.1% Triton X100 and 10 mg/ml streptavidin coated SPA beads.
After 15 minutes of incubation at room temperature to allow the biotinylated MCM2-streptavidin SPA beads interaction to occur, beads were trapped in a 96 wells filter plate (Unifilter$^{R}$ GF/B$^{TM}$) using a Packard Cell Harvester (Filtermate), washed with distilled water and then counted using a Top Count (Packard).
Counts were blank-subtracted and then the experimental data (each point in triplicate) were analyzed for IC50 determination using a non-linear regression analysis (Sigma Plot).
The compounds of formulae (Ic), (Ie), (If) and (Ig) of the present invention, suitable for administration to a mammal, e.g. to humans, can be administered by the usual routes and the dosage level depends upon the age, weight, conditions of the patient and the administration route. For example, a suitable dosage adopted for oral administration of a compound of formulae (Ic), (Ie), (If) and (Ig) may range from about 10 to about 500 mg pro dose, from 1 to 5 times daily.
The compounds of the invention can be administered in a variety of dosage forms, e.g. orally, in the form of tablets, capsules, sugar or film coated tablets, liquid solutions or suspensions; rectally in the form of suppositories; parenterally, e.g. intramuscularly, or by intravenous and/or intrathecal and/or intraspinal injection or infusion.
In addition, the compounds of the invention can be administered either as single agents or, alternatively, in combination with known anticancer treatments such as radiation therapy or chemotherapy regimen in combination with cytostatic or cytotoxic agents, antibiotic-type agents, alkylating agents, antimetabolite agents, hormonal agents, immunological agents, interferon-type agents, cyclooxygenase inhibitors (e.g. COX-2 inhibitors), metallomatrixprotease inhibitors, telomerase inhibitors, tyrosine kinase inhibitors, anti-growth factor receptor agents, anti-HER agents, anti-EGFR agents, anti-angiogenesis agents, farnesyl transferase inhibitors, ras-raf signal transduction pathway inhibitors, cell cycle inhibitors, other cdks inhibitors, tubulin binding agents, topoisomerase I inhibitors, topoisomerase II inhibitors, and the like.
As an example, the compounds of the invention can be administered in combination with one or more chemotherapeutic agents such as, for instance, exemestane, formestane, anastrozole, letrozole, fadrozole, taxane, taxane derivatives, encapsulated taxanes, CPT-11, camptothecin derivatives, anthracycline glycosides, e.g., doxorubicin, idarubicin, epirubicin, etoposide, navelbine, vinblastine, carboplatin, cisplatin, estramustine phosphate, celecoxib, tamoxifen, raloxifen, Sugen SU-5416, Sugen SU-6668, Herceptin, and the like, optionally within liposomal formulations thereof.
If formulated as a fixed dose, such combination products employ the compounds of this invention within the dosage range described above and the other pharmaceutically active agent within the approved dosage range.
Compounds of formulae (Ic), (Ie), (If) and (Ig) may be used sequentially with known anticancer agents when a combination formulation is inappropriate.
The present invention also includes pharmaceutical compositions comprising a compound of formulae (Ic), (Ie), (If) and (Ig) or a pharmaceutically acceptable salt thereof in association with a pharmaceutically acceptable excipient (which can be a carrier or a diluent).
The pharmaceutical compositions containing the compounds of the invention are usually prepared following conventional methods and are administered in a pharmaceutically suitable form.
For example, the solid oral forms may contain, together with the active compound, diluents, e.g. lactose, dextrose, saccharose, sucrose, cellulose, com starch or potato starch; lubricants, e.g. silica, talc, stearic, magnesium or calcium stearate, and/or polyethylene glycols; binding agents, e.g. starches, arabic gum, gelatin, methylcellulose, carboxymethylcellulose or polyvinyl pyrrolidone; disaggregating agents, e.g. a starch, alginic, alginates or sodium starch glycolate; effervescing mixtures; dyestuffs; sweeteners; wetting agents such as lecithin, polysorbates, laurylsulfates;

and, in general, non-toxic and pharmacologically inactive substances used in pharmaceutical formulations. Said pharmaceutical preparations may be manufactured in known manner, for example, by means of mixing, granulating, tabletting, sugar-coating, or film-coating processes.

The liquid dispersions for oral administration may be e.g. syrups, emulsions and suspensions.

The syrups may contain as carrier, for example, saccharose or saccharose with glycerin and/or mannitol and/or sorbitol.

The suspensions and the emulsions may contain as carrier, for example, a natural gum, agar, sodium alginate, pectin, methylcellulose, carboxymethylcellulose, or polyvinyl alcohol.

The suspension or solutions for intramuscular injections may contain, together with the active compound, a pharmaceutically acceptable carrier, e.g. sterile water, olive oil, ethyl oleate, glycols, e.g. propylene glycol, and, if desired, a suitable amount of lidocaine hydrochloride. The solutions for intravenous injections or infusions may contain as carrier, for example, sterile water or preferably they may be in the form of sterile, aqueous, isotonic saline solutions or they may contain as a carrier propylene glycol.

The suppositories may contain together with the active compound a pharmaceutically acceptable carrier, e.g. cocoa butter, polyethylene glycol, a polyoxyethylene sorbitan fatty ester surfactant or lecithin.

The following examples are herewith intended to better illustrate the present invention without posing any limitation to it.

## Example 1

**Ethyl 1-(4-methoxyphenyl)-1,4,5,6-tetrahydropyrazolo[3,4-e]indazole-3-carboxylate**

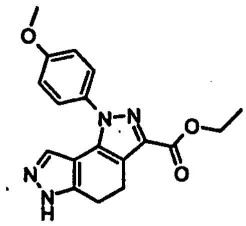

**Step 1:** Hydrazine hydrochloride (6.28 g, 59.8 mmols) was suspended in methanol (100 ml) and treated with 2N NaOH solution (90 ml, 3 eq). 2-[(dimethylamino)methylene]-cyclohexane-1,3-dione (10 g, 59.8 mmols) was then added and the mixture was kept at 80°C for 3 hours. After cooling, the mixture was neutralised with N HCl and evaporated to dryness. The solid was extracted with ethyl acetate (100 ml x 3) at 50°C. The extracts were collected and evaporated to give pure 1,5,6,7-tetrahydro-4H-indazol-4-one (8.03 g, Y=98%) as a yellow crystalline solid
$^1$H NMR ($CDCl_3$ / 300 MHz) 2.16 (m, 2H); 2.52 (m, 2H); 2.90 (t, 2H); 8.00 (s, 1H).

**Step 2:** To a suspension of 1,5,6,7-tetrahydro-4H-indazol-4-one (8 g, 58.75 mmols) and trityl chloride (18.02 g, 64.64 mmols) in dichloromethane (160 ml), triethylamine (9.8 ml, 70.50 mmols) was added dropwise. The reaction was slightly exothermic.

After stirring overnight, the organic layer was washed with water, dried over MgSO4 and evaporated to dryness. The crude material was taken up with hexane, kept under vigorous stirring for 15 minutes and filtered on buchner to give 2-trityl-2,5,6,7-tetrahydro-4H-indazol-4-one (21 g, Y=94%)
$^1$H NMR ($CDCl_3$ / 300 MHz) 2.14 (m, 2H); 2.48 (m, 2H); 2.89 (m, 2H); 7.13 (m, 6H); 7.32 (m, 9H); 7.87 (s, 1H).

**Step 3:** To a suspension of 2-trityl-2,5,6,7-tetrahydro-4H-indazol-4-one (20 g, 52.84 mmols) and ethyl oxalate (7.88 ml, 58.13 mmols) in ethyl ether (150 ml), lithium bis(trimethylsilyl)amide 1M in THF (56.54 ml) was added dropwise. The slurry was stirred overnight, poured into a 20% $NaH_2PO_4$ solution (200 ml) and extracted with ethyl acetate. The extracts were washed with brine, dried over $MgSO_4$ and evaporated to dryness. The residue was taken up with ethanol and filtered to give ethyl oxo(4-oxo-2-trityl-4,5,6,7-tetrahydro-2H-indazol-5-yl)acetate as a pink solid (23.5 g, Y=93%)
$^1$H NMR ($CDCl_3$ / 300 MHz) 1.40 (t, 3H); 2.86 (m, 2H); 3.07 (m, 2H); 4.35 (q, 2H); 7.14 (m, 6H), 7.33 (m, 9H); 7.91 (s, 1H).

**Step 4:** A suspension of ethyl oxo(4-oxo-2-trityl-4,5,6,7-tetrahydro-2H-indazol-5-yl)acetate (400 mg, 0.84 mmols) and (4-methoxyphenyl)-hydrazine hydrochloride (164 mg, 0.94 mmols) in acetic acid (4 ml) was stirred at 65°C for 3 hours. After cooling, the resulting suspension was filtered on buchner and washed, in sequence, with acetic acid, ethyl ether and water to obtain ethyl 1-(4-methoxyphenyl)-1,4,5,6-tetrahydropyrazolo[3,4-e]indazole-3-carboxylate (230 mg, Y=81%) as white solid
$^1$H NMR (DMSO-$d_6$/ 400 MHz) 1.25 (t, 3H; 2.83-3.15 (m, H); 3.82 (s, 3H); 4.23 (q, 2H);7.16 (d, 2H); 7.46 (d, 2H).

By working according to an analogous procedure, the following compounds were prepared:

Ethyl 1-[4-(aminosulfonyl)phenyl]-1,4,5,6-tetrahydropyrazolo[3,4-e]indazole-3-carboxylate
$^1$H NMR (DMSO-$d_6$/ 400 MHz) 1.3 (t, 3H); 2.8-3.1 (2d, 4H); 4.3 (q, 2H); 7.35 (s, 1H); 7.5 (d, 2H); 7.8-8.1 (2d, 4H);
Ethyl 1-{4-[(methylamino)sulfonyl]phenyl}-1,4,5,6-tetrahydropyrazolo[3,4-e]indazole-3-carboxylate
$^1$H NMR (DMSO-$d_6$/ 400 MHz) 1.26 (t, 3H); 2.43 (m, 3H); 2.85-3.09 (m, 4H); 4.31 (q, 2H); 7.39 (bs, 1H); 7.58 (q, 1H); 7.87 (d, 2H); 7.97 (d, 2H);
Ethyl 1-{4-[(butylamino)sulfonyl]phenyl}-1,4,5,6-tetrahydropyrazolo[3,4-e]indazole-3-carboxylate
$^1$H NMR (DMSO-$d_6$/ 400 MHz) 0.79 (t, 3H);1.13-1.39 (m, 7H); 2.79 (q, 2H); 2.83 (t, 2H); 3.07 (t, 2H); 4.31 (q, 2H); 7.32 (s, 1H); 7.71 (t, 1H); 7.85 (d, 2H); 7.88 (d, 2H);
Ethyl 1-{4-[(dimethylamino)sulfonyl]phenyl}-1,4,5,6-tetrahydropyrazolo[3,4-e]indazole-3-carboxylate
$^1$H NMR (DMSO-$d_6$/ 400 MHz) 1.25 (t, 3H); 2.62 (s, 6H); 2.89 (t, 2H); 3.07 (t, 2H); 4.26 (q, 2H); 7.41 (s, 1H); 7.89-7.96 (m, 4H);
Ethyl 1-{4-[(diprop-2-ynylamino)sulfonyl]phenyl}-1,4,5,6-tetrahydropyrazolo[3,4-e]indazole-3-carboxylate
$^1$H NMR (DMSO-$d_6$/ 400 MHz) 1.25 (t, 3H); 2.85-3.1 (m, 4H); 3.2 (s, 2H); 4.18 (d, 4H); 4.35 (q, 2H); 7.35 (s, 1H); 7.89-8.1 (2d, 4H);
Ethyl 1-[4-(anilinosulfonyl)phenyl]-1,4,5,6-tetrahydropyrazolo[3,4-e]indazole-3-carboxylate
$^1$H NMR (DMSO-$d_6$/ 400 MHz) 1.24 (t, 3H); 2.86 (t, 2H); 3.04 (t, 2H); 4.29 (q, 2H); 7.01-7.25 (m, 6H); 7.79 (d, 2H); 7.85 (d, 2H);
Ethyl 1-[4-(methylsulfonyl)phenyl]-1,4,5,6-tetrahydropyrazolo[3,4-e]indazole-3-carboxylate;
$^1$H NMR (DMSO-$d_6$/ 400 MHz) 1.31 (t, 3H); 2.89 (t, 2H); 3.07 (t, 2H); 4.31 (q, 2H); 7.40 (s, 1H); 7.91 (d, 2H); 8.12 (d, 2M;
Ethyl 1-(4-{[(2-hydroxypropyl)amino]sulfonyl}phenyl)-1,4,5,6-tetrahydropyrazolo-[3,4-e]indazole-3-carboxylate
$^1$H NMR (DMSO-$d_6$/ 400 MHz) 1.01 (d, 3 H); 1.25 (t, 3 H); 2.75 (m, 2 H); 2.85-3.1 (2 t, 4 H); 3.6 (dd, 1 H); 4.35 (dd, 1 H); 7.35 (s, 1 H); 7.7 (t, 1 H); 7.85-8.05 (2d, 4 H);
Ethyl 1-[4-(aminocarbonyl)phenyl]-1,4,5,6-tetrahydropyrazolo[3,4-e]indazole-3-carboxylate
$^1$H NMR (DMSO-$d_6$/ 400 MHz) 1.31 (t, 3H); 2.88 (t, 2H); 3.07 (t, 2H); 4.31 (q, 2H); 7.21 (bs, 1H); 7.43 (s, 1H); 7.69 (d; 2H); 8.07 (d, 2H); 8.19 (s, 1H);
Ethyl 1,4,5,6-tetrahydropyrazolo[3,4-e]indazole-3-carboxylate
$^1$H NMR (DMSO-$d_6$/ 400 MHz) 1.3 (t, 3H); 2.8-3.05 (2t, 4 H); 4.3 (m, 2H); 7.7 (bs, 1 H);
Ethyl 1-phenyl-1,4,5,6-tetrahydropyrazolo[3,4-e]indazole-3-carboxylate
$^1$H NMR (DMSO-$d_6$/ 400 MHz) 1.3 (t, 3H); 2.86 (m, 2H); 3.07 (m, 2H); 4.29 (q, 2H); 7.47 (s, 1H); 7.47 (s, 1H); 7.54-7.60 (m, 5H);
Ethyl 1-(4-fluorophenyl)-1,4,5,6-tetrahydropyrazolo[3,4-e]indazole-3-carboxylate
$^1$H NMR (DMSO-$d_6$/ 400 MHz) 1.3 (t, 3H); 2.85-3.1 (m, 4 H); 4.3 (q, 2H); 7.2 (s, 1H); 7.4-7.7 (m, 4H);
Ethyl 1-(4-bromophenyl)-1,4,5,6-tetrahydropyrazolo[3,4-e]indazole-3-carboxylate
$^1$H NMR (DMSO-$d_6$/ 400 MHz) 1.30 (t, 3H), 2.88 (t, 2H), 3.04 (t, 2H), 4.29 (q, 2H); 7.27 (s, 1H); 7.57 (d, 2H); 7.78 (d, 2H);
Ethyl 1-(4-methylphenyl)-1,4,5,6-tetrahydropyrazolo[3,4-e]indazole-3-carboxylate
$^1$H NMR (DMSO-$d_6$/ 400 MHz) 1.3 (t, 3 H); 2.4 (s, 3H); 2.8-3.1 (2t, 4H); 4.3 (q, 2H); 7.1 (bs, 1H); 7.4-7.5 (2d, 4H);
Ethyl 1-(4-chlorophenyl)-1,4,5,6-tetrahydropyrazolo[3,4-e]indazole-3-carboxylate
$^1$H NMR (DMSO-$d_6$/ 400 MHz) 1.3 (t, 3 H); 2.8-3.1 (m, 4 H); 4.3 (q, 2H); 7.35 (bs, 1H); 7.65 (s, 4H);
Ethyl 1-(4-cyanophenyl)-1,4,5,6-tetrahydropyrazolo[3,4-e]indazole-3-carboxylate
$^1$H NMR (DMSO-$d_6$/ 400 MHz) 1.31 (t, 3H); 2.88 (m, 2H); 3.05 (m, 2H); 4.31 (q, 2H); 7.25 (s, 1H); 7.85 (d, 2H); 8.06 (d, 2H);
Ethyl 1-(4-nitrophenyl)-1,4,5,6-tetrahydropyrazolo[3,4-e]indazole-3-carboxylate
$^1$H NMR (DMSO-$d_6$/ 400 MHz) 1.31(t, 3H); 2.89 (m, 2H), 3.05 (t, 2H); 4.32 (q, 2H); 7.47 (s, 1H); 7.93 (d, 2H); 8.43 (d, 2H);
Ethyl 1-[4-(trifluoromethyl)phenyl]-1,4,5,6-tetrahydropyrazolo[3,4-e]indazole-3-carboxylate
$^1$H NMR (DMSO-$d_6$/ 400 MHz) 1.31 (t, 3H); 2.87 (m, 2H); 3.09 (M, 2H); 4.32 (q, 2H); 7.42 (bs, 1H); 7.47 (s, 1H); 7.87 (d, 2H); 7.96 (d, 2H);
Ethyl 1-benzyl-1,4,5,6-tetrahydropyrazolo[3,4-e]indazole-3-carboxylate
$^1$H NMR (DMSO-$d_6$/ 400 MHz) 1.3 (t, 3H); 2.8-3.05 (m, 4 H); 4.2 (q, 2H); 5.5 (s, 1H); 7.1-7.25 (m, 5H);
Ethyl 1-(3-hydroxybenzyl)-1,4,5,6-tetrahydropyrazolo[3,4-e]indazole-3-carboxylate
$^1$H NMR (DMSO-$d_6$/ 400 MHz) 1.3 (t, 3H); 2.8-3.05 (2t, 4 H); 4.25 (q, 2H); 5.4 (s, 2H); 6.5 (s, 1H); 6.7 (m, 2H); 7.1 (t, 1H); 7.8 (bs, 1H); 9.4 (s, 1 H);
Ethyl 1-pyridin-2-yl-1,4,5,6-tetrahydropyrazolo[3,4-e]indazole-3-carboxylate
$^1$H NMR (DMSO-$d_6$/ 400 MHz) 1.3 (t, 3H); 2.8-3.1 (2t, 4 H); 4.35 (q, 2H); 7.45 (t, 1H); 7.9-8.1 (d + t, 2 H); 8.15 (bs, 1 H); 8.6 (d, 1H);
Ethyl 1-(6-chloropyridazin-3-yl)-1,4,5,6-tetrahydropyrazolo[3,4-e]indazole-3-carboxylate

$^1$H NMR (DMSO-$d_6$/ 400 MHz) 1.35 (t, 3H); 2.9-3.1 (2t, 4H); 4.38 (dd, 2H); 8.1(s, 1H); 8.15-8.25 (2d, 2H);
Ethyl 1-[4-(trifluoromethyl)pyrimidin-2-yl]-1,4,5,6-tetrahydropyrazolo[3,4-e]indazole-3-carboxylate
$^1$H NMR (DMSO-$d_6$/ 400 MHz) 1.3 (t, 3H); 2.85-3.1 (2t, 4H); 4.35 (q, 2H); 8.15 (s, 1H); 8.2-9.4 (2d, 2H);
Ethyl 1-(3-methylphenyl)-1,4,5,6-tetrahydropyrazolo[3,4-e]indazole-3-carboxylate
$^1$H NMR (DMSO-$d_6$/ 400 MHz) 1.3 (t, 3H); 2.4 (s, 3H); 2.8-3.1 (2t, 4H); 4.3 (q, 2H); 7.2 (bs, 1H); 7.35-7.5 (m, 4H);
Ethyl 1-(3-chlorophenyl)-1,4,5,6-tetrahydropyrazolo[3,4-e]indazole-3-carboxylate
$^1$H NMR (DMSO-$d_6$/ 400 MHz) 1.3 (t, 3H); 2.8-3.1 (2m, 4H); 4.3 (q, 2H); 7.3 (s, 1H); 7.6, 7.7 (2s, 4H);
Ethyl 1-(3-fluorophenyl)-1,4,5,6-tetrahydropyrazolo[3,4-e]indazole-3-carboxylate
$^1$H NMR (DMSO-$d_6$/ 400 MHz) 1.3 (t, 3H); 2.8-3.1 (2m, 4H); 4.3 (q, 2H); 7.3 (s, 1H); 7.5-7.7 (m, 4H);
Ethyl 4,4-dimethyl-1-(4-methylphenyl)-1,4;s,6-tetrahydropyrazolo[3,4-e]indazole-3-carboxylate
$^1$H NMR (DMSO-$d_6$/ 400 MHz) 1.29 (t, 3H); 1.33 (s, 6H); 2.40 (s, 3H); 2.73 (s, 2H); 4.28 (q, 2H); 7.02 (bs, 1H); 7.43 (dd, 4H); 12.05 (bs, 1H).
Ethyl 1-pyridin-3-yl-1,4,5,6-tetrahydropyrazolo[3,4-e]mdazole-3-carboxylate
$^1$HNMR (DMSO-d6/ 400 MHz); 1.3 (t,3H); 2.85 (m,2H); 3.15 (t,2H); 4.31 (q, 2H); 7.21 (bs, 1H); 7.6 (m, 1H); 8.15 (d, 1H); 8.75 (d, 1H); 8.85 (s,1H); 12.7 (bs, 1H)
Ethyl 1-[4-(acetylamino)phenyl]-1,4,5,6-tetrahydropyrazolo[3,4-e]indazole-3-carboxylate
$^1$HNMR (DMSO-d6/ 400 MHz); 1.25 (t,3H); 2.05 (s,3H); 2.8-3.05 (2 t,4H); 4.31 (q, 2H); 7.15 (s,1H); 7.45 (d, 2H); 7.8 (d, 2H); 10.21 (s, 1H)
Ethyl 1-{4-[(4-methylpiperazin-1-yl)sulfonyl]phenyl}-1,4,5,6-tetrahydropyrazolo[3,4-e]indazole-3-carboxylate
$^1$H NMR (DMSO-$d_6$/ 400 MHz); 1.30 (t, 3H); 2.40 (s, 3H); 2.65-3.30 (m, 12H); 4.31 (q, 2H); 7.33 (bs, 1H); 7.95 (m, 4H)
4-[3-(ethoxycarbonyl)-5,6-dihydropyrazolo[3,4-e]indazol-1(4H)-yl]benzoic acid
$^1$H NMR (DMSO-$d_6$/ 400 MHz); 1.31 (t, 3H); 2.89 (t, 2H); 3.07 (t, 3H); 4.31 (q, 2H); 7.34 (s, 1H); 7.76 (dd, 2H); 8.14 (dd, 2H)
Ethyl 1-[4-(trifluoromethoxy)phenyl]-1,4,5,6-tetrahydropyrazolo[3,4-e]indazole-3-carboxylate
$^1$H NMR (DMSO-$d_6$/ 400 MHz); 1.30 (t, 3H); 2.88 (bt, 2H); 3.06 (t, 2H); 4.29 (q, 2H); 7.26 (bs,1H); 7.58 (bd, 2H); 7.75 (bd, 2H)
Ethyl 1-butyl-1,4,5,6-tetrahydropyrazolo[3,4-e]indazole-3-carboxylate
$^1$H NMR (DMSO-$d_6$/ 400 MHz); 0.85 (t, 3H); 1.28 (m, 5H); 1.71 (m, 2H); 2.80 (bt, 2H); 2.97 (bt, 2H); 4.25 (q, 2H); 8.04 (br, 1H)
Ethyl 1-(2,5-dimethylphenyl)-1,4,5,6-tetrahydropyrazolo[3,4-e]indazole-3-carboxylate
$^1$H NMR (DMSO-$d_6$/ 400 MHz); 1.29 (t, 3H); 1.91 (s, 3H); 2.33 (s, 3H); 2.85 (bt, 2H); 3.08 (bt, 2H); 4.27 (q, 2H); 6.64 (bs, 1H); 7.18 (bs, 1H); 7.34 (dd, 2H)
Ethyl 1-{4-[amino(imino)methyl]phenyl}-1,4,5,6-tetrahydropyrazolo[3,4-e]indazole-3-carboxylate hydrochloride
$^1$H NMR (DMSO-$d_6$/ 400 MHz); 1.31 (t, 3H); 2.90 (bt, 2H); 3.07 (b, 2H); 4.30 (q, 2H); 7.19 (s, 1H); 7.89 (d, 2H); 8.01 (d, 2H); 9.03 (bs, 2H); 9.44 (bs, 2H)
Ethyl 1-[4-(1H-imidazol-2-yl)phenyl]-1,4,5,6-tetrahydropyrazolo[3,4-e]indazole-3-carboxylate hydrochloride
$^1$H NMR (DMSO-$d_6$/ 400 MHz); 1.31 (t, 3H); 2.90 (t, 2H); 3.08 (t, 2H); 4.30 (q, 2H); 7.19 (s,1H);7.86 (s, 2H); 7.94 (d, 2H); 8.24 (d, 2H)
Ethyl 1-methyl-1,4,5,6-tetrahydropyrazolo[3,4-e]indazole-3-carboxylate
$^1$H NMR (DMSO-$d_6$/ 400 MHz); 1.31 (t, 3H); 2.82 (bt, 2H); 3.00 (bt, 2H); 3.98 (s, 3H); 4.27 (q, 2H); 8:13 (bs, 1H)

**Example 2**

**Ethyl 8-anilino-1-methyl-1,4,5,6-tetrahydropyrazolo[3,4-e]indazole-3-carboxylate**

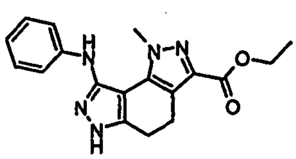

**Step 1:** A solution of 3-Ethoxy-cyclohex-2-enone (4.65 ml, 31.92 mmols) and diethyl oxalate (6.49 ml, 47.89 mmols) in anhydrous ethyl ether (50 ml) is treated dropwise with a 1M solution of lithium bis(trimethylsilyl)amide in tetrahydrofuran (47.9 ml, 47.9 mmols) under argon atmosphere. After standing at room temperature overnight, the mixture is poured into a 20% NaH2PO4 solution (150 ml) and extracted with-ethyl acetate (100 ml x2). The organic

exctracts are washed with brine, dried on Na2SO4 and evaporated to dryness to afford crude ethyl (4-ethoxy-2-oxocyclohex-3-en-1-yl)(oxo)acetate (8 g) as an orange oil which is used for the next step without further purification.

**Step 2:** (4-Ethoxy-2-oxo-cyclohex-3-enyl)-oxo-acetic acid ethyl ester (8 g, 31.92 mmols theoretically) is treated with methylhydrazine (1.69 ml, 31.92 mmol) in EtOH (75 ml) and AcOH (5 ml) at room temperature. After 3 hours the solution was concentrated and the precipitate was collected to afford ethyl 6-ethoxy-1-methyl-4,5-dihydro-1H-indazole-3-carboxylate (7.59 g, Y=95%).

1H NMR (400 MHz, DMSO-D6) δ ppm 1.28 (t, *J*=7.07 Hz, 3 H) 1.31 (t, *J*=7.01 Hz, 3 H) 2.42 (t, *J*=8.60 Hz, 2 H) 2.85 (t, *J*=8.66 Hz, 2 H) 3.76 (s, 3 H) 3.93 (td, *J*=7.07, 6.83 Hz, 2 H) 4.23 (q, *J*=7.15 Hz, 2 H) 5.74 (s, 1H)

**Step 3:** Ethyl 6-ethoxy-1-methyl-4,5-dihydro-1H-indazole-3-carboxylate (7.59 g, 30.36 mmols) was dissolved in dioxane (50 ml) and treated with HCl 2N (17 ml) overnight. The solution was concentrated, diluted with water and extracted with ethyl acetate. The organic phase was washed with brine, dried over sodium sulfate and evaporated to afford ethyl 1-methyl-6-oxo-4,5,6,7-tetrahydro-1H-indazole-3-carboxylate (5.6 g, Y=83%).

1H NMR (400 MHz, DMSO-D6) δ ppm 1.30 (t, *J*=7.07 Hz, 3 H) 2.58 (t, *J*=6.89 Hz, 2 H) 2.99 (t, *J*=6.89 Hz, 2 H) 3.61 (s, 2 H) 3.76 (s, 3 H) 4.26 (q, *J*=7.07 Hz, 2 H)

**Step 4:** A solution of ethyl 1-methyl-6-oxo-4,5,6,7-tetrahydro-1H-indazole-3-carboxylate (2.66 g, 12 mmol) in DMF (45 ml) was treated with lithium bis(trimethylsilyl)amide 1M in THF (13.2 ml, 13.2 mmol) at -40°C. After 15 minutes phenyl isothiocyanate (1.58 ml, 13.2 mmol) was added, dropwise.

After further 30 minutes the reaction mixture was treated with a 20% solution of sodium dihydrogen phosphate. The precipitate was filtered and washed with water to afford ethyl 7-(anilinocarbonothioyl)-1-methyl-6-oxo-4,5,6,7-tetrahydro-1H-indazole-3-carboxylate (3.11 g, Y=72%).

1H NMR (400 MHz, DMSO-D6) δ ppm 1.32 (t, *J*=7.07 Hz, 1 H) 2.90 (m, 4 H) 3.70 (s, 3 H) 4.30 (q, *J*=7.07 Hz, 2 H) 5.19 (s, 1 H) 7.32 (t, *J*=7.44 Hz, 1H) 7.46 (t, *J*=7.93 Hz, 2 H) 7.81 (d, *J*=7.56 Hz, 2 H) 12.27 (s, 1H)

**Step 5:** A suspension of ethyl 7-(anilinocarbonothioyl)-1-methyl-6-oxo-4,5,6,7-tetrahydro-1H-indazole-3-carboxylate (3.10 g, 8.7 mmol) in EtOH (50 ml) and AcOH (0.5 ml) was treated with hydrazine hydrate (0.5 ml, 10.3 mmol) for 30 minutes under reflux. After cooling the white precipitated was collected by filtration to give ethyl 8-anilino-1-methyl-1,4,5,6-tetrahydropyrazolo[3,4-e]indazole-3-carboxylate (2.2 g, Y=75%).

1H NMR (400 MHz, DMSO-D6) δ ppm 1.30 (t, *J*=7.07 Hz, 1 H) 2.90 (m, 4 H) 3.83 (s, 3 H) 4.25 (q, *J*=7.07 Hz, 2 H) 7.32 (t, *J*=7.44 Hz, 1 H) 7.46 (t, *J*=7.93 Hz, 2 H) 7.81 (d, *J*=7.56 Hz, 2 H) 8.07 (s, 1 H) 12.79 (s, 1 H)

By working analogously, the following compounds were prepared:

Ethyl 8-anilino-1-(2,2,2-trifluoroethyl)-1,4,5,6-tetrahydropyrazolo[3,4-e]indazole-3-carboxylate
1H NMR (400 MHz, DMSO-D6) δ ppm 1.32 (t, *J*=7.07 Hz, 1 H) 2.90 (m, 4 H) 4.32 (q, *J*=7.07 Hz, 2 H) 5.31 (m, 2H) 6.7 (m, 3 H) 7.14 (m, 2 H) 8.07 (s, 1 H) 12.7 (s, 1 H)

Ethyl 8-anilino-2-{2-[(tert-butoxycarbonyl)amino]ethyl}-2,4,5,6-tetrahydropyrazolo[3,4-e]indazole-3-carboxylate
1H NMR (400 MHz, DMSO-D6) δ ppm 1.36 (s, 9 H), 1.36 (t, *J*=7.07 Hz, 1H) 2.82 (m, 2 H) 3.05 (m, 2 H) 3.33 (m, 1 H), 4.33 (q, *J*=7.07 Hz, 2 H) 4.50 (m, 2 H), 6.77 (m, 2H) 7.21 (m, 3 H) 7.45 (m, 2 H) 12.11 (bs, 1 H)

Ethyl 8-amino-1,4,5,6-tetrahydropyrazolo[3,4-e]indazole-3-carboxylate
1H NMR (400 MHz, DMSO-D6) δ ppm 1.34 (t, 3H), 2.87 (t, 2H), 3.05 (t, 2H), 4.32 (q, 2H)

## Example 3

**1-(4-methoxyphenyl)-1,4,5,6-tetrahydro-pyrazolo[3,4-e]indazole-3-carboxamide**

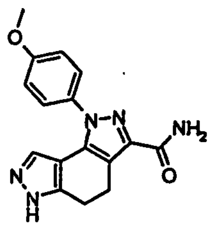

A suspension of ethyl 1-(4-methoxyphenyl)-1,4,5,6-tetrahydropyrazolo[3,4-e]indazole-3-carboxylate (200 mg; 0.59 mmols) in concentrated ammonium hydroxide (5 ml) and methanol (2.5 ml) was heated in a sealed tube at 65°C for 8 hours. The mixture was then diluted with water and filtered to give 1-(4-methoxyphenyl)-1,4,5,6-tetrahydropyrazolo

[3,4-e]indazole-3-carboxamide (137 mg; Y=75%) as a white solid
$^1$H NMR (DMSO-$d_6$/ 400 MHz) 3.06 (t, 2H); 3.83 (s, 3H); 7.09-7.11 (m, 3H); 7.20 (s, 1H); 7.40 (s, 1H); 7.50 (m, 2H).

By working analogously, the following compounds were prepared:

1-[4-(aminosulfonyl)phenyl]-1,4,5,6-tetrahydropyrazolo[3,4-e]indazole-3-carboxamide
$^1$H NMR (DMSO-$d_6$/ 400 MHz) 2.8-3.2 (m, 4H); 7.3-7.5 (2s, 2H); 7.4 (s, 1H); 7.45 (d, 2H); 7.8-8.05 (2d, 4H);
1-{4-[(methylamino)sulfonyl]phenyl}-1,4,5,6-tetrahydropyrazolo[3,4-e]indazole-3-carboxamide
$^1$H NMR (DMSO-$d_6$/ 400 MHz) 2.47 (m, 3H; 2.81-3.07 (m, 4H); 7.25 (s, 2H); 7.41 (s, 1H); 7.45 (s, 2H); 7.54 (q, 1H); 7.88-7.97 (m, 4H);
1-{4-[(butylamino)sulfonyl]phenyl}-1,4,5,6-tetrahydropyrazolo[3,4-e]indazole-3-carboxamide
$^1$H NMR (DMSO-$d_6$/ 400 MHz) 0.79 (t, 3H); 1.20 (q, 2H); 1.40 (q, 2H); 2.79-2.90 (m, 4H); 3.06 (t, 2H); 7.23-7.55 (m, 3H); 7.65 (t, 1H); 7.85-7.98 (m, 4H);
1-{4-[(dimethylamino)sulfonyl]phenyl}-1,4,5,6-tetrahydropyrazolo[3,4-e]indazole-3-carboxamide
$^1$H NMR (DMSO-$d_6$/ 400 MHz) 2.66 (s, 6H); 3.15 (t, 2H); 7.20-7.50 (m, 3H); 7.95 (m, 4H);
1-{4-[(diprop-2-ynylamino)sulfonyl]phenyl}-1,4,5,6-tetrahydropyrazolo[3,4-e]indazole-3-carboxamide
$^1$H NMR (DMSO-$d_6$/ 400 MHz) 2.8-3.1 (2t, 4H); 3.25 (s, 2H); 4.20 (s, 4H); 7.25-7.45 (2s, 2H); 7.39 (s, 4H); 7.95-8.1 (2d, 4H);
1-[4-(anilinosulfonyl)phenyl]-1,4,5,6-tetrahydropyrazolo[3,4-e]indazole-3-carboxamide
$^1$H NMR (DMSO-$d_6$/ 400 MHz) 2.75-3.10 (m, 4H); 7.0-7.45 (m, 8H); 7.80 (d, 2H); 7.85 (d, 2H); 10.25 (s, 1H);
1-[4-(methylsulfonyl)phenyl]-1,4,5,6-tetrahydropyrazolo[3,4-e]indazole-3-carboxamide
$^1$H NMR (DMSO-$d_6$/ 400 MHz) 2.80 (m, 2H); 3.10 (m, 2H); 3.29 (s, 3H); 7.30 (s, 2H); 7.49 (s, 1H); 7.50 (s, 2H); 7.93 (d, 2H); 8.12 (d, 2H);
1-[4-(aminocarbonyl)phenyl]-1,4,5,6-tetrahydropyrazolo[3,4-e]indazole-3-carboxamide
$^1$H NMR (DMSO-$d_6$/ 400 MHz) 2.85 (m, 2H); 3.07 (t, 2H); 7.20 (s, 1H); 7.73 (d, 2H); 8.07 (d, 2H);
1-(4-{[(2-hydroxypropyl)ammo]sulfonyl}phenyl)-1,4,5,6-tetrahydropyrazolo[3,4-e]indazole-3-carboxamide
$^1$H NMR (DMSO-$d_6$/ 400 MHz) 1.02 (d, 3H); 2.72 (m, 2H); 2.8-3.15 (m, 4H); 3:6 (m, 1H); 4.65 (d, 1H); 7.25-7. 5 (2d, 2H); 7.4 (s, 1H); 7.65 (s, 1H); 7.85 (d, 2H); 8.01 (d, 2H);
1,4,5,6-tetrahydropyrazolo[3;4-e]indazole-3-carboxamide
$^1$H NMR (DMSO-$d_6$/ 400 MHz) 2.79 (bs, 2H); 2.98 (m, 2H); 7.28 (bs, 1H);
1-phenyl-1,4,5,6-tetrahydropyrazolo[3,4-e]indazole-3-carboxamide
$^1$H NMR (DMSO-$d_6$/ 400 MHz) 2.82 (m, 2H); 3.09 (t, 2H); 7.20 (bs, 1H); 7.21 (bs, 1H); 7.43 (bs, 1H); 7.45-7.63 (m, 5H);
1-(4-fluorophenyl)-1,4,5,6-tetrahydropyrazolo[3,4-e]indazole-3-carboxamide
$^1$H NMR (DMSO-$d_6$/ 400 MHz) 2.8-3.1 (m, 4H); 7.2 (m, 2H); 7.45 (m, 3H); 7.65 (m, 2H);
1-(4-bromophenyl)-1,4,5,6-tetrahydropyrazolo[3,4-e]indazole-3-carboxamide
$^1$H NMR (/ 400 MHz) 2.82 (bt, 2H); 3.28 (bt, 2H); 7.24 (bs, 1H); 7.36 (s, 1H); 7.46 (bs, 1H); 7.61 (d, 2H); 7.76 (d, 2H);
1-(4-nitrophenyl)-1,4,5,6-tetrahydropyrazolo[3,4-e]indazole-3-carboxamide
$^1$H NMR (/ 400 MHz) 2.85 (m, 2H); 3.06 (m, 2H); 7.32 (bs, 1H); 7.55 (bs, 1H); 7.57(s, 1H); 7.95 (d, 2H); 8.42 (d, 2H);
1-(4-methylphenyl)-1,4,5,6-tetrahydropyrazolo[3,4-e]indazole-3-carboxamide
$^1$H NMR (DMSO-$d_6$/ 400 MHz) 2.4 (s, 3H); 2.8-3.1 (m, 4H); 7.15 (s, 1H); 7.2-7.4 (2s, 2H); 7.35-7.45 (2d, 4H);
1-(4-chlorophenyl)-1,4,5,6-tetrahydropyrazolo[3,4-e]indazole-3-carboxamide
$^1$H NMR (DMSO-$d_6$/ 400 MHz) 2.8-3.1 (m, 4H); 7.2-7.45 (2s, 2H); 7.3 (s, 1H); 7.65 (m, 4H);
1-(4-cyanophenyl)-1,4,5,6-tetrahydropyrazolo[3,4-e]indazole-3-carboxamide
$^1$H NMR (DMSO-$d_6$/ 400 MHz) 2.85 (m, 2H); 3.06 (m, 2H); 7.30 (bs, 1H); 7.45 (bs, 1H); 7.55 (bs, 1H); 7.87 (d, 2H); 8.05 (d, 2H);
1-[4-(trifluoromethyl)phenyl]-1,4,5,6-tetrahydropyrazolo[3,4-e]indazole-3-carboxamide
$^1$H NMR (DMSO-$d_6$/ 400 MHz) 2.85 (bs, 2H); 3.09 (m, 2H); 7.25 (bs, 1H); 7.45 (bs, 1H); 7.51 (bs, 1H); 7.89 (d, 2H); 7.95 (d, 2H);
1-benzyl-1,4,5,6-tetrahydropyrazolo[3,4-e]indazole-3-carboxamide
$^1$H NMR (DMSO-$d_6$/ 400 MHz) 2.75-3.1 (m, 4H); 5.5 (s, 2H); 7.1-7.35 (m, 7H); 7.9 (s, 1H);
1-(3-hydroxybenzyl)-1,4,5,6-tetrahydropyrazolo[3,4-e]indazole-3-carboxamide
$^1$H NMR (DMSO-$d_6$/ 400 MHz) 2.65-3.05 (2t, 4H); 5.4 (s, 2H); 6.45-6.65 (m, 3H); 7.1-7.3 (m, 3H); 7.8 (s, 1H); 9.39 (s, 1H);
1-pyridin-2-yl-1,4,5,6-tetrahydropyrazolo[3,4-e]indazole-3-carboxamide
$^1$H NMR (DMSO-$d_6$/ 400 MHz) 2.85-3.1 (m, 4H); 7.3-7.65 (2s, 2H); 7.45 (m, 1H); 8.1 (m, 2H); 8.2 (s, 1H); 8.6 (d, 1H);
1-(3-methylphenyl)-1,4,5,6-tetrahydropyrazolo[3,4-e]indazole-3-carboxamide
$^1$H NMR (DMSO-$d_6$/ 400 MHz) 2.35 (s, 3H); 2.8-3.1 (m, 4H); 7.20 (s, 1H); 7.35-7.5 (m, 6H);
1-(3-chlorophenyl)-1,4,5,6-tetrahydropyrazolo[3,4-e]indazole-3-carboxamide
$^1$H NMR (DMSO-$d_6$/ 400 MHz) 2.8-3.1 (m, 4H); 7.2-7.35 (d + s, 3H); 7.4-7.8 (m, 4H);

1-(3-fluorophenyl)-1,4,5,6-tetrahydropyrazolo[3,4-e]indazole-3-carboxamide
$^1$H NMR (DMSO-$d_6$/ 400 MHz) 2.8-3.1 (2t, 4H); 7.2-7.4 (2s, 2H); 7.35 (s, 1H); 7.45-7.65 (m, 4H);
1-(6-chloropyridazin-3-yl)-1,4,5,6-tetrahydropyrazolo[3,4-e]indazole-3-carboxamide
$^1$H NMR (DMSO-$d_6$/ 400 MHz) 2.83 (t, 2H); 3.10 (t, 2H); 7.45 (s, 1H); 7.83 (s, 1H); 8.12 (s, 1H); 8.19 (d, 1H); 8.48 (d, 1H);
4,4-dimethyl-1-(4-methylphenyl)-1,4,5,6-tetrahydropyrazolo[3,4-e]indazole-3-carboxamide
$^1$H NMR (DMSO-$d_6$/ 400 MHz) 1.34 (s, 6H); 2.38 (s, 3H); 2.70 (s, 2H); 7.04 (bs, 1H); 7.24 (bs, 1H); 7.41 (dd, 4H); 7.53 (bs, 1H), 12.60 (bs, 1H).
1-pyridin-3-yl-1,4,5,6-tetrahydropyrazolo[3,4-e]indazole-3-carboxamide
$^1$HNMR (DMSO-d6/ 400 MHz); 2.8 (t,2H); 3.1 (t,2H); 7.1-7.3 (d,2H); 7.45 (s,1H); 7.61 (m,1H); 8.10 (d, 1H); 8.71 (d, 1H); 8.9 (s,1H); 12.7 (bs,1H)
1-[4-(acetylamino)phenyl]-1,4,5,6-tetrahydropyrazolo[3,4-e]indazole-3-carboxamide
$^1$HNMR (DMSO-d6/ 400 MHz); 2.05 (s,3H); 2.8 (t,2H); 3.01 (t,2H); 7.1 (s,1H); 7.2 (s,1H); 7.4 (s, 1H); 7.51 (d, 2H); 7.8 (d,2H); 10.2 (s,1H)
1-(4-aminophenyl)-1,4,5,6-tetrahydropyrazolo[3,4-e]indazole-3-carboxamide
$^1$H NMR (DMSO-$d_6$/ 400 MHz); 2.80 (bt, 2H); 3.04 (bt, 2H); 5.45 (bs, 2H); 6.66 (d, 2H); 7.04 (bs, 1H); 7.11 (bs, 1H); 7.16 (d, 2H); 7.32 (bs, 1H)
1-{4-[(4-methylpiperazin-1-yl)sulfonyl]phenyl}-1,4,5,6-tetrahydropyrazolo[3,4-e]indazole-3-carboxamide
$^1$H NMR (DMSO-$d_6$/ 400 MHz); 2.15 (m, 7H); 2.96 (m, 6H); 3.96 (bt, 2H); 7.30 (bs, 1H); 7.52 (bs, 2H); 7.93 (bs, 4H)
4-[3-(aminocarbonyl)-5,6-dihydropyrazolo[3,4-e]indazol-1(4H)-yl]benzoic acid
$^1$H NMR (DMSO-$d_6$/ 400 MHz); 2.84 (t, 2H); 3.06 (t, 2H); 7.27 (bs, 1H); 7.77 (d, 2H); 8.12 (d, 2H)
1-(4-morpholin-4-ylphenyl)-1,4,5,6-tetrahydropyrazolo[3,4-e]indazole-3-carboxamide
$^1$H NMR (DMSO-$d_6$/ 400 MHz); 2.82 (t, 2H); 3.05 (t, 2H); 3.19 (t, 4H); 3.75 (t, 4H); 7.08 (d, 2H); 7.09 (bs, 1H); 7.16 (bs, 1H); 7.36 (bs, 1H); 7.42.(d, 2H)
1-[4-(trifluoromethoxy)phenyl]-1,4,5,6-tetrahydropyrazolo[3,4-e]indazole-3-carboxamide
$^1$H NMR (DMSO-$d_6$/ 400 MHz); 2.85 (bt, 2H); 3.07 (t, 2H); 7.26 (bs, 2H); 7.47 (bs, 1H); 7.59 (d, 2H); 7.78 (d, 2H); 12.70 (bs, 1H)
1-butyl-1,4,5,6-tetrahydropyrazolo[3,4-e]indazole-3-carboxamide
$^1$H NMR (DMSO-$d_6$/ 400 MHz); 0.86 (t, 3H); 1.27 (m, 2H); 1.73 (m, 2H); 2.76 (t, 2H); 2.97 (t, 2H); 4.20 (t, 2H); 7.05 (bs, 1H); 7.16 (bs, 1H); 7.99 (bs, 1H); 12.75 (bs, 1H)
1-(2-hydroxyethyl)-1,4,5,6-tetrahydropyrazolo[3,4-e]indazole-3-carboxamide
$^1$H NMR (DMSO-$d_6$/ 400 MHz); 2.72 (t, 2H); 2.98 (t, 2H); 3.67 (t, 2H); 4.05 (t, 2H); 4.80 (bs (1H); 7.08 (bs, 1H); 7.24 (bs, 1H); 7.69 (s, 1H)
1-(2,5-dimethylphenyl)-1,4,5,6-tetrahydropyrazolo[3,4-e]indazole-3-carboxamide
$^1$H NMR (DMSO-$d_6$/ 400 MHz); 1.94 (s, 3H); 2.32 (s, 3H); 2.81 (t, 2H); 3.09 (t, 2H); 6.65 (bs, 1H); 7.14 (bs, 1H); 7.20 (s, 1H); 7.29 (d, 1H); 7.33 (d, 1H); 7.39 (bs, 1H)
1-(2,2,2-trifluoroethyl)-1,4,5,6-tetrahydropyrazolo[3,4-e]indazole-3-carboxamide
$^1$H NMR (DMSO-$d_6$/ 400 MHz); 2.77 (t, 2H); 3.00 (t, 2H); 5.17 (q, 2H); 7.23 (bs, 2H); 8.16 (bs, 1H); 12.61 (bs, 1H)
1-(2-amino-2-oxoethyl)-1,4,5,6-tetrahydropyrazolo[3,4-e]indazole-3-carboxamide
$^1$H NMR (DMSO-$d_6$/ 400 MHz); 2.76 (t, 2H); 2.97 (t, 2H); 4.84 (s, 2H); 7.08-7.53 (br, 4H); 7.82 (bs, 1H); 12.69 (br, 1H)
1-[4-(1H-imidazol-2-yl)phenyl]-1,4,5,6-tetrahydropyrazolo[3,4-e]indazole-3-carboxamide
$^1$H NMR (DMSO-$d_6$/ 400 MHz); 2.82 (t, 2H); 3.07 (t, 2H); 7.17-7.34 (m, 5H); 7.70 (d, 2H); 8.11 (d, 2H); 12.68 (bs, 1H)
4,4-dimethyl-1-(2,2,2-trifluoroethyl)-1,4,5,6-tetrahydropyrazolo[3,4-e]indazole-3-carboxamide
$^1$H NMR (DMSO-$d_6$/ 400 MHz); 1.29 (s, 6H); 2.64 (s, 2H); 5.16 (q, 2H); 7.30 (bs, 1H); 7.38 (bs, 1H); 8.11 (bs, 1H); 12.63 (bs, 1H)
1-methyl-1,4,5,6-tetrahydropyrazolo[3,4-e]indazole-3-carboxamide
$^1$H NMR (DMSO-$d_6$/ 400 MHz); 2.79 (t, 2H); 3.01 (t, 2H); 3.95 (s, 3H); 7.09 (bs, 1H); 7.25 (bs, 1H); 8.08 (bs, 1H); 12.80 (bs, 1H)
2-(2-hydroxyethyl)-2,4,5,6-tetrahydropyrazolo[3,4-e]indazole-3-carboxamide
$^1$H NMR (DMSO-$d_6$/ 400 MHz); 2.77 (t, 2H); 2.85 (t, 2H); 3.07 (t, 2H); 4.30 (t, 2H); 4.97 (bs, 1H); 7.61-7.86 (br, 3H); 12.58 (bs, 1H)
8-Anilino-1-methyl-1,4,5,6-tetrahydropyrazolo[3,4-e]indazole-3-carboxamide
NMR (400 MHz, DMSO-D6) δ ppm 2.78 (m, 4 H) 3.79 (s, 3 H) 6.8 (m, 3 H) 7.08 (m, 4 H) 8.05 (s, 1 H)
8-Anilino-1-(2,2,2-trifluoroethyl)-1,4,5,6-tetrahydropyrazolo[3,4-e]indazole-3-carboxamide
1H NMR (400 MHz, DMSO-D6) δ ppm 3.05 (m, 4 H) 5.21 (m, 2 H) 6.7 (m, 3 H) 7.14 (m, 2 H) 7.26 (m, 2H) 8.07 (s, 1 H)
8-amino-1,4,5,6-tetrahydropyrazolo[3,4-e]indazole-3-carboxamide
1H NMR (400 MHz, DMSO-D6) δ ppm 2.86 (t, 2H), 3.06 (t; 2H), 7.2-7.6 (br, 4H)

**Example 4**

**1-[4-methoxyphenyl]-1,6-dihydropyrazolo[3,4-e]indazole-3-carboxamide**

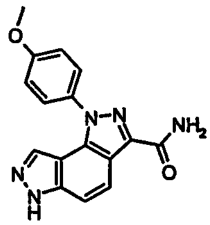

A suspension of 1-[4-methoxyphenyl]-1,4,5,6-tetrahydropyrazolo[3,4-e]indazole-3-carboxamide (137mg, 0.44 mmols) in anhydrous dioxane (7 ml) was treated with DDQ (114 mg, 0.50 mmols) and stirred at 100°C for 3 hours. After cooling, the mixture was evaporated to dryness, taken up with a diluted solution of $K_2CO_3$, filtered on buchner and washed with water to obtain 1-[4-methoxyphenyl]-1,6-dihydropyrazolo[3,4-e]indazole-3-carboxamide (98mg, Y=73%) as a solid

$^1$H NMR (DMSO-$d_6$/ 400 MHz) 3.81 (s, 3H); 7.21 (d, 2H); 7.48 (d, 1H); 7.65 (s, 1H); 7.72 (d, 2H); 8.17 (d, 1H).

By working analogously, the following compounds were prepared:

1-[4-(aminosulfonyl)phenyl]-1,6-dihydropyrazolo[3,4-e]indazole-3-carboxamide
$^1$H NMR (DMSO-$d_6$/ 400 MHz) 7.51 (s, 2H); 7.56 (d, 1H); 7.91 (s, 1H); 8.11 (m, 4H); 8.21 (d, 1H);
1-{4-[(methylamino)sulfonyl]phenyl}-1,6-dihydropyrazolo[3,4-e]indazole-3-carboxamide
$^1$H NMR (DMSO-$d_6$/ 400 MHz) 2.51 (m, 3H); 7.54 (s, 1H); 7.56 (d, 1H); 7.60 (m, 1H); 7.89-7.97 (m, 2H); 8.06-8.14 (m, 4H); 8.21 (d, 1H);
1-{4-[(butylamino)sulfonyl]phenyl}-1,6-dihydropyrazolo[3,4-e]indazole-3-carboxamide
$^1$H NMR (DMSO-$d_6$/ 400 MHz) 0.81 (t, 3H); 1.30 (q, 2H); 1.38 (q, 2H); 2.83 (q, 2H); 7.11 (s, 1H); 7.54 (s, 1H); 7.56 (d, 1H); 7.73 (t, 1H); 7.80 (s, 1H); 8.10 (m, 4H); 8.21 (d, 1H);
1-{4-[(dimethylamino)sulfonyl]phenyl}-1,6-dihydropyrazolo[3,4-e]indazole-3-carboxamide
$^1$H NMR (DMSO-$d_6$/ 400 MHz) 2.71 (s, 6H); 7.57 (d, 1H); 8.02 (s, 1H); 8.11 (m, 4H); 8.21 (d, 1H);
1-{4-[(diprop-2-ynylamino)sulfonyl]phenyl}-1,6-dihydropyrazolo[3,4-e]indazole-3-carboxamide
$^1$H NMR (DMSO-$d_6$/ 400 MHz) 3.25 (s, 2H); 4.2 (d, 4H); 7.75-8.2 (2d, 2H); 7.8-8.0 (2s, 2H); 7.9 (s, 1H); 8.15 (m, 4H);
1-[4-(anilinosulfonyl)phenyl]-1,6-dihydropyrazolo[3,4-e]indazole-3-carboxamide
$^1$H NMR (DMSO-$d_6$/ 400 MHz) 7.1-7.21 (2m, 4H); 7. 5 (s, 1H); 7.55, 8.1 (2d, 2H); 7.8, 7.9 (2s, 2H); 8.05 (s, 4H); 10.2 (s, 1H);
1-(4-{[(2-hydroxypropyl)amino]sulfonyl}phenyl)-1,6-dihydropyrazolo[3,4-e]indazole-3-carboxamide
$^1$H NMR (DMSO-$d_6$/ 400 MHz) 1.01 (d, 3H); 2.75 (m, 2H); 3.62 (d, 1H); 4.65 (s, 1H); 7.55 (s, 1H); 7.6-8.2 (2d, 2H); 7.75 (t, 1H); 7.9-8.0 (2s, 2H); 8.1 (s, 4H);
1-[4-(methylsulfonyl)phenyl]-1,6-dihydropyrazolo[3,4-e]indazole-3-carboxamide
$^1$H NMR (DMSO-$d_6$/ 400 MHz) 3.35 (s, 3H); 7.5 (s, 1H); 7.6-8.2 (2d, 2H); 7.9-8.05 (2s, 2H); 8.10-8.25 (m, 4H);
1,6-dihydropyrazolo[3,4-e]indazole-3-carboxamide
$^1$H NMR (DMSO-$d_6$/ 400 MHz) 7.3-7.7 (2s, 2H); 7.35-8.2 (2d, 2H); 7.45 (s, 1H);
1-phenyl-1,6-dihydropyrazolo[3,4-e]indazole-3-carboxamide
$^1$H NMR (DMSO-$d_6$/ 400 MHz) 7.47 (s, 1H); 7.52 (d, 1H); 7.55-7.85 (m, 7H); 8.19 (d, 1H);
1-(4-fluorophenyl)-1,6-dihydropyrazolo[3,4-e]indazole-3-carboxamide
$^1$H NMR (DMSO-$d_6$/ 400 MHz) 7.43-7.9 (m, 8H); 8.2 (d, 1H);
1-(4-methylphenyl)-1,6-dihydropyrazolo[3,4-e]indazole-3-carboxamide
$^1$H NMR (DMSO-$d_6$/ 400 MHz) 2.45 (s, 3H); 7.4-7.7 (2d, 4H); 7.5-8.2 (2d, 2H); 7.39-7.8 (2s, 2H); 7.75 (s, 1H);
1-(4-cyanophenyl)-1,6-dihydropyrazolo[3,4-e]indazole-3-carboxamide
$^1$H NMR (DMSO-$d_6$/ 400 MHz) 7.54 (bs, 1H); 7.55 (d, 1H); 7.93 (bs, 1H); 7.99 (bs, 1H); 8.11 (d, 2H); 8.17 (d, 2H); 8.21 (d, 1H);
1-[4-(trifluoromethyl)phenyl]-1,6-dihydropyrazolo[3,4-e]indazole-3-carboxamide
$^1$H NMR (DMSO-$d_6$/ 400 MHz) 7.55 (bs, 1H); 7.55 (d, 2H); 7.90 (bs, 1H); 7.95 (bs, 1H); 8.07 (d, 2H); 8.12 (d, 2H); 8.21 (d, 1H);
1-(4-chlorophenyl)-1,6-dihydropyrazolo[3,4-e]indazole-3-carboxamide

$^1H$ NMR (DMSO-$d_6$/ 400 MHz) 7.5-8.2 (2d, 2H); 7.55-7.9 (s, 2H); 7.8 (s, 1H); 7.75-7.95 (2d, 4H);
1-(4-bromophenyl)-1,6-dihydropyrazolo[3,4-e]indazole-3-carboxamide
$^1$HNMR (/ 400 MHz) 7.47-7.53 (m, 2H); 7.81-7.90 (m, 6H); 8.19 (d, 1H); 13.64 (s, 1H);
1-(4-nitrophenyl)-1,6-dihydropyrazolo[3,4-e]indazole-3-carboxamide
$^1H$ NMR (DMSO-$d_6$/ 400 MHz) 7.47 (s, 1H); 7.57 (d, 1H); 7.96 (bs, 1H); 8.07 (bs, 1H); 8.20 (m, 3H); 8.54 (d, 2H);
1-benzyl-1,6-dihydropyrazolo[3,4-e]indazole-3-carboxamide
$^1H$ NMR (DMSO-$d_6$/ 400 MHz) 5.9 (s, 2H); 7.2-7.45 (m, 6H); 7.4-7.7 (2s, 2H); 8.20 (d, 1H);
1-(3-hydroxybenzyl)-1,6-dihydropyrazolo[3,4-e]indazole-3-carboxamide
$^1H$ NMR (DMSO-$d_6$/ 400 MHz) 5.8 (s, 2H); 6.5-7.1 (m, 4H); 7.4-8.1 (2d, 2H); 7.35-7.7 (2s, 2H); 8.25 (s, 1H); 8.35 (s, 1H);
1-pyridin-2-yl-1,6-dihydropyrazolo[3,4-e]indazole-3-carboxamide
$^1H$ NMR (DMSO-$d_6$/ 400 MHz) 7.4-7.59 (m, 2H); 7.6-8.15 (2s, 2H); 8.1(m, 1H); 8.2-8.3 (2d, 2H); 8.8 (d, 1H); 8.9 (s, 1H);
1-(3-chlorophenyl)-1,6-dihydropyrazolo[3,4-e]indazole-3-carboxamide
$^1H$ NMR (DMSO-$d_6$/ 400 MHz) 7.52 (bs, 1H); 7.52 (d, 1H); 7.64-7.88 (m, 3H); 7.90 (s, 1H); 7.95 (bs, 1H); 7.97 (s, 1H); 8.20 (d, 1H);
1-(3-methylphenyl)-1,6-dihydropyrazolo[3,4-e]indazole-3-carboxamide
$^1H$ NMR (DMSO-$d_6$/ 400 MHz) 2.45 (s, 3H); 7.4-7.8 (2d, 2s, 4H); 7.45-7.65 (2s, 2H); 7.8 (s, 1H); 7.5-8.2 (2d, 2H);
1-(3-fluorophenyl)-1,6-dihydropyrazolo[3,4-e]indazole-3-carboxamide
$^1H$ NMR (DMSO-$d_6$/ 400 MHz) 7.4-7.7 (2m, 4H); 7.5-8.2 (2d, 2H); 7.65-7.9 (2s, 2H); 7.85 (s, 1H);
1-(6-chloropyridazin-3-yl)-1,6-dihydropyrazolo[3,4-e]indazole-3-carboxamide
$^1H$ NMR (DMSO-$d_6$/ 400 MHz) 7.45 (s, 1H); 7.6-7.65 (2s, 2 H); 8.25-8.7 (2d, 2H); 8.2 (m, 2H);
1-(4-methoxyphenyl)-1,6-dihydropyrazolo[3,4-e]indazole-3-carboxylic acid
$^1H$ NMR (DMSO-$d_6$/ 400 MHz) 3.9 (s, 3H); 7.2-7.7 (2d, 2H); 7.5-8.2 (2d, 2H); 7.69 (s, 1H);
Ethyl 1-phenyl-1,6-dihydropyrazolo[3,4-e]indazole-3-carboxylate
$^1H$ NMR ($CDCl_3$ /400 MHz) 1.51 (t, 3H); 4.57 (q, 2H); 7.51 (d, 1H); 7.72 (m, 5H); 7.82 (s, 1H); 8.29 (d, 1H);
Ethyl 1-(4-methoxyphenyl)-1,6-dihydropyrazolo[3,4-e]indazole-3-carboxylate
$^1H$ NMR (DMSO-$d_6$/ 400 MHz) 1.4 (t, 3 H); 3.9 (s, 3H); 4.45 (q, 2H); 7.25-7.7 (2d, 2H); 7.55-8.2 (2d, 2H); 7.6 (s, 1 H);
N-methyl-1-[4-(aminosulfonyl)phenyl]-1,6-dihydropyrazolo[3,4-e]indazole-3-carboxamide
$^1H$ NMR (DMSO-$d_6$/ 400 MHz) 2.85 (d, 3H); 7.45 (s, 2H); 7.6-8.2 (2d, 2H); 7.9 (s, 1H); 8.10 (m, 4H); 8.5 (d, 1H); 13.73 (s, 1H);
N-methyl-1-{4-[(butylamino)sulfonyl]phenyl}-1,6-dihydropyrazolo[3,4-e]indazole-3-carboxamide
$^1H$ NMR (DMSO-$d_6$/ 400 MHz) 0.8 (t, 3H); 1.2-1.4 (2m, 4H); 2.81 (m, 5H); 7.45-8.2 (2d, 2H); 7.75 (t, 1H); 7.95 (s, 1H); 8.10 (m, 4H); 8.5 (m, 1H); 13.7 (s, 1H);
N-methyl-1-{4-[(dimethylamino)sulfonyl]phenyl}-1,6-dihydropyrazolo[3,4-e]indazole-3-carboxamide
$^1H$ NMR (DMSO-$d_6$/ 400 MHz) 2.7 (s, 6H); 2.85 (d, 3H); 7.6-8.2 (2d, 2H); 8.05 (s, 1H); 8.07-8.15 (2d, 4H);
N-methyl-1-[4-(methylsulfonyl)phenyl]-1,6-dihydropyrazolo[3,4-e]indazole-3-carboxamide
$^1H$ NMR (DMSO-$d_6$/ 400 MHz) 2.83 (d, 3H); 3.18 (s, 3H); 7.6-8.25 (2d, 2H); 8.05 (s, 1H); 8.15-8.20 (m, 4H); 8.45 (m, 1H);
N-(allyloxy)-1-{4-[(butylamino)sulfonyl]phenyl}-1,6-dihydropyrazolo[3,4-e]indazole-3-carboxamide
$^1H$ NMR (DMSO-$d_6$/ 400 MHz) 0.8 (t, 3H); 1.2-1.4 (2m, 4H); 2.8 (q, 2H); 4.45 (d, 2H); 5.2-5.4 (2d, 2H); 6.1 (m, 1H); 7.55-8.15 (2d, 2H); 7.7 (t, 1H); 7.9 (s, 1H); 8.1 (s, 4H);
7,8,9,10-tetrahydro[1,4]diazepino[1,2-b]pyrazolo[3,4-g]indazol-6(3H)-one
$^1H$ NMR (DMSO-$d_6$/ 400 MHz) 2.24 (m, 2H); 3.21 (m, 2H); 4.68 (bt, 2H); 7.33-7.38(dd, 1H); 7.73-7.79 (dd, 1H); 8.21 (s, 1H); 8.32 (bs, 1H); 13.36 (bs, 1H).
1-pyridin-3-yl-1,6-dihydropyrazolo[3,4-e]indazole-3-carboxamide
$^1$HNMR (DMSO-d6/ 400 MHz); 7.45 (d,2H); 7.75 (m,2H); 7.91 (s,1H); 8.2 (d,1H); 8.35 (d,1H); 8.81 (d, 1H); 9.1 (s, 1H)
1-[4-(acetylamino)phenyl]-1,6-dihydropyrazolo[3,4-e]indazole-3-carboxamide
$^1H$ NMR (DMSO-$d_6$/ 400 MHz); 2.10 (s, 3H); 7.5 (bs, 1H); 7.48 (d, 1H); 7.73 (d, 2H); 7.79 (bs, 1H); 7.87 (d, 2H); 8.17 (d, 1H)
4-[3-(aminocarbonyl)pyrazolo[3,4-e]indazol-1(6H)-yl]benzoic acid
$^1H$ NMR (DMSO-$d_6$/ 400 MHz); 7.53 (bs, 1H); 7.54 (d, 1H); 7.89 (bs, 1H); 7.95 (bs, 1H); 8.01 (d, 2H); 8.20 (d, 1H); 8.24 (d, 2H)
1-{4-[(4-methylpiperazin-1-yl)sulfonyl]phenyl}-1,6-dihydropyrazolo[3,4-e]indazole-3-carboxamide
$^1H$ NMR (DMSO-$d_6$/ 400 MHz); 2.15 (s, 3H); 2.39 (bt, 4H); 3.01 (bt, 4H); 7.55 (d, 1H); 7.56 (bs, 1H); 7.91n(bs, 1H); 8.02 (bs, 1H); 8.04 (d, 2H); 8.16 (d, 2H); 8.21 (d, 1H); 13.72 (bs, 1H)
1-[4-(trifluoromethoxy)phenyl]-1,6-dihydropyrazolo[3,4-e]indazole-3-carboxamide
$^1H$ NMR (DMSO-$d_6$/ 400 MHz); 7.52 (bs, 1H); 7.53 (d, 1H); 7.70 (d, 2H); 7.86 (bs, 2H); 8.01 (d, 2H); 8.20 (d, 1H); 13.69 (bs, 1H)

4-[3-(ethoxycarbonyl)pyrazolo[3,4-e]indazol-1(6H)-yl]benzoic acid
$^1$H NMR (DMSO-$d_6$/ 400 MHz); 1.39 (t, 3H); 4.43 (q, 2H); 7.62 (d, 1H); 7.93 (s, 1H); 7.98 (d, 2H); 8.09 (d, 2H); 8.24 (d, 2H); 13.27 (bs, 1H); 13.43 (bs, 1H)
1-(4-morpholin-4-ylphenyl)-1,6-dihydropyrazolo[3,4-e]indazole-3-carboxamide
$^1$H NMR (DMSO-$d_6$/ 400 MHz); 3.26 (bt, 4H); 3.78 (bt, 4H); 7.19 (d, 2H); 7.42 (bs, 1H); 7.46 (d, 1H); 7.63 (d, 2H); 7.71 (bs, 1H); 7.75 (bs, 1H); 8.16 (d, 1H); 13.59 (bs, 1H)
1-(2-hydroxyethyl)-1,6-dihydropyrazolo[3,4-e]indazole-3-carboxamide
$^1$H NMR (DMSO-$d_6$/ 400 MHz); 3.92 (t, 2H); 4.67 (t, 2H); 7.31 (bs, 1H); 7.38 (d, 1H); 7.61 (bs, 1H); 8.06 (d, 1H); 8.47 (bs, 1H); 13.50 (bs, 1H)
1-(2,5-dimethylphenyl)-1,6-dihydropyrazolo[3,4-e]indazole-3-carboxamide
$^1$H NMR (DMSO-$d_6$/ 400 MHz); 1.94 (s, 3H); 2.37 (s, 3H); 7.20 (s, 1H); 7.40 (m, 4H); 7.47 (d, 1H); 7.79 (bs, 1H); 8.18 (d, 1H); 13.58 (bs, 1H)
1-(2-aminoethyl)-1,6-dihydropyrazolo[3,4-e]indazole-3-carboxamide hydrochloride
$^1$H NMR (DMSO-$d_6$/ 400 MHz); 3.38 (t, 2H); 4.90 (t, 2H); 7.43 (d, 1H); 7.44 (bs, 1H); 7.89 (bs, 1H); 8.08 (d, 1H); 8.21 (bs 3H); 8.60 (s, 1H)
1-(2,2,2-trifluoroethyl)-1,6-dihydropyrazolo[3,4-e]indazole-3-carboxamide
$^1$H NMR (DMSO-$d_6$/ 400 MHz); 5.63 (m, 2H); 7.46 (d, 1H); 7.50 (bs, 1H); 8.11 (d, 1H); 8.62 (s, 1H); 13.62 (bs, 1H)
1-[4-(1H-imidazol-2-yl)phenyl]-1,6-dihydropyrazolo[3,4-e]indazole-3-carboxamide
$^1$H NMR (DMSO-$d_6$/ 400 MHz); 7.25 (s, 2H); 7.50 (bs, 1H); 7.52 (d, 1H); 7.86 (bs, 2H); 7.94 (d, 2H); 8.20 (d, 1H); 8.23 (d, 2H)12.50 (bs, 1H)
1-methyl-1,6-dihydropyrazolo[3,4-e]indazole-3-carboxamide
$^1$H NMR (DMSO-$d_6$/ 400 MHz); 4.35 (s, 3H); 7.35 (bs, 1H); 7.42 (d, 1H); 7.65 (bs, 1H); 8.10 (d, 1H); 8.56 (s, 1H); 13.56 (bs, 1H)
8,9-dihydro-3H-pyrazino[1,2-b]pyrazolo[3,4-g]indazol-6(7H)-one
$^1$H NMR (DMSO-$d_6$/ 400 MHz); 3.72 (m, 2H); 4.57 (t, 2H); 7.43 (d, 1H); 7.83 (d, 1H); 8.27 (bs, 2H); 13.42 (bs, 1H)
2-(2-aminoethyl)-2,6-dihydropyrazolo[3,4-e]indazole-3-carboxamide hydrochloride
$^1$H NMR (DMSO-$d_6$/ 400 MHz); 3.55 (t, 2H); 4.84 (t, 2H); 7.42 (d, 1H); 7:66 (d, 1H); 8.00-8.12 (bs, 6H); 8.27 (s, 1H)
2-(2-hydroxyethyl)-2,6-dihydropyrazolo[3,4-e]indazole-3-carboxamide
$^1$H NMR (DMSO-$d_6$/ 400 MHz); 3.84 (t, 2H); 4.67 (t, 2H); 7.35 (d, 1H); 7.62 (d, 1H); 7.90 (bs, 1H); 8.177 (bs, 1H); 8.25 (s, 1H)
2-methyl-2,6-dihydropyrazolo[3,4-e]indazole-3-carboxamide
$^1$H NMR (DMSO-$d_6$/ 400 MHz); 4.27 (s, 3H); 7.40 (d, 1H); 7.66 (d, 1H); 7.93 (bs, 1H); 8.04 (bs, 1H); 8.25 (s, 1H) 13.38 (bs, 1H)
1-anilino-8,9-dihydro-3H-pyrazino[1,2-b]pyrazolo[3,4-g]indazol-6(7H)-one
NMR (400 MHz, DMSO-D6) δ ppm 3.74 (m, 2 H) 4.64 (m, 2 H) 6.82 (t, *J*=7.32 Hz, 1 H) 7.25 (t, *J*=7.32 Hz, 2 H) 7.36 (d, *J*=9.02 Hz, 1 H) 7.57 (d, *J*=8.5 Hz, 2 H) 7.84 (bs, 1 H) 7.88 (d, *J*=9.02 Hz, 1 H) 8.34 (bs, 1 H)

**Example 5**

**1-(4-methoxy-phenyl)-1,6-dihydropyrazolo[3,4-e]indazol-3-amine**

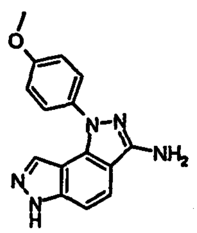

A solution of 1-[4-methoxyphenyl]-1,6-dihydropyrazolo[3,4-e]indazole-3-carboxamide (100 mg; 0.325 mmols) in 2.5 N NaOH (500 mg in 5 ml of water; 12.5 mmols) was treated with a 1M solution of NaClO (0.325 ml). The resulting mixture was heated at 100°C for 15 minutes. After cooling to room temperature, the solution was filtered and neutralized with HCl. The resulting precipitate was extracted with ethyl acetate, dried over $Na_2SO_4$ and evaporated to dryness. The crude material was then chromatographed on silica gel eluted with ethyl acetate to obtain 1-(4-methoxy-phenyl)-1,6-dihydropyrazolo[3,4-e]indazol-3-amine (45 mg; Y=49%) as a brown solid

$^1H$ NMR (DMSO-$d_6$/ 400 MHz) 5.65 (bs, 2H); 7.11 (d, 2H); 7.17 (d, 1H); 7.56 (d, 2H); 7.67 (d, 1H); 7.80 (s, 1H).

**Example 6**

**1-[1-(4-methylphenyl)-1,6-dihydropyrazolo[3,4-e]indazol-3-yl]ethanone**

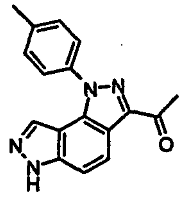

Triethylamine (0.82 ml; 6 mmols) was added to a 3M solution of EtMgCl in THF (0.67 ml; 2 mmols) at 0°C under argon atmosphere. After 10 min, a solution of ethyl 1-(4-methylphenyl)-7-trityl-1,7-dihydropyrazolo[3,4-e]indazole-3-carboxylate (564 mg; 1 mmol) in anhydrous THF (6 ml) was added dropwise. After leaving at 0°C for 1 hour and 30 min at room temperature the resulting mixture was poured into a 20% $NaH_2PO_3$ solution and extracted with ethyl acetate. The organic extracts were collected, dried over $Na_2SO_4$ and evaporated to dryness. The crude material was chromatographed on silica gel eluted with hexane/ethyl acetate 8/2 to obtain 1-[1-(4-methylphenyl)-7-trityl-1,7-dihydropyrazolo [3,4-e]indazol-3-yl]ethanone (120 mg; Y=22%) as a white solid. The latter was then suspended in acetone (6 ml) and treated with few drops of 37% HCl. The resulting mixture was left at room temperature for 1.5 hours and filtered to give 1-[1-(4-methylphenyl)-1,6-dihydropyrazolo[3,4-e]indazol-3-yl]ethanone (50 mg; Y=78%) as a white solid
$^1H$ NMR (DMSO-$d_6$/ 400 MHz) 2.41 (s, 3H); 2.84 (t, 2H); 3.06 (t, 2H); 7.13 (s, 1H); 7.40 (d, 2H); 7.50 (d, 2H).

**Example 7**

**1-(4-methoxyphenyl)-1,6-dihydropyrazolo[3,4-e]indazole-3-carbonitrile**

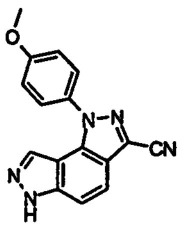

A suspension of 1-[4-methoxyphenyl]-1,6-dihydropyrazolo[3,4-e]indazole-3-carboxamide (20 mg; 0.065 mmols) in anhydrous THF (0.5 ml) was treated at room temperature under argon atmosphere with pyridine (0.05 ml; 0.65 mmols) and trifluoroacetic anhydride (0.05 ml; 0.39 mmols). The resulting solution was left at room temperature for 1 hour, diluted with water and filtered to obtain 1-(4-methoxyphenyl)-1,6-dihydropyrazolo[3,4-e]indazole-3-carbonitrile (16 mg; Y=85%) as a white solid
$^1H$ NMR (DMSO-$d_6$/ 400 MHz) 3.89 (s,3H); 7.23 (d, 2H); 7.65 (d, 1H); 7.74 (d, 2H), 7.75 (s, 1H), 7.78 (db, 1H).

**Example 8**

**1-(4-methoxyphenyl)-1,6-dihydropyrazolo[3,4-e]indazole-3-carbohydrazide**

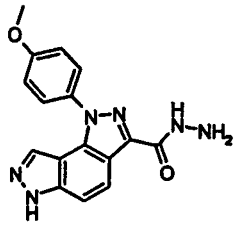

Ethyl 1-(4-methoxyphenyl)-1,6-dihydropyrazolo[3,4-e]indazole-3-carboxylate (100 mg; 0.295 mmols) was suspended on MeOH (5 ml) and treated with hydrazine hydrate (2.5 ml). The mixture was refluxed for 7 hours, after cooling, concentrated under reduced pressure, diluted with water and filtred to obtain 1-(4-methoxyphenyl)-1,6-dihydropyrazolo [3,4-e]indazole-3-carbohydrazide (85 mg; Y=89%) as a white solid
$^1$H NMR (DMSO-$d_6$/ 400 MHz) 3.88 (s, 3H); 4.51 (bs, 2H); 7.22 (d, 2H); 7.47 (s, 1H); 7.49 (d, 1H); 7.72 (d, 2H); 8.13 (d, 1H).

By working analogously, the following compound was prepared:

1-(4-methoxyphenyl)-1,4,5,6-tetrahydropyrazolo[3,4-e]indazole-3-carbohydrazide
$^1$H NMR (DMSO-$d_6$/ 400 MHz) 2.80 (m, 2H); 3.05 (m, 2H); 3.82 (s, 3H); 4.38 (bs, 2H); 7.10 (d, 2H); 7.19 (bs, 1H); 7.50 (d, 2H).

**Example 9**

**N'-hydroxy-1-(4-methoxyphenyl)-1,6-dihydropyrazolo[3,4-e]indazole-3-carboximidamide**

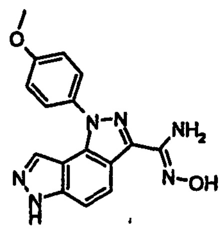

1-(4-methoxyphenyl)-1,6-dihydropyrazolo[3,4-e]indazole-3-carbonitrile (10 mg; 0.035 mmols) was suspended in ethanol (2.5 ml) and treated with hydroxylamine hydrochloride (116 mg; 1.68 mmols) and with a solution of sodium carbonate (146 mg) in water (1 ml). The resulting mixture was refluxed for 4 hours, after cooling, concentrated under vacuum, diluted with water and filtered to give N'-hydroxy-1-(4-methoxyphenyl)-1,6-dihydropyiazolo[3,4-e]indazole-3-carboximidamide (8.3 mg; Y=76%) as a white solid
$^1$H NMR (DMSO-$d_6$/ 400 MHz) 3.9 (s, 3H); 7.2-7.71 (2d, 4H); 7.69 (s, 1H); 7.45-8.15 (2d, 2H); 9.1 (s, 1H).

**Example 10**

**1-(4-methoxyphenyl)-1,6-dihydropyrazolo[3,4-e]indazole-3-carboxylic acid**

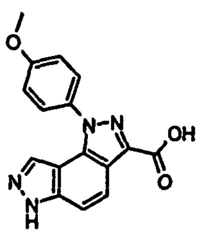

To a suspension of ethyl 1-(4-methoxyphenyl)-1,6-dihydropyrazolo[3,4-e]indazole-3-carboxylate (400 mg; 1.18 mmols) in methanol (10 ml) N NaOH (5.9 ml) was added dropwise. The resulting mixture was kept at 80°C for 2 hours. After cooling, the solution was acidified with 2N HCl and the product was filtered on buckner to give 1-(4-methoxyphenyl)-1,6-dihydropyrazolo[3,4-e]indazole-3-carboxylic acid (360 mg; Y=98%)
$^1$H NMR (DMSO-$d_6$/ 400 MHz) 2.85-3.15 (2t, 4H); 3.85 (s, 3H); 7.1 (s, 1H); 7.15-7.45 (2d, 4H).
By working analogously, the following compounds were prepared:

1-(4-bromophenyl)-1,4,5,6-tetrahydropyrazolo[3,4-e]indazole-3-carboxylic acid
$^1$H NMR (DMSO-$d_6$/ 400 MHz) 2.8-3.1 (m, 4H); 7.25 (s, 1H); 7.45-7.75 (d, 4H);
1-{4-[(butylamino)sulfonyl]phenyl}-1,4,5,6-tetrahydropyrazolo[3,4-e]indazole-3-carboxylic acid
$^1$H NMR (DMSO-$d_6$/ 400 MHz) 0.8 (t, 3H); 1.2-1.4 (m, 4H); 2.8 (dd, 2H); 2.85-3.15 (2t, 4H); 7.35 (s, 1H); 7.7 (t, 1H); 7.85-8.05 (2d, 4H);
4,4-dimethyl-1-(4-methylphenyl)-1,4,5,6-tetrahydropyrazolo[3,4-e]indazole-3-carboxylic acid
$^1$H NMR (DMSO-$d_6$/ 400 MHz) 1.34 (s, 6H); 2.39 (s, 3H); 2.71 (s, 2H); 7.03 (bs, 1H); 7.42 (dd, 4H); 12.70 (bs, 2H).

## Example 11

**N-hydroxy-1-(4-methoxyphenyl)-1,6-dihydropyrazolo[3,4-e]indazole-3-carboxamide**

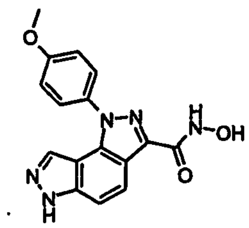

To a solution of 1-(4-methoxyphenyl)-1,6-dihydropyrazolo[3,4-e]indazole-3-carboxylic acid (100 mg; 0.325mmols) in DMF (2 ml), carbonyl diimidazole (106 mg; 0.65 mmols) was added and the mixture was stirred for 1 hour. $Na_2CO_3$ (65 mg; 0.60 mmols) and hydroxylamine hydrochloride (45 mg; 0.65 mmols) were then added and the mixture was stirred for 3 hours at room temperature. After evaporation of the solvent under reduced pressure, the crude material was taken up with water and filtered on buckner to give a solid compound that was further purified by chromatography on silica gel eluted with methylene chloride/methanol 10/1, to afford the desired N-hydroxy-1-(4-methoxyphenyl)-1,6-dihydropyrazolo[3,4-e]indazole-3-carboxamide (50 mg; Y=48%)
$^1$H NMR (DMSO-$d_6$/ 400 MHz) 2.8-3.1 (m, 4H); 3.91 (s, 3H); 7.2 (s, 1H); 7.15-7.45 (2d, 4H); 8.85 (s, 1H); 10.81 (s, 1H).
By working analogously, the following compounds were prepared:

N-(allyloxy)-1-{4-[(butylamino)sulfonyl]phenyl}-1,4,5,6-tetrahydropyrazolo[3,4-e]indazole-3-carboxamide
$^1$H NMR (DMSO-$d_6$/ 400 MHz) 0.8 (m, 3H); 1.21-1.39 (m, 4H); 2.75 (m, 2H); 2.85-3.1 (m, 4H); 4.39 (d, 2H); 5.23-5.39 (2d, 2H); 5.9 (m, 1H); 7.4 (s, 1H); 7.65 (t, 1H); 7.85-8.0 (2d, 4H); 11.45 (s, 1H);
N-(allyloxy)-1-(4-methoxyphenyl)-1,4,5,6-tetrahydropyrazolo[3,4-e]indazole-3-carboxamide
$^1$H NMR (DMSO-$d_6$/ 400 MHz) 2.85-3.15 (m, 4H); 3.8 (s, 3H); 4.4 (d, 2H); 5.2-5.35 (2d, 2H); 5.9 (m, 1H); 7.1 (s, 1H); 7.15-7.45 (2d, 4H); 11.39 (s, 1H).

## Example 12

**N-methyl-1-[4-(methylsulfonyl)phenyl]-1,4,5,6-tetrahydropyrazolo[3,4-e]indazole-3-carboxamide**

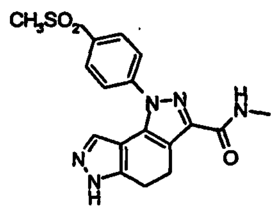

Ethyl 1-[4-(methylsulfonyl)phenyl]-1,4,5,6-tetrahydropyrazolo[3,4-e]indazole-3-carboxylate (200 mg; 0.52 mmols) was dissolved in a 33% solution of methylamine in ethanol (10 ml) and stirred at 65°C overnight. After evaporation of the solvent under reduced pressure, the residue was purified by chroatography on silica gel eluted with methylene chloride/methanol 10/1, to give N-methyl-1-[4-(methylsulfonyl)phenyl]-1,4,5,6-tetrahydropyrazolo[3,4-e]indazole-3-carboxamide (180 mg; Y=93%)

$^1$H NMR (DMSO-$d_6$/ 400 MHz) 2.75 (d, 3H); 2.85-3.1 (2m, 4H); 3.25 (s, 3H); 7.46 (s, 1H); 7.95-8.18 (2d, 4H).

By working analogously, the following compounds were prepared:

1-[4-(aminosulfonyl)phenyl]-N-methyl-1,4,5,6-tetrahydropyrazolo[3,4-e]indazole-3-carboxamide
$^1$H NMR (DMSO-$d_6$/ 400 MHz) 2.75 (d, 3H); 2.8-3.1 (m, 4H); 7.35 (s, 1H); 7.45 (s, 2H); 7.85-8.05 (2d, 4H); 8.15 (d, 1H);
1-{4-[(butylamino)sulfonyl]phenyl}-N-methyl-1,4,5,6-tetrahydropyrazolo[3,4-e]indazole-3-carboxamide
$^1$H NMR (DMSO-$d_6$/ 400 MHz) 0.8 (q, 3H); 1.2-1.4 (2m, 4H); 2.75 (d, 3H); 2.8 (m, 2H); 2.85-3.1 (2m, 4H); 7.35 (s, 1H); 7.65 (t, 1H); 7.85-7.95 (2d, 4H); 8.15 (q, 1H);
1-{4-[(dimethylamino)sulfonyl]phenyl}-N-methyl-1,4,5,6-tetrahydropyrazolo[3,4-e]indazole-3-carboxamide
$^1$H NMR (DMSO-$d_6$/ 400 MHz) 2.65 (s, 6H); 2.85-3.1 (2m, 4H); 7.45 (s, 1H); 7.95 (m, 4H); 8.18 (m, 1H).

## Example 13

**Ethyl 2-(3-aminopropyl)-2,4,5,6-tetrahydropyrazolo[3,4-e]indazole-3-carboxylate** hydrochloride

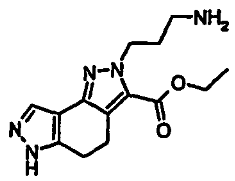

A solution of ethyl 7-trityl-1,4,5,7-tetahydropyrazolo[3,4-e]indazole-3-carboxylate (2 g; 4.2 mmols) in DMF (20 ml) was cooled to 0°C and treated dropwise with 1M lithium t-butoxide in THF (8.4 ml; 8.4 mmols). The solution was kept at 0°C for 30 min and boc-aminopropyl bromide (1.1 g; 4.6 mmols) in THF (5 ml) was added dropwise. The mixture was stirred overnight at room temperature, poured in NaHPO4 aqueous solution and extracted with ethyl acetate. The organic layer was evaporated to dryness and the crude material dissolved in dioxane (20 ml), treated with 37% hydrochloric acid (8 ml) and stirred at room temperature for four hours. After removing the solvent under reduced pressure, the residue was taken up with ethyl acetate and filtered to give ethyl 2-(3-aminopropyl)-2,4,5,6-tetrahydropyrazolo[3,4-e]indazole-3-carboxylate hydrochloride (1.21g; Y=88%)

$^1$H NMR (DMSO-$d_6$/ 400 MHz) 1.33 (t, 3H); 2.55 (m, 2H); 2.76 (m, 2H); 2.83 (t, 2H); 3.01 (t, 2H); 4.31 (q, 2H); 4.49 (t, 2H); 7.79 (s, 1H).

## Example 14

**4,5,7,8,9,10-hexahydro[1,4]diazepino[1,2-b]pyrazolo[3,4-g]indazol-6(3H)-one**

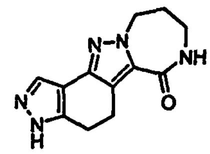

A solution of ethyl 2-(3-aminopropyl)-2,4,5,6-tetrahydropyrazolo[3,4-e]indazole-3-carboxylate (1.21 g; 3.71 mmols) in methanol (50 ml) was treated with oesium carbonate (2.42 g; 7.43 mmols) and stirred at room temperature for one day. The solution was evaporatd to dryness, taken up with water and, after vigorous stirring, filtred to give 4,5,7,8,9,10-hexahydro[1,4]diazepino[1,2-b]pyrazolo[3,4-g]indazol-6(3H)-one (0.715 g; Y=79%)

$^1$H NMR (DMSO-$d_6$/ 400 MHz) 2.05 (m, 2H); 2.75 (m, 2H); 2.86 (m, 2H), 3.14 (m, 2H), 4.30 (t, 2H); 7.79 (bs, 1H); 8.09 (bs, 1H).

By working analogously, the following compounds were prepared:

5,5-dimethyl-4,5,7,8,9,10-hexahydro[1,4]diazepino[1,2-b]pyrazolo[3,4-g]indazol-6(3H)-one
$^1$H NMR (DMSO-$d_6$/ 400 MHz) 1.24 (s, 6H); 1.99 (m, 2H); 2.62 (s, 2H); 3.04 (bt, 2H); 4.25 (t, 2H); 7.72 (s, 1H); 8.29 (bs, 1H); 12.54 (bs, 1H);
5,5-dimethyl-4,5,8,9-tetrahydro-3H-pyrazino[1,2-b]pyrazolo[3,4-g]indazol-6(7H)-one
$^1$H NMR (DMSO-$d_6$/ 400 MHz) 1.35 (s, 6H); 2.64 (bs, 2H); 3.54 (t, 2H); 4.20 (t, 2H); 7.81 (bs, 1H); 8.14 (bs, 1H); 12.57 (bs, 1H).
4,5,8,9-tetrahydro-3H-pyrazino[1,2-b]pyrazolo[3,4-g]indazol-6(7H)-one
$^1$HNMR (DMSO-d6/ 400 MHz); 2.65 (t,2H); 2.95 (t,2H); 3.56 (m,2H); 4.19 (t,2H); 7.8 (bs,1H); 8.05 (s, 1H); 12.7 (bs,1H)
1-anilino-4,5,8,9-tetrahydro-3H-pyrazino[1,2-b]pyrazolo[3,4-g]indazol-6(7H)-one
NMR (400 MHz, DMSO-D6) δ ppm 2.85 (m, 2 H) 3.03 (m, 2 H) 3.60 (m, 2 H) 4.25 (m, 2 H) 6.75 (m, 1 H) 7.19 (m, 2 H) 7.30 (bs, 1 H) 7.45 (m, 2 H) 8.12 (s, 1 H)

## Claims

1. Use, for manufacturing a medicament for treating diseases caused by and/or associated with an altered protein kinase activity by administration to a mammal in need thereof of an effective amount, of a compound of formulae (Ic), (Ie), (If) or (Ig)

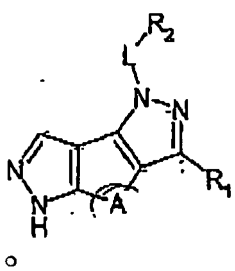

(Ic)

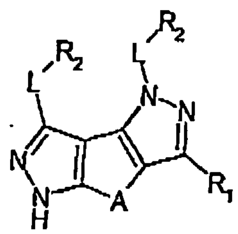

(Ie)

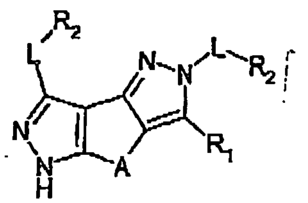

(If)

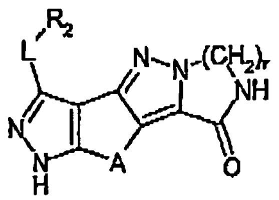

(Ig)

wherein:

$R_1$ in formula (Ic) is selected from the group consisting of hydrido, lower alkyl, perfluorinated lower alkyl, heterocyclyl, CN, $CO_2R'$, $COCF_3$, COR', CONR'R", NR'R", C(=NR')NR'R", $CONHNH_2$, CONHOR', NHCOR', $CH_2NH_2$, and $CH_2NHCOR'$; or

$R_1$ in formulae (Ic) and (If) is selected from the group consisting of CN, $CO_2R'$, COR', CONR'R", NR'R", C (=NOH)NR'R", $CONHNH_2$, CONHOR';

R' and R" in formula (Ic) are selected, each independently, from the group consisting of hydrido, hydroxy, alkyl, hydroxyalkyl, alkenyl, alkynyl, aryl, arylalkyl, heterocyclyl or heterocyclylalkyl; or

R' and R" in formulae (Ie) and (If) are selected, each independently, from the group consisting of hydrido or lower alkyl;

A in formulae (Ic) is selected from the group consisting of $-CH_2-$; $-CH_2-CH_2-$; -CH=CH-; $(CH_2)-C(CH_3)_2$; or

A in formulae (Ie), (If) and (Ig) is selected from the group consisting of $-CH_2-CH_2-$; -CH=CH-; $(CH_2)-C(CH_3)_2$;

L represents, each independently, a single bond, an alkylidene group or a divalent group selected from NH, NHCO, CONH, NHCONH, $SO_2NH$ and $NHSO_2$;

$R_2$ is, each independently, hydrido, alkyl, 5 to 12 membered mono- or bi-cyclic ring having from 0 to 3 heteroatoms selected from S, O and N, optionally substituted with one or more $-(CH_2)_q-R_3$ groups; or $R_2$ is a group of formula

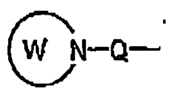

W is a 3 to 7 membered ring having one N heteroatom directly linked to Q and from 0 to 2 additional heteroatoms selected from the group consisting of S, SO, $SO_2$, O, N and NR', wherein R' is as above defined;

Q is a divalent group selected from CO, $SO_2$ and $(CH_2)_n$;

$R_3$ is selected, each independently, from the group consisting of alkyl, halogen, $CF_3$, $OCF_3$, $NO_2$, CN, C(=NR') NR'R", OR', SR', OCOR', OCONR'R", $COCF_3$, COR', $CO_2R'$, CONR'R", $SO_2R'$, $SO_2NR'R"$, NR'R", NR'COR', NR'COOR', NR'CONR'R", $NR'SO_2R'$, $NR'SO_2NR'R"$, wherein R' and R" are as above defined;

n is, each independently, 0, 1, or 2;

q is, each independently, 0 or an integer from 1 to 3;

r is an integer from 1 to 3;

or isomers, tautomers, carriers, prodrugs, and pharmaceutically acceptable salts thereof.

2. The use of claim 1 wherein the disease caused by and/or associated with an altered protein kinase activity is a cell proliferative disorder selected from the group consisting of cancer, Alzheimer's disease, viral infections, auto-immune diseases and neurodegenerative disorders.

3. The use of claim 2 wherein the cancer is selected from carcinoma, squamous cell carcinoma, hematopoietic tumors of lymphoid or myeloid lineage, tumors of meseuchymal origin, tumors of the central and peripheral nervous system, melanoma, seminoma, teratocarcinoma, osteosarcoma, xeroderma pigmentosum, keratoxanthoma, thyroid follicular cancer and Kaposi's sarcoma.

**4.** The use of claim 1 wherein the cell proliferative disorder is selected from benign prostate hyperplasia, familial adenomatosis, polyposis, neuro-fibromatosis, psoriasis, vascular smooth cell proliferation associated with atherosclerosis, pulmonary fibrosis, arthritis glomerulonephritis and post-surgical stenosis and restenosis.

**5.** The use of claim 1 which provides tumor angiogenesis and metastasis inhibition.

**6.** The use of claim 1 wherein said administration is associated with subjecting the mammal in need thereof to a radiation therapy or chemotherapy regimen in combination with at least one cytostatic of cytotoxic agent.

**7.** The use of claim 1 wherein the mammal in need thereof is a human.

**8.** Use of a compound of formulae (Ic), (Ie), (If) or (Ig) as defined in claim 1 for manufacturing a medicament for inhibiting protein kinase activity wherein said compound is to be contacted with said kinase in an effective amount.

**9.** A compound represented by formulae (Ic), (Ie), (If) or (Ig)

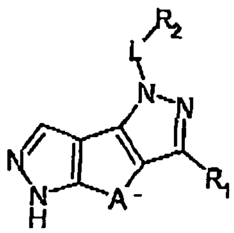

(Ic)

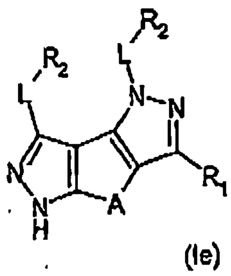

(Ie)

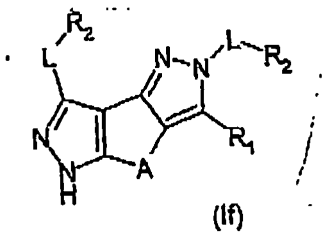

(If)

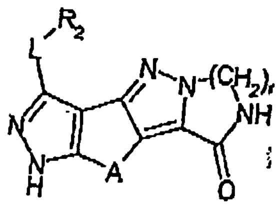

(Ig)

wherein:

$R_1$ in formula (Ic) is selected from the group consisting of perfluorinated lower alkyl, heterocyclyl, CN, $CO_2R'$, $COCF_3$, COR', CONR'R", NR'R", C(=NR')NR'R", $CONHNH_2$, CONHOR', NHCOR', $CH_2NH_2$, and $CH_2NHCOR'$; or

$R_1$ in formulae (Ie) and (If) is selected from the group consisting of CN, $CO_2R'$, COR', CONR'R", NR'R", C (=NOH)NR'R", $CONHNH_2$, CONHOR',

R' and R" in formula (Ic) are selected, each independently, from the group consisting of hydrido, hydroxy, alkyl, hydroxyalkyl, alkenyl, alkynyl, aryl, arylalkyl, heterocyclyl or heterocyclylalkyl; or

R' and R" in formulae (Ie) and (If) are selected, each independently, from the group consisting of hydrido or lower alkyl;

A in formula (Ic) is selected from the group consisting of $-CH_2-$; $-CH_2-CH_2-$; $-CH=CH-$; $(CH_2)-C(CH_3)_2$;

A in formulae (Ie), (If) and (Ig) is selected from the group consisting of $-CH_2-CH_2-$; -CH=CH-; $(CH_2)-C(CH_3)_2$;
L represents, each independently, a single bond, an alkylidene group or a divalent group selected from NH, NHCO, CONH, NHCONH, $SO_2NH$ and $NHSO_2$;
$R_2$ is, each independently, hydrido, alkyl, 5 to 12 membered mono- or bi-cyclic ring having from 0 to 3 heteroatoms selected from S, O and N, optionally substituted with one or more $-(CH2)_q-R_3$ groups; or $R_2$ is a group of formula

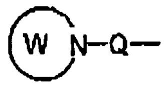

W is a 3 to 7 membered ring having one N heteroatom directly linked to Q and from 0 to 2 additional heteroatoms selected from the group consisting of S, SO, $SO_2$, O, N and NR', wherein R' is as above defined;
Q is a divalent group selected from CO, $SO_2$ and $(CH_2)_n$;
$R_3$ is selected, each independently, from the group consisting of alkyl, halogen, $CF_3$, $OCF_3$,$NO_2$, CN, C(=NR') NR'R", OR', SR', OCOR', OCONR'R", $COCF_3$, COR', $CO_2R'$, CONR'R", $SO_2R'$, $SO_2NR'R"$, NR'R", NR'COR', NR'COOR', NR'CONR'R", $NR'SO_2R'$, $NR'SO_2NR'R"$, wherein R' is as above defined;
n is, each independently, 0,1, or 2;
q is, each independently, 0 or an integer from 1 to 3;
r is an integer from 1 to 3;

or isomers, tautomers, carriers; prodrugs, and pharmaceutically acceptable salts thereof.

**10.** A compound of formula (Ic) according to claim 9 wherein each L is independently selected from methylene or a single bond and each R2 is independently selected from hydrido, phenyl or a 5 or 6 membered aromatic heterocycle having 1 or 2 heteroatoms selected among N, O and S.

**11.** A compound of formula (Ic) according to claim 10 wherein $R_2$, being optionally further substituted as defined in claim 9, is selected from the group consisting of hydrido phenyl, pyridyl, pyridazinyl or pyrimidinyl.

**12.** A compound of formulae (Ic), (Ie), (If) or (Ig) as defined in claim 9, optionally in the form of a pharmaceutically acceptable salt, selected from the group consisting of:

1. Ethyl 1-(4-methoxyphenyl)-1,4,5,6-tetrahydropyrazolo[3,4-e]indazole-3-carboxylate;
2. Ethyl 1-[4-(aminosulfonyl)phenyl]-1,4,5,6,-tretrahydropyrazolo[3,4-e]indazole-3-carboxylate;
3. Ethyl 1-{4-[(methylamino)sulfonyl]phenyl}-1,4,5,6-tetrahydropyrazolo[3,4-e]indazole-3-carboxylate;
4. Ethyl 1-{4-[(butylamino)sulfonyl]phenyl}-1,4,5,6-tetrahydropyrazolo[3,4-e]indazole-3-carboxylate;
5. Ethyl 1-{4-[(dimethylamino)sulfonyl}phenyl}-1,4,5,6-tetrahydropyrazolo[3,4-e]indazole-3-carboxylate;
6. Ethyl 1-{4-(diprop-2-ynylamino)sulfonyl]phenyl}-1,4,5,6-tetrahydropyrazolo[3,4-e]indazole-3-carboxylane;
7. Ethyl 1-[4-(aminosulfonyl)phenyl]-1,4,5,6-tetrahydropyrazolo[3,4-e]indazole-3-carboxylate;
8. Ethyl 1-[4-(methylsulfonyl)phenyl]-1,4,5,6-tetrahydropyrazolo[3,4-e]indazole-3-carboxylate;
9. Ethyl 1-(4-{[(2-hydroxypropyl)amino]sulfonyl}phenyl)-1,4,5,6-tetrahydropyrazolo-[3,4-e]indazole-3-carboxylate;
10. Ethyl 1-[4-(aminocarbonyl)phenyl]-1,4,5,6-tetrahydropyrazolo[3,4-e]indazole-3-carboxylate;
11. Ethyl 1,4,5,6-tetrahydropyrazolo[3,4-e]indazole-3-carboxylate;
12. Ethyl 1-phenyl-1,4,5,6-tetrahydropyrazolo[3,4-e]indazole-3-carboxylate;
13. Ethyl 1-(4-fluorophenyl)-1,4,5,6-tetrahydropyrazolo[3,4-e]indazole-3-carboxylate;
14. Ethyl 1-(4-bromophenyl)-1,4,5,6-tetrahydropyrazolo[3,4-e]indazole-3-carboxylate,
15. Ethyl 1-(4-methylphenyl)-1,4,5,6-tetrahydropyrazolo[3,4-e]indazole-3-carboxylate;
16. Ethyl 1-(4-chlorophenyl)-1,4,5,6-tetrahydropyrazolo[3,4-e]indazole-3-carboxylate;
17. Ethyl 1-(4-cyanophenyl)-1,4,5,6-tetrahydropyrazolo[3,4-e]indazole-3-carboxylate;
18. Ethyl 1-(4-nitrophenyl)-1,4,5,6-tetrahydropyrazolo[3,4-e]indazole-3-carboxylate;
19. Ethyl 1-[4-(trifluoromethyl)phenyl]-1,4,5,6-tetrahydropyrazolo[3,4-e]indazole-3-cuboxylate;
20. Ethyl 1-benzyl-1,4,5,6-tetrahydropyrazolo[3,4-e]indazole-3-carboxylate;
21. Ethyl 1-(3-hydroxybenzyl)-1,4,5,6-tetrahydropyrazolo[3,4-e]indazole-3-caxboxylate;
22. Ethyl 1-pyridin-2-yl-1,4,5,6-tetrahydropyrazolo[3,4-e]indazole-3-carboxylate;
23. Ethyl 1-(6-chloropyridazin-3-yl)-1,4,5,6-tetrahydropyrazolo[3,4-e]indazole-3-carboxylate;
24. Ethyl 1-[4(trifluoromethyl)pyrimidin-2-yl]-1,4,5,6-tetrahydropyrazolo[3,4-e]indazole-3-carboxylate,

25. Ethyl 1-(3-methylphenyl)-1,4,5,6-tehahydropyrazolo[3,4-e]indazole-3-carboxylate;
26. Ethyl 1-(3-chlorophenyl)-1,4,5,6-tetrahydropyrazolo[3,4-e]indazole-3-carboxylate;
27. Ethyl 1-(3-fluorophenyl)-1,4,5,6-tetrahydropyrazolo[3,4-e]indazole-3-carboxylate;
28. Ethyl 4,4-dimethyl-1-(4-methylphenyl)-1,4,5,6-tetrahydropyrazolo[3,4-e]indazole-3-carboxylate;
29. Ethyl 1-pyridin-3-yl-1,4,5,6-tetrahydropyrazolo[3,4-e]indazole-3-carboxylate;
30. Ethyl 1-[4(acetylamino)phenyl]-1,4,5,6-tetrahydropyrazolo[3,4-e]indazole-3-carboxylate;
31. Ethyl 1-{4-[(4-methylpiperazin-1-yl)sufonyl]phenyl}-1,4,5,6-tetrahydropyrazolo[3,4-e]indazole-3-carboxylate;
32. 4-[3-(ethoxycarbonyl)-5,6-dihydropyrazolo[3,4-e]indazol-1(4H)-yl]benzoic acid;
33. Ethyl 1-[4-(trifluoromethoxy)phenyl]-1,4,5,6-tetrahydropyrazolo[3,4-e]indazole-3-carboxylate;
34. Ethyl 1-butyl-1,4,5,6-tetrahydropyrazolo[3,4-e]indazole-3-carboxylate;
35. Ethyl 1-(2,5-dimethylphenyl)-1,4,5,6-tetrahydropyrazolo[3,4-e]indazole-3-carboxylate;
36. Ethyl 1-{4-[amino(imino)methyl]phenyl}-1,4,5,6-tetrahydropyrazolo[3,4-e]indazole-3-carboxylate hydrochloride;
37. Ethyl 1-[4-(1H-imidazol-2-yl)phenyl]-1,4,5,6-tetrahydropyrazolo[3,4-e]indazole-3-carboxylate hydrochloride;
38. Ethyl 1-methyl-1,4,5,6-tetrahydropyrazolo[3,4-e]indazole-3-carboxylate;
39. Ethyl 8-anilino-1-methyl-1,4,5,6-tetrahydropyrazolo[3,4-e]indazole-3-carboxylate;
40. Ethyl 8-anilino-1-(2,2,2-trifluoroethyl)-1,4,5,6-tetrahydropyrazolo[3,4-e]indazole-3-carboxylate;
41. Ethyl 8-anilino-2-{2-[(tert-butoxycarbonyl)amino]ethyl}-2,4,5,6-tetrahydropyrazolo[3,4-e]indazole-3-carboxylate;
42. Ethyl 8-amino-1,4,5,6-tetrahydropyrazolo[3,4-e]indazole-3-carboxylate;
43. 1-(4-methoxyphenyl)-1,4,5,6-tetrahydro-pyrazolo[3,4-e]indazole-3-carboxamide;
44. 1-[4-(aminosulfonyl)phenyl]-1,4,5,6-tetrahydropyrazolo[3,4-e]indazole-3-carboxamide;
45. 1-{4-[(methylamino)sulfonyl]phenyl}-1,4,5,6-tetrahydropyrazolo[3,4-e]indazole-3-carboxamide;
46. 1-{4-[(butylamino)sulfonyl]phenyl}-1,4,5,6-tetrahydropyrazolo[3,4-e]indazole-3-carboxamide;
47. 1-{4-[(dimethylamino)sulfonyl]phenyl}-1,4,5,6-tehahydropyrazolo[3,4-e]indazole-3-carboxamide;
48. 1-{4-[(diprop-2-ynylamino)sulfonyl]phenyl}-1,4,5,6-tetrahydropyrazolo[3,4-e]indazole-3-carboxamide;
49. 1-[4-(amilinosulfonyl)phenyl]-1,4,5,6-tetrahydropyrazolo[3,4-e]indazole-3-carboxamide;
50. 1-[4-(methylsulfonyl)phenyl]-1,4,5,6-tetrahydropyrazolo[3,4-e]indazole-3-carboxamide;
51. 1-[4-(aminocarbonyl)phenyl]-1,4,5,6-tetrahydropyrazolo[3,4-e]indazole-3-carboxamide;
52. 1-(4-{[(2-hydroxypropyl)amino]sulfonyl}phenyl)-1,4,5,6-tetrahydropyrazolo[3,4-e]indazole-3-carboxamide;
53. 1,4,5,6-tetrahydropyrazol[3,4-e]indazole-3-carboxamide;
54. 1-phenyl-1,4,5,6-tetrahydropyrazolo[3,4-e]indazole-3-carboxamide;
55. 1-(4-fluorophenyl)-1,4,5,6-tetrahydropyrazolo[3,4-e]indazole-3-carboxamide;
56. 1-(4-bromophenyl)-1,4,5,6-tetrahydropyrazolo[3,4-e]indazole-3-carboxamide;
57. 1-(4-nitrophenyl)-1,4,5,6-tetrahydropyrazolo[3,4-e]indazole-3-carboxamide;
58. 1-(4-methylphenyl)-1,4,5,6-tetrahydropyrazolo[3,4-e]indazole-3-carboxamide;
59. 1-(4-chlorophenyl)-1,4,5,6-tetrahydropyrazolo[3,4-e]indazole-3-carboxamide;
60. 1-(4-cyanophenyl)-1,4,5,6-tetrahydropyrazolo[3,4-e]indazole-3-carboxamide;
61. 1-[4-(trifluoromethyl)phenyl]-1,4,5,6-tetrahydropyrazolo[3,4-e]indazole-3-carboxamide;
62. 1-benzyl-1,4,5,6-tetrahydropyrazolo[3,4-e]indazole-3-carboxamide;
63. 1-(3-hydroxybenzyl)1,4,5,6-tetrahydropyrazolo[3,4-e]indazole-3-carboxamide;
64. 1-pyridin-2-yl-1,4,5,6-tetrahydropyrazolo[3,4-e]indazole-3-carboxamide;
65. 1-(3-methylphenyl)1,4,5,6-tetrahydropyrazolo[3,4-e]indazole-3-carboxamide;
66. 1-(3-chlorophenyl)-1,4,5,6,-tetrahydropyrazolo[3,4-e]indazole-3-carboxamide;
67. 1-(3-fluorophenyl)-1,4,5,6-tetrahydropyrazolo[3,4-e]indazole-3-carboxamide;
68. 1-(6-chloropyridazin-3-yl)-1,4,5,6-tetrahydropyrazolo[3,4-e]indaxole-3-carboxamide;
69. 4,4-dimethyl-1-(4-methylphenyl)-1,4,5,6-tetrahydropyrazolo[3,4-e]indazole-3-carboxamide;
70. 1-pyridin-3-yl-1,4,5,6-tetrahydropyrazolo[3,4-e]indazole-3-carboxamide;
71. 1-[4-(acetylamino)phenyl]-1,4,5,6-tetrahydropyrazolo[3,4-e]indazole-3-carboxamide;
72. 1-(4-aminophenyl)-1,4,5,6-tetrahydropyrazolo[3,4-e]indazole-3-carboxamide;
73. 1-{4-[(4-methylpiperazin-1-yl)sulfonyl]phenyl}-1,4,5,6-tetrahydropyrazolo[3,4-e]indazole-3-carboxamide;
74. 4-[3-(aminocarbonyl)-5,6-dihydropyrazolo[3,4-e]indazol-1(4H)-yl]benzoic acid;
75. 1-(4-morpholin-4-ylphenyl)-1,4,5,6-tetrahydropyrazolo[3,4-e]indazole-3-carboxamide;
76. 1-[4-(trifluoromethoxy)phenyl]-1,4,5,6-tétrahydropyrazolo[3,4-e]indazole-3-carboxamide;
77. 1-butyl-1,4,5,6-tetrahydropyrazolo[3,4-e]indazole-3-carboxamide;
78. 1-(2-hydroxyethyl)-1,4,5,6-tetrahydropyrazolo[3,4-e]indazole-3-carboxamide;

79. 1-(2,5-dimethylphenyl)-1,4,5,6-tetrahydropyrazolo[3,4-e]indazole-3-carboxamide;
80. 1-(2,2,2-trifluoroethyl)-1,4,5,6-tetrahydropyrazole[3,4-c]indazole-3-carboxamide;
81. 1-(2-amino-2-oxoethyl)-1,4,5,6-tetrahydropyrazolo[3,4-e]indazole-3-carboxamide;
82. 1-[4-(1H-imidazol-2-yl)phenyl]-1,4,5,6-tetrahydropyrazole[3,4-c]indazole-3-carboxamide;
83. 4,4-dimethyl-1-(2,2,2-trifluoroethyl)-1,4,5,6-tetrahydropyrazole[3,4-e]indazole-3-carboxamide;
84. 1-methyl-1,4,5,6-tetrahydropyrazolo[3,4-e]indazole-3-carboxamide;
85. 2-(2-hydroxyethyl)-2,4,5,6-tetrahydropyrazolo[3,4-e]indazole-3-carboxamide;
86. 8-Anilion-1-methyl-1,4,5,6-tetrahydropyrazolo[3,4-c]indazole-3-carboxamide;
87. 8-Anilino-1-(2,2,2-trifluoroethyl)-1,4,5,6-tetrahydropyrazolo[3,4-e]indazole-3-carboxamide;
88. 8-amino-1,4,5,6-tetrahydropyrazolo[3,4-e]indazole-3-carboxamide;
89. 1-[4-methoxyphenyl]-1,6-dihydropyrazolo[3,4-e]indazole-3-carboxamide;
90. 1-[4-(aminosulfonyl)phenyl]-1,6-dihydropyrazolo[3,4-e]indazole-3-carboxamide;
91. 1-{4-[(methylamino)sulfonyl]phenyl}-1,6-dihydropyrazolo[3,4-e]indazole-3-carboxamide;
92. 1-{4-[(butylamino)sulfonyl]phenyl}-1,6-dihydropyrazolo[3,4-e]indazole-3-carboxamide;
93. 1-{4[(dimethylamino)sulfonyl]phenyl}-1,6-dihydropyrazolo[3,4-e]indazole-3-carboxamide;
94. 1-{4-[(diprop-2-ynylamino)sulfonyl]phenyl}-1,6-dihydropyrazole[3,4-e]indazole-3-carboxamide;
95. 1-[4-anilinosulfonyl)phenyl]-1,6-dihydropyrazole[3,4-e]indazole-3-carboxamide;
96. 1-(4-{[(2-hydroxypropyl)amino]sulfonyl}phenyl)-1,6-dihydropyrazolo[3,4-e]indazole-3-carboxamide;
97. 1-[4-(methylsufonyl)phenyl]-1,6-dihydropyrazolo[3,4-c]indazole-3-carboxamide
98. 1,6-dihydropyrazolo[3,4-e]indazole-3-carboxamide;
99. 1-phenyl-1,6-dihydropyrazolo[3,4-e]indazole-3-carboxamide;
100. 1-(4-fluorophenyl)-1,6-dihydropyrazolo[3,4-e]indazole-3-carboxamide
101. 1-(4-methylphenyl)-1,6-dihydropyrazolo[3,4-e]indazole-3-carboxamide;
102. 1-(4-cyanophenyl)-1,6-dihydropyrazolo[3,4-e]indazole-3-carboxamide;
103. 1-[4-(trifluoromethyl)phenyl]-1,6-dihydropyrazolo[3,4-e]indazole-3-carboxamide;
104. 1-(4-chlorophenyl)-1,6-dihydropyrazolo[3,4-e]indazole-3-carboxamide;
105. 1-(4-bromophenyl)-1,6-dihydropyrazolo[3,4-e]indazole-3-carboxamide;
106. 1-(4-nitrophenyl)-1,6-dihydropyrazolo[3,4-e]indazole-3-carboxamide
107. 1-benzyl-1,6-dihydropyrazolo[3,4-e]indazole-3-carboxamide;
108. 1-(3-hydroxybenzyl)-1,6-dihydropyrazolo[3,4-e]indazole-3-carboxamide;
109. 1-pyridin-2-yl-1,6-dihydropyrazolo[3,4-e]indazole-3-carboxamide;
110. 1-(3-chlorophenyl)-1,6-dihydropyrazolo[3,4-e]indazole-3-carboxamide;
111. 1-(3-methylphenyl)-1,6-dihydropyrazolo[3,4-e]indazole-3-carboxamide;
112. 1-(3-fluorophenyl)-1,6-dihydropyrazolo[3,4-e]indazole-3-carboxamide;
113. 1-(6-chloropyridazin-3-yl)-1,6-dihydropyazolo[3,4-e]indazole-3-carboxamide;
114. 1-(4-methoxyphenyl)-1,6-dihydropyrazolo[3,4-e]indazole-3-carboxylic acid;
115. Ethyl 1-phenyl-1,6-dihydropyrazolo[3,4-e]indazole-3-carboxylate;
116. Ethyl 1-(4-methoxyphenyl)-1,6-hydropyrazolo[3,4-e]indazole-3-carboxylate;
117. N-methyl-1-[4-(aminosulfonyl)phenyl]-1,6-dihydropyrazolo[3,4-e]indazole-3-carboxamide;
118. N-methyl-1-{4-[(butylamino)sulfonyl]phenyl}-1,6-dihydropyrazolo[3,4-e]indazole-3-carboxamide;
119. N-methyl-1-{4-[(dimethylamino)sulfonyl]phenyl}-1,6-dihydropyrazolo[3,4-e]indazole-3-carboxamide;
120. N-methyl-1-[4-(methylsulfonyl)phenyl]-1,6-dihydropyrazolo[3,4-e]indozole-3-carboxamide;
121. N-(allyloxy)-1-{4-[(butylamino)sulfonyl]phenyl}-1,6-dihydropyrazolo[3,4-e]indazole-3-carboxamide,
122. 7,8,9,10-tetrahydro[1,4]diazepino[1,2-b]pyrazolo[3,4-g]indazol-6(3H)one;
123. 1-pyridin-3-yl-1,6-dihydropyrazolo[3,4-e]indazole-3-carboxamide;
124. 1-[4-(acetylamino)phenyl]-1,6-dihydropyrazolo[3,4-e]indazole-3-carboxamide;
125. 4-[3-(aminocarbonyl)pyrazolo[3,4-e]indazol-1(6H)-yl]benzoic acid;
126. 1-{4-[(4-methylpiperazin-1-yl)sulfonyl]phenyl}-1,6-dihydropyrazolo[3,4-e]indazole-3-carboxamide;
127. 1-[4-(trifluoromethoxy)phenyl]-1,6-dihydropyrazolo[3,4-e]indazole-3-carboxamide;
128. 4-[3-(ethoxycarbonyl)pyrazolo[3,4-e]indazol-1(6H)-yl]benzoic acid;
129. 1-(4-morpholin-4-ylphenyl)-1,6-dihydropyrazolo[3,4-e]indazole-3-carboxamide;
130. 1-(2-hydroxyethyl)-1,6-dihydropyrazolo[3,4-e]indazole-3-carboxamide;
131. 1-(2,5-dimethylphenyl)-1,6-dihydropyrazolo[3,4-e]indazole-3-carboxamide;
132. 1-(2-aminoethyl)-1,6-dihydropyrazolo[3,4-e]indazole-3-carboxamide hydrochloride;
133. 1-(2,2,2-trifluomethyl)-1,6-dihydropyrazolo[3,4-e]indazole-3-carboxamide;
134. 1-[4-(1H-imidazol-2-yl)phenyl]-1,6-dihydropyrazolo[3,4-e]indazole-3-carboxamide;
135. 1-methyl-1,6-dihydropyrazolo[3,4-e]indazole-3-carboxamide;
136. 8,9-dihydro-3H-pyrazino[1,2-b]pyrazolo[3,4-g]indazol-6(7H)-one;

137. 2-(2-aminoethyl)-2,6-dihydropyrazolo[3,4-e]indazole-3-carboxamide hydrochloride;
138. 2-(2-hydroxyethyl)-2,6-dihydropyrazolo[3,4-e]indazole-3-carboxamide;
139. 2-methyl-2,6-dihydropyrazolo[3,4-e]indazole-3-carboxamide;
140. 1-anilino-8,9-dihydro-3H-pyrazino[1,2-b]pyrazolo[3,4-g]indazol-6(7H)-one;
141. 1-(4-methoxy-phenyl)-1,6-dihydropyrazolo[3,4-e]indazol-3-amine;
142. 1-[1-(4-methylphenyl)-1,6-dihydropyrazolo[3,4-e]indazol-3-yl]ethanone;
143. 1-(4-methoxyphenyl)-1,6-dihydropyrazolo[3,4-e]indazole-3-carbonitrile;
144. 1-(4-methoxyphenyl)-1,6-dihydropyrazolo[3,4-e]indazole-3-carbohydrazide;
145. 1-(4-methoxyphenyl)-1,4,5,6-tetrahydropyrazolo[3,4-e]indazole-3-carbohydrazide;
146. N'-hydroxyl-(4-methoxyphenyl)-1,6-dihydropyrazolo[3,4-e]indazole-3-carboximidamide;
147. 1-(4-methoxyphenyl)-1,6-dihydropyrazolo[3,4-e]indazole-3-carboxylic acid;
148. 1-(4-bromophenyl)-1,4,5,6-tetrahydropyrazolo[3,4-e]indazole-3-carboxylic acid;
149. 1-{4-[(butylamino)sulfonyl]phenyl}-1,4,5,6-tetrahydropyrazolo[3,4-e]indazole-3-carboxylic acid;
150. 4,4-dimethyl-1-(4-methylphenyl)-1,4,5,6-tetrahydropyrazolo[3,4-e]indazole-3-carboxylic acid;
151. N-hydroxyl-1-(4-methoxyphenyl)-1,6-dihydropyrazolo[3,4-e]indazole-3-carboxamide;
152. N-(allyloxy)-1-{4-[(butylamino)sulfonyl]phenyl}-1,4,5,6-tetrahydropyrazolo[3,4-e]indazole-3-carboxamide;
153. N-(allyloxy)-1-(4-methoxyphenyl)-1,4,5,6-tetrahydropyrazolo[3,4-e]indazole-3-carboxamide;
154. N-methyl-1-[4-(methylsulfonyl)phenyl]-1,4,5,6-tetrahydropyrazolo[3,4-e]indazole-3-carboxamide;
155. 1-[4-(aminosulfonyl)phenyl]-N-methyl-1,4,5,6-tetrahydropyrazolo[3,4-e]indazole-3-carboxamide;
156. 1-{4-[(butylamino)sulfonyl]phenyl}-N-methyl-1,4,5,6-tetrahydropyrazolo[3,4-e]indazole-3-carboxamide;
157. 1-{4-[(dimethylamino)sulfonyl]phenyl}-N-methyl-1,4,5,6-tetrahydropyrazolo[3,4-e]indazole-3-carboxamide;
158. Ethyl 2-(3-aminopropyl)-2,4,5,6-tetrahydropyrazolo[3,4-e]indazole-3-carboxylate hydrochloride;
159. 4,5,7,8,9,10-hexahydro[1,4]diazepino[1,2-b]pyrrolo[3,4-g]indazol-6(3H)-one;
160. 5,5-dimethyl-4,5,7,8,9,10-hexahydro[1,4]diazepino[1,2]pyrazolo[3,4-g]indazol-6(3H)-one;
161. 5,5-dimethyl-4,5,8,9-tetrahydro-3H-pyrazino[1,2-b]pyrazolo[3,4-g]indazol-6(7H)-one;
162. 4,5,8,9-tetrahydro-3H-pyrazino[1,2-b]pyrazolo[3,4-g]indazol-6(7H)-one;
163. 1-anilino-4,5,8,9-tetrahydro-3H-pyrazino[1,2-b]pyrazolo[3,4-g]indazol-6(7H)-one.

**13.** A process for preparing a compound of formula (Ic) as defined in claim 9

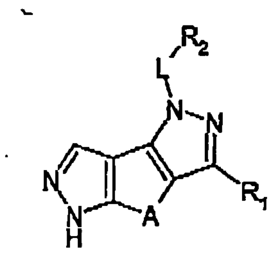

(Ic)

wherein L and $R_2$ are as defined in claim 16, $R_1$ is a group -COOEt or $-CONH_2$, and A is selected from the group consisting of $-CH_2-$, $-CH_2CH_2-$, -CH=CH- and $-CH_2-C(CH_3)_2-$, which process comprises:

a) reacting the compound (10) with hydrazine dihydrochloride, so as to obtain the compound (11)

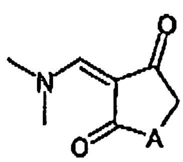

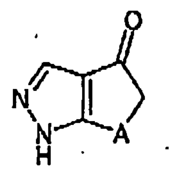

(10) (11)

wherein A is as above defined, other than -CH=CH-;
b) reacting the compound (11) with trityl chloride, so as to obtain the compound (12)

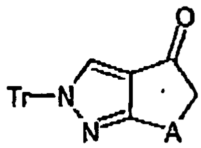

(12)

wherein Tr stands for trityl, and condensing it with oxalyl chloride so as to obtain the compound (13)

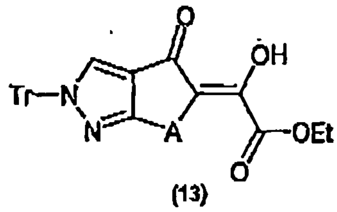

(13)

c) reacting the compound (13) with a substituted hydrazine (8)

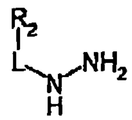

(8)

Wherein L and $R_2$ are as defined in claim 16; so as to obtain a compound of formula (Ic) wherein $R_1$ is a group -COOEt and A is as above defined except -CH=CH-; and, optionally
d) reacting this latter with ammonium hydroxide so as to obtain the corresponding derivative of formula (Ic) wherein $R_1$ is $-CONH_2$; and, optionally
e) reacting the compound of formula (Ic) wherein A is $-CH_2-CH_2-$, as obtained in steps c) or d), with a suitable oxidizing agent so as to obtain the corresponding derivative of formula (Ic) wherein A is -CH=CH-.

**14.** The process of claim 13 wherein, in step e), the oxidizing agent is 2.3-dichloro-5,6-dicyano-1,4-benzoquinone.

**15.** A pharmaceutical composition comprising an effective amount of a compound of formula (I) as defined in claim 9 and, at least, one pharmaceutically acceptable excipient, carrier or diluent.

**16.** A pharmaceutical composition according to claim 15 further comprising one or more chemotherapeutic agents, as a combined preparation for simultaneous, separate or sequential use in anticancer therapy.

**17.** A product or kit comprising a compound of claim 9 or a pharmaceutical composition thereof as defined, in claim 15, and one or more chemotherapeutic agents, as a combined preparation for simultaneous, separate or sequential use in anticancer therapy.

**18.** Use of a compound of formula (Ic), (Ie), (If) or (Ig) or a pharmaceutically acceptable salt thereof, as defined in claim 9, in the manufacture of a medicament for treating diseases caused by and/or associated with an altered protein kinase activity.

**19.** Use according to claim 1 for treating tumors,

## Patentansprüche

**1.** Verwendung einer wirksamen Menge einer Verbindung der Formeln (1c), (1e), (1f) oder (1g) zur Herstellung eines Medikaments zur Behandlung von Krankheiten, die ausgelöst werden durch und/oder in Zusammenhang stehen mit einer veränderten Proteinkinase-Aktivität, durch Verabreichen an ein Säugetier, das dies benötigt,

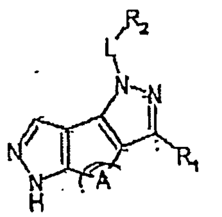
(Ic)

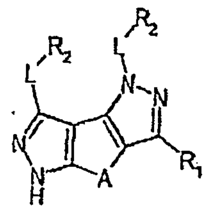
(Ie)

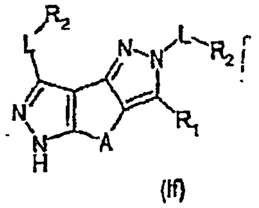
(If)

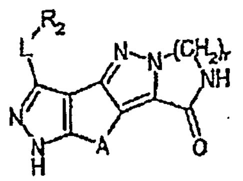
(Ig)

worin:

$R_1$ in Formel (1c) gewählt ist aus der Gruppe bestehend aus Hydrido, Niederalkyl, perfluoriertem Niederalkyl, Heterocyclyl, CN, $CO_2R'$, $COCF_3$, COR', CONR'R", NR'R", C(=NR')NR'R", $CONHNH_2$, CONHOR', NHCOR', $CH_2NH_2$ und $CH_2NHCOR'$; oder
$R_1$ in Formeln (1e) und (1f) ist gewählt aus der Gruppe bestehend aus CN, $CO_2R'$, COR', CONR'R", NR'R", C (=NOH)NR'R", $CONHNH_2$, CONHOR';
R' und R" in Formel (1c) sind, jeweils unabhängig, gewählt aus der Gruppe bestehend aus Hydrido, Hydroxy, Alkyl, Hydroxyalkyl, Alkenyl, Alkinyl, Aryl, Arylalkyl, Heterocyclyl oder Heterocyclylalkyl; oder
R' und R" in Formeln (1e) und (1f) sind, jeweils unabhängig, gewählt aus der Gruppe bestehend aus Hydrido oder Niederalkyl;
A in Formeln (1c) ist gewählt aus der Gruppe bestehend aus $-CH_2-$; $-CH_2-CH_2-$; -CH=CH; $(CH_2)-C(CH_3)_2$; oder
A in Formeln (1e), (1f) und (1g) ist gewählt aus der Gruppe bestehend aus $-CH_2-CH_2-$; -CH=CH; $(CH_2)-C(CH_3)_2$;
L stellt, jeweils unabhängig, eine Einzelbindung, eine Alkylidengruppe oder eine bivalente Gruppe, gewählt aus NH, NHCO, CONH, NHCONH, $SO_2NH$ und $NHSO_2$ dar;
$R_2$ ist, jeweils unabhängig, Hydrido, Alkyl, 5- bis 12- gliedriger mono- oder bicyclischer Ring mit von 0 bis 3 Heteroatomen, gewählt aus S, 0 und N, wahlweise substituiert mit einer oder mehreren $-(CH_2)_4-R_3$ Gruppen; oder $R_2$ ist eine Gruppe der Formel

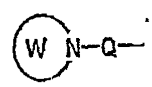

W ist ein 3- bis 7-gliedriger Ring mit einem N-Heteroatom, direkt an Q geknüpft, und von 0 bis 2 zusätzlichen Heteroatomen, gewählt aus der Gruppe bestehend aus S, SO, $SO_2$, O, N und NR', worin R' wie oben definiert ist;
Q ist eine bivalente Gruppe, gewählt aus CO, $SO_2$ und $(CH_2)_a$;
$R_3$ ist, jeweils unabhängig, gewählt aus der Gruppe bestehend aus Alkyl, Halogen, $CF_3$, $OCF_3$, $NO_2$, CN, C (=NR')NR'R", OR', SR', OCOR', OCONR'R", $COCF_3$, COR', $CO_2R'$, CONR'R", $SO_2R'$, $SO_2NR'R"$, NR'R",

NR'COR', NR'COOR', NR'CONR'R", NR'$SO_2$R', NR'$SO_2$NR'R", worin R' und R" wie oben definiert sind;
n ist, jeweils unabhängig, 0, 1 oder 2;
q ist, jeweils unabhängig, 0 oder eine ganze Zahl von 1 bis 3;
r ist eine ganze Zahl von 1 bis 3;

oder Isomere, Tautomere, Träger, Prodrugs und pharmazeutisch verträgliche Salze davon.

2. Die Verwendung von Anspruch 1, worin die Krankheit, die ausgelöst wird durch und/oder in Zusammenhang steht mit einer veränderten Proteinkinase-Aktivität, eine Zell-proliferative Erkrankung ist, gewählt aus der Gruppe bestehend aus Krebs, Alzheimer'scher Krankheit, viralen Infektionen, AutoimmunErkrankungen und neurodegenerativen Krankheiten.

3. Die Verwendung von Anspruch 2, worin der Krebs gewählt ist aus Karzinom, Plattenepithelkarzinom, hämatopoietischen Tumoren lymphoider oder myeloider Abstammung, Tumoren mesenchymalen Ursprungs, Tumoren des zentralen und peripheren Nervensystems, Melanom, Seminom, Teratokarzinom, Osteosarkom, Xeroderma Pigmentosum, Keratoxanthom, Schilddrüsenfollikelkrebs und Kaposi-Sarkom.

4. Die Verwendung von Anspruch 1, worin die Zell-proliferative Erkrankung gewählt ist aus benigner Prostatahyperplasie, familiärer Adenomatosis, Polyposis, Neurofibromatose, Psoriasis, glatter Gefäßzell-Proliferation in Zusammenhang mit Atherosklerose, Lungenfibrose, Arthritis glomerulonephritis und post-operativer Stenose und Restenose.

5. Die Verwendung von Anspruch 1, welche Tumorangiogenese- und Metastase-Hemmung bereitstellt.

6. Die Verwendung von Anspruch 1, worin die Verabreichung mit dem Aussetzen des Säugetiers, das dies benötigt, gegenüber einer Strahlungstherapie oder einem Chemotherapie-Regime in Kombination mit mindestens einem Zytostatikum eines zytotoxischen Wirkstoffs in Zusammenhang steht.

7. Die Verwendung von Anspruch 1, worin das Säugetier, das dies benötigt, ein Mensch ist.

8. Verwendung einer Verbindung der Formeln (1c), (1e), (1f) oder (1g), wie in Anspruch 1 definiert, zur Herstellung eines Medikaments zur Hemmung der Proteinkinase-Aktivität, worin die Verbindung in einer wirksamen Menge mit der Kinase in Kontakt gebracht werden soll.

9. Eine Verbindung dargestellt durch Formeln (1c), (1e), (1f), oder (1g)

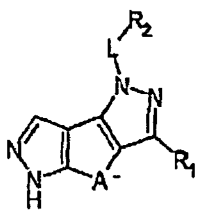

(1c)

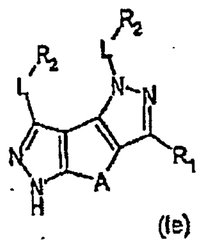

(1e)

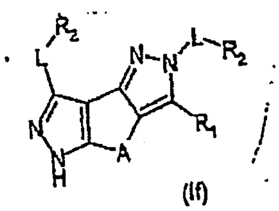

(1f)

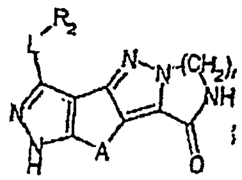

(1g)

worin:

$R_1$ in Formel (1c) gewählt ist aus der Gruppe bestehend aus perfluoriertem Niederalkyl, Heterocyclyl, CN,

$CO_2R'$, $COCF_3$, COR', CONR'R", NR'R", C(=NR')NR'R", $CONHNH_2$, CONHOR', NHCOR', $CH_2NH_2$ und $CH_2NHCOR'$; oder
$R_1$ in Formeln (1e) und (1f) ist gewählt aus der Gruppe bestehend aus CN, $CO_2R'$, COR', CONR'R", NR'R", C (=NOH)NR'R", $CONHNH_2$, CONHOR';
R' und R" in Formel (1c), sind, jeweils unabhängig, gewählt aus der Gruppe bestehend aus Hydrido, Hydroxy, Alkyl, Hydroxyalkyl, Alkenyl, Alkynyl, Aryl, Arylalkyl, Heterocyclyl oder Heterocyclylalkyl; oder
R' und R" in Formeln (1e) und (1f), sind, jeweils unabhängig, gewählt aus der Gruppe bestehend aus Hydrido oder Niederalkyl;
A in Formel (1c) ist gewählt aus der Gruppe bestehend aus $-CH_2-$; $-CH_2-CH_2-$; -CH=CH; $(CH_2)-C(CH_3)_2$;
A in Formeln (1e), (1f) und (1g) ist gewählt aus der Gruppe bestehend aus $-CH_2-CH_2-$; -CH=CH, $(CH_2)-C(CH_3)_2$;
L stellt, jeweils unabhängig, eine Einzelbindung, eine Alkylidengruppe oder eine bivalente Gruppe, gewählt aus NH, NHCO, CONH, NHCONH, $SO_2NH$ und $NHSO_2$, dar;
$R_2$ ist, jeweils unabhängig, Hydrido, Alkyl, 5- bis 12- gliedriger mono- oder bizyklischer Ring mit von 0 bis 3 Heteroatomen, gewählt aus S, O und N, wahlweise substituiert mit einer oder mehreren $-(CH_2)_q-R_3$ Gruppen, oder $R_2$ ist eine Gruppe der Formel

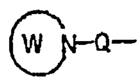

W ist ein 3- bis 7-gliedriger Ring mit einem N-Heteroatom, direkt an Q geknüpft, und von 0 bis 2 zusätzlichen Heteroatomen, gewählt aus der Gruppe bestehend aus S, SO, $SO_2$, 0, N und NR', worin R' wie oben definiert ist;
Q ist eine bivalente Gruppe gewählt aus CO, $SO_2$ und $(CH_2)_n$;
$R_3$ ist, jeweils unabhängig, gewählt aus der Gruppe bestehend aus Alkyl, Halogen, $CF_3$, $OCF_3NO_2$, CN, C (=NR')NR'R", OR', SR', OCOR', OCONR'R", $COCF_3$, COR', $CO_2R'$, CONR'R", $SO_2R'$, $SO_2NR'R"$, NR'R", NR'COR', NR'COOR', NR'CONR'R", $NR'SO_2R'$, $NR'SO_2NR'R"$, worin R' wie oben definiert ist;
n ist, jeweils unabhängig, 0, 1 oder 2;
q ist, jeweils unabhängig, 0 oder eine ganze Zahl von 1 bis 3;
r ist eine ganze Zahl von 1 bis 3;

oder Isomere, Tautomere, Träger, Prodrugs und pharmazeutisch verträgliche Salze davon.

**10.** Eine Verbindung der Formel (1c) gemäß Anspruch 9, worin jedes L unabhängig gewählt ist aus Methylen oder einer Einzelbindung, und jedes $R_2$ ist unabhängig gewählt aus Hydrido, Phenyl oder einem 5- oder 6-gliedrigen aromatischen Heterozyklus mit einem oder zwei Heteroatomen, gewählt aus N, 0 und S.

**11.** Eine Verbindung der Formel (1c) gemäß Anspruch 10, worin $R_2$, welches wahlweise weiter substituiert ist, wie in Anspruch 9 definiert, gewählt ist aus der Gruppe bestehend aus Hydrido, Phenyl, Pyridyl, Pyridazinyl oder Pyrimidinyl.

**12.** Eine Verbindung der Formeln (1c), (1e), (1f) oder (1g), wie in Anspruch 9 definiert, wahlweise in der Form eines pharmazeutisch verträglichen Salzes, gewählt aus der Gruppe bestehend aus:

1. Ethyl 1-(4-Methoxyphenyl)-1,4,5,6-tetrahydropyrazol[3,4-e]indazol-3-carboxylat;
2. Ethyl 1-[4-(Aminosulfonyl)phenyl]-1,4,5,6-tetrahydropyrazol[3,4-e]indazol-3-carboxylat;
3. Ethyl 1-{4-[(Methylamino)sulfonyl]phenyl}-1,4,5,6-tetrahydropyrazol[3,4-e]indazol-3-carboxylat;
4. Ethyl 1-{4-[(Butylamino)sulfonyl]phenyl}-1,4,5,6-tetrahydropyrazol[3,4-e]indazol-3-carboxylat;
5. Ethyl 1-{4-[(Dimethylamino)sulfonyl]phenyl}-1,4,5,6-tetrahydropyrazol[3,4-e]indazol-3-carboxylat;
6. Ethyl 1-{4-[(Diprop-2-inylamino)sulfonyl]phenyl}-1,4,5,6-tetrahydropyrazol[3,4-e]indazol-3-carboxylat;
7. Ethyl 1-[4-(Anilinosulfonyl)phenyl]-1,4,5,6-tetrahydropyrazol[3,4-e]indazol-3-carboxylat;
8. Ethyl 1-[4-(Methylsulfonyl)phenyl]-1,4,5,6-tetrahydropyrazol[3,4-e]indazol-3-carboxylat;
9. Ethyl 1-(4-{[(2-Hydroxypropyl)amino]sulfonyl}phenyl)-1,4,5,6-tetrahydropyrazol[3,4-e]indazol-3-carboxylat;
10. Ethyl 1-[4-(Aminocarbonyl)phenyl]-1,4,5,6-tetrahydropyrazol[3,4-e]indazol-3-carboxylat;
11. Ethyl 1,4,5,6-tetrahydropyrazol[3,4-e]indazol-3-carboxylat;
12. Ethyl 1-Phenyl-1,4,5,6-tetrahydropyrazol[3,4-e]indazol-3-carboxylat;
13. Ethyl 1-(4-fluorphenyl)-1,4,5,6-tetrahydropyrazol[3,4-e]indazol-3-carboxylat;
14. Ethyl 1-(4-Bromphenyl)-1,4,5,6-tetrahydropyrazol[3,4-e]indazol-3-carboxylat;
15. Ethyl 1-(4-Methylphenyl)-1,4,5,6-tetrahydropyrazol[3,4-e]indazol-3-carboxylat;

16. Ethyl 1-(4-Chlorphenyl)-1,4,5,6-tetrahydropyrazol[3,4-e]indazol-3-carboxylat;
17. Ethyl 1-(4-Cyanophenyl)-1,4,5,6-tetrahydropyrazol[3,4-e]indazol-3-carboxylat;
18. Ethyl 1-(4-Nitrophenyl)-1,4,5,6-tetrahydropyrazol[3,4-e]indazol-3-carboxylat;
19. Ethyl 1-[4-(Trifluormethyl)phenyl]-1,4,5,6-tetrahydropyrazol[3,4-e]indazol-3-carboxylat;
20. Ethyl 1-Benzyl-1,4,5,6-tetrahydropyrazol[3,4-e]indazol-3-carboxylat;
21. Ethyl 1-(3-Hydroxybenzyl)-1,4,5,6-tetrahydropyrazol[3,4-e]indazol-3-carboxylat;
22. Ethyl 1-Pyridin-2-yl-1,4,5,6-tetrahydropyrazol[3,4-e]indazol-3-carboxylat;
23. Ethyl 1(6-Chlorpyridazin-3-yl)-1,4,5,6-tetrahydropyrazol[3,4-e]indazol-3-carboxylat;
24. Ethyl 1-[4-(Trifluormethyl)pyrimidin-2-yl]-1,4,5,6-tetrahydropyrazol[3,4-e]indazol-3-carboxylat;
25. Ethyl 1-(3-Methylphenyl)-1,4,5,6-tetrahydropyrazol[3,4-e]indazol-3-carboxylat;
26. Ethyl 1-(3-Chlorphenyl)-1,4,5,6-tetrahydropyrazol[3,4-e]indazol-3-carboxylat;
27. Ethyl 1-(3-Fluorphenyl)-1,4,5,6-tetrahydropyrazol[3,4-e]indazol-3-carboxylat;
28. Ethyl 4,4-Dimethyl-1-(4-methylphenyl)-1,4,5,6-tetrahydropyrazol[3,4-e]indazol-3-carboxylat;
29. Ethyl 1-Pyridin-3-yl-1,4,5,6-tetrahydropyrazol[3,4-e]indazol-3-carboxylat;
30. Ethyl 1-[4-(Acetylamino)phenyl]-1,4,5,6-tetrahydropyrazol[3,4-e]indazol-3-carboxylat;
31. Ethyl 1-{4-[(4-Methylpiperazin-1-yl)sulfonyl]phenyl}-1,4,5,6-tetrahydropyrazol[3,4-e]indazol-3-carboxylat;
32. 4-[3-(Ethoxycarbonyl)-5,6-dihydropyrazol[3,4-e]indazol-1(4H)-yl]benzoesäure;
33. Ethyl 1-[4-(Trifluormethoxy)phenyl]-1,4,5,6-tetrahydropyrazol[3,4-e]indazol-3-carboxylat;
34. Ethyl 1-Butyl-1,4,5,6-tetrahydropyrazol[3,4-e]indazol-3-carboxylat;
35. Ethyl 1-(2,5-Dimethylphenyl)-1,4,5,6-tetrahydropyrazol[3,4-e]indazol-3-carboxylat;
36. Ethyl 1-{4-[Amino(imino)methyl]phenyl}-1,4,5,6-tetrahydropyrazol[3,4-e]indazol-3-carboxylathydrochlorid;
37. Ethyl 1-[4-(1H-Imidazol-2-yl)phenyl]-1,4,5,6-tetrahydropyrazol[3,4-e]indazol-3-carboxylathydrochlorid;
38. Ethyl 1-Methyl-1,4,5,6-tetrahydropyrazol[3,4-e]indazol-3-carboxylat;
39. Ethyl 8-Anilino-1-methyl-1,4,5,6-tetrahydropyrazol[3,4-e]indazol-3-carboxylat;
40. Ethyl 8-Anilino-1-(2,2,2-trifluorethyl)-1,4,5,6-tetrahydropyrazol[3,4-e]indazol-3-carboxylat;
41. Ethyl 8-Anilino-2-{2-[(tert-butoxycarbonyl)amino]ethyl}-2,4,5,6-tetrahydropyrazol[3,4-e]indazol-3-carboxylat;
42. Ethyl 8-Amino-1,4,5,6-tetrahydropyrazol[3,4-e]indazol-3-carboxylat;
43. 1-(4-Methoxyphenyl)-1,4,5,6-tetrahydropyrazol[3,4-e]indazol-3-carboxamid;
44. 1-[4-(Aminosulfonyl)phenyl]-1,4,5,6-tetrahydropyrazol[3,4-e]indazol-3-carboxamid;
45. 1-{4-[(Methylamino)sulfonyl]phenyl)-1,4,5,6-tetrahydropyrazol[3,4-e]indazol-3-carboxamid;
46. 1-{4-[(Butylamino)sulfonyl]phenyl}-1,4,5,6-tetrahydropyrazol[3,4-e]indazol-3-carboxamid;
47. 1-{4-[(Dimethylamino)sulfonyl]phenyl}-1,4,5,6-tetrahydropyrazol[3,4-e]indazol-3-carboxamid;
48. 1-{4-[(Diprop-2-inylamino)sulfonyl]phenyl}-1,4,5,6-tetrahydropyrazol[3,4-e]indazol-3-carboxamid;
49. 1-[4-Anilinosulfonyl)phenyl]-1,4,5,6-tetrahydropyrazol[3,4-e]indazol-3-carboxamid;
50. 1-[4-(Methylsulfonyl)phenyl]-1,4,5,6-tetrahydropyrazol[3,4-e]indazol-3-carboxamid;
51. 1-[4-(Aminocarbonyl)phenyl]-1,4,5,6-tetrahydropyrazol[3,4-e]indazol-3-carboxamid;
52. 1-(4-{[(2-Hydroxypropyl)amino]sulfonyl}phenyl)-1,4,5,6-tetrahydropyrazol[3,4-e]indazol-3-carboxamid;
53. 1,4,5,6-tetrahydropyrazol[3,4-e]indazol-3-carboxamid;
54. 1-Phenyl-1,4,5,6-tetrahydropyrazol[3,4-e]indazol-3-carboxamid;
55. 1-(4-Fluorphenyl)-1,4,5,6-tetrahydropyrazol[3,4-e]indazol-3-carboxamid;
56. 1-(4-Bromphenyl)-1,4,5,6-tetrahydropyrazol[3,4-e]indazol-3-carboxamid;
57. 1-(4-Nitrophenyl)-1,4,5,6-tetrahydropyrazol[3,4-e]indazol-3-carboxamid;
58. 1-(4-Methylphenyl)-1,4,5,6-tetrahydropyrazol[3,4-e]indazol-3-carboxamid;
59. 1-(4-Chlorphenyl)-1,4,5,6-tetrahydropyrazol[3,4-e]indazol-3-carboxamid;
60. 1-(4-Cyanophenyl)-1,4,5,6-tetrahydropyrazol[3,4-e]indazol-3-carboxamid;
61. 1-[4-(Trifluormethyl)phenyl]-1,4,5,6-tetrahydropyrazol[3,4-e]indazol-3-carboxamid;
62. 1-Benzyl-1,4,5,6-tetrahydropyrazol[3,4-e]indazol-3-carboxamid;
63. 1-(3-Hydroxybenzyl)-1,4,5,6-tetrahydropyrazol[3,4-e]indazol-3-carboxamid;
64. 1-Pyridin-2-yl-1,4,5,6-tetrahydropyrazol[3,4-e]indazol-3-carboxamid;
65. 1-(3-Methylphenyl)-1,4,5,6-tetrahydropyrazol[3,4-e]indazol-3-carboxamid;
66. 1-(3-Chlorphenyl)-1,4,5,6-tetrahydropyrazol[3,4-e]indazol-3-carboxamid;
67. 1-(3-Fluorphenyl)-1,4,5,6-tetrahydropyrazol[3,4-e]indazol-3-carboxamid;
68. 1-(6-Chlorpyridazin-3-yl)-1,4,5,6-tetrahydropyrazol[3,4-e]indazol-3-carboxamid;
69. 4,4-Dimethyl-1-(4-methylphenyl)-1,4,5,6-tetrahydropyrazol[3,4-e]indazol-3-carboxamid;
70. 1-Pyridin-3-yl-1,4,5,6-tetrahydropyrazol[3,4-e]indazol-3-carboxamid;
71. 1-[4-(Acetylamino)phenyl]-1,4,5,6-tetrahydropyrazol[3,4-e]indazol-3-carboxamid;
72. 1-(4-Aminophenyl)-1,4,5,6-tetrahydropyrazol[3,4-e]indazol-3-carboxamid;

73. 1-{4-[(4-Methylpiperazin-1-yl)sulfonyl]phenyl}-1,4,5,6-tetrahydropyrazol[3,4-e]indazol-3-carboxamid;
74. 4-[3-(Aminocarbonyl)-5,6-dihydrpyrazol[3,4-e]indazol-1(4H)-yl)benzoesäure;
75. 1-(4-Morpholin-4-ylphenyl)-1,4,5,6-tetrahydropyrazol[3,4-e]indazol-3-carboxamid;
76. 1-[4-(Trifluormethoxy)phenyl]-1,4,5,6-tetrahydropyrazol[3,4-e]indazol-3-carboxamid;
77. 1-Butyl-1,4,5,6-tetrahydropyrazol[3,4-e]indazol-3-carboxamid;
78. 1-(2-Hydroxyethyl)-1,4,5,6-tetrahydropyrazol[3,4-e]indazol-3-carboxamid;
79. 1-(2,5-Dimethylphenyl)-1,4,5,6-tetrahydropyrazol[3,4-e]indazol-3-carboxamid;
80. 1-(2,2,2-Trifluorethyl)-1,4,5,6-tetrahydropyrazol[3,4-e]indazol-3-carboxamid;
81. 1-(2-Amino-2-oxoethyl)-1,4,5,6-tetrahydropyrazol[3,4-e]indazol-3-carboxamid;
82. 1-[4-(1H-Imidazol-2-yl)phenyl]-1,4,5,6-tetrahydropyrazol[3,4-e]indazol-3-carboxamid;
83. 4,4-Dimethyl-1-(2,2,2-trifluorethyl)-1,4,5,6-tetrahydropyrazol[3,4-e]indazol-3-carboxamid;
84. 1-Methyl-1,4,5,6-tetrahydropyrazol[3,4-e]indazol-3-carboxamid;
85. 2-(2-Hydroxyethyl)-2,4,5,6-tetrahydropyrazol[3,4-e]indazol-3-carboxamid;
86. 8-Anilino-1-methyl-1,4,5,6-tetrahydropyrazol[3,4-e]indazol-3-carboxamid;
87. 8-Anilino-1-(2,2,2-trifluorethyl)-1,4,5,6-tetrahydropyrazol[3,4-e]indazol-3-carboxamid;
88. 8-Amino-1,4,5,6-tetrahydropyrazol[3,4-e]indazol-3-carboxamid;
89. 1-[4-Methoxyphenyl]-1,6-dihydropyrazol[3,4-e]indazol-3-carboxamid;
90. 1-[4-(Aminosulfonyl)phenyl]-1,6-dihydropyrazol[3,4-e]indazol-3-carboxamid;
91. 1-{4-[(Methylamino)sulfonyl]phenyl}-1,6-dihydropyrazol[3,4-e]indazol-3-carboxamid;
92. 1-[4-[(Butylamino)sulfonyl]phenyl}-1,6-dihydropyrazol[3,4-e]indazol-3-carboxamid;
93. 1-[4-[(Dimethylamino)sulfonyl]phenyl}-1,6-dihydropyrazol[3,4-e]indazol-3-carboxamid;
94. 1-{4-[(Diprop-2-inylamino)sulfonyl]phenyl}-1,6-dihydropyrazol[3,4-e]indazol-3-carboxamid;
95. 1-[4-(Anilinosulfonyl)phenyl]-1,6-dihydropyrazol[3,4-e]indazol-3-carboxamid;
96. 1-(4-{[(2-Hydroxypropyl]amino]sulfonyl}phenyl)-1,6-dihydropyrazol[3,4-e]indazol-3-carboxamid;
97. 1-[4-(Methylsulfonyl)phenyl]-1,6-dihydropyrazol[3,4-e]indazol-3-carboxamid;
98. 1,6-dihydropyrazol[3,4-e]indazol-3-carboxamid;
99. 1-Phenyl-1,6-dihydropyrazol[3,4-e]indazol-3-carboxamid;
100. 1-(4-fluorphenyl)-1,6-dihydropyrazol[3,4-e]indazol-3-carboxamid;
101. 1-(4-Methylphenyl)-1,6-dihydropyrazol[3,4-e]indazol-3-carboxamid;
102. 1-(4-Cyanophenyl)-1,6-dihydropyrazol[3,4-e]indazol-3-carboxamid;
103. 1-[4-(Trifluormethyl)phenyl]-1,6-dihydropyrazol[3,4-e]indazol-3-carboxamid;
104. 1-(4-Chlorphenyl)-1,6-dihydropyrazol[3,4-e]indazol-3-carboxamid;
105. 1-(4-Bromphenyl)-1,6-dihydropyrazol[3,4-e]indazol-3-carboxamid;
106. 1-(4-Nitrophenyl)-1,6-dihydropyrazol[3,4-e]indazol-3-carboxamid;
107. 1-Benzyl-1,6-dihydropyrazol[3,4-e]indazol-3-carboxamid;
108. 1-(3-Hydroxybenzyl)-1,6-dihydropyrazol[3,4-e]indazol-3-carboxamid;
109. 1-Pyridin-2-yl-1,6-dihydropyrazol[3,4-e]indazol-3-carboxamid;
110. 1-(3-Chlorphenyl)-1,6-dihydropyrazol[3,4-e]indazol-3-carboxamid;
111. 1-(3-Methylphenyl)-1,6-dihydropyrazol[3,4-e]indazol-3-carboxamid;
112. 1-(3-Fluorphenyl)-1,6-dihydropyrazol[3,4-e]indazol-3-carboxamid;
113. 1-(6-Chlorpyridazin-3-yl)-1,6-dihydropyrazol[3,4-e]indazol-3-carboxamid;
114. 1-(4-Methoxyphenyl)-1,6-dihydropyrazol[3,4-e]indazol-3-carbonsäure;
115. Ethyl 1-Phenyl-1,6-dihydropyrazol[3,4-e]indazol-3-carboxylat;
116. Ethyl 1-(4-Methoxyphenyl)-1,6-dihydropyrazol[3,4-e]indazol-3-carboxamid;
117. N-Methyl-1-[4-(aminosulfonyl)phenyl]-1,6-dihydropyrazol[3,4-e]indazol-3-carboxamid;
118. N-Methyl-1-{4-[(butylamino)sulfonyl]phenyl}-1,6-dihydropyrazol[3,4-e]indazol-3-carboxamid;
119. N-Methyl-1-[4-[(dimethylamino)sulfonyl]phenyl)-1,6-dihydropyrazol[3,4-e]indazol-3-carboxamid;
120. N-Methyl-1-[4-(methylsulfonyl)phenyl]-1,6-dihydropyrazol[3,4-e]indazol-3-carboxamid;
121. N-(Allyloxy)-1-[4-[(butylamino)sulfonyl]phenyl}-1,6-dihydropyrazol[3,4-e]indazol-3-carboxamid;
122. 7,8,9,10-tetrahydro[1,4]diazepino[1,2-b]pyrazol[3,4-g]indazol-6(3H)-on;
123. 1-Pyridin-3-yl-1,6-dihydropyrazol[3,4-e]indazol-3-carboxamid;
124. 1-[4-(acetylamino)phenyl]-1,6-dihydropyrazol[3,4-e]indazol-3-carboxamid;
125. 4-[3-(Aminocarbonyl)pyrazol[3,4-e]indazol-1(6H)-yl]Benzoesäure;
126. 1-{4-[(4-Methylpiperazin-1-yl)sulfonyl]phenyl}-1,6-dihydropyrazol[3,4-e]indazol-3-carboxamid;
127. 1-[4-(Trifluormethoxy)phenyl]-1,6-dihydropyrazol[3,4-e]indazol-3-carboxamid;
128. 4-[3-(Ethoxycarbonyl)pyrazol[3,4-e]indazol-1(6H)-yl]Benzoesäure;
129. 1-(4-Morpholin-4-ylphenyl)-1,6-dihydropyrazol[3,4-e]indazol-3-carboxamid;
130. 1-(2-Hydroxyethyl)-1,6-dihydropyrazol[3,4-e]indazol-3-carboxamid;

131. 1-(2,5-Dimethylphenyl)-1,6-dihydropyrazol[3,4-e]indazol-3-carboxamid,
132. 1-(2-Aminoethyl)-1,6-dihydropyrazol[3,4-e]indazol-3-carboxamidhydrochlorid;
133. 1-(2,2,2-Trifluorethyl)-1,6-dihydropyrazol[3,4-e]indazol-3-carboxamid;
134. 1-[4-(1H-Imidazol-2-yl)phenyl]-1,6-dihydropyrazol[3,4-e] indazol-3-carboxamid;
135. 1-Methyl-1,6-dihydropyrazol[3,4-e]indazol-3-carboxamid;
136. 8,9-Dihydro-3H-pyrazino[1,2-b]pyrazol[3,4-g]indazol-6(7H)-on;
137. 2-(2-Aminoethyl)-2,6-dihydropyrazol[3,4-e]indazol-3-carboxamidhydrochlorid;
138. 2-(2-Hydroxyethyl)-2,6-dihydropyrazol[3,4-e]indazol-3-carboxamid;
139. 2-Methyl-2,6-dihydropyrazol[3,4-e]indazol-3-carboxamid;
140. 1-Anilino-8,9-dihydro-3H-pyrazino[1,2-b]pyrazol[3,4-g]indazol-6(7H)-on;
141. 1-(4-Methoxy-phenyl)-1,6-dihydropyrazol[3,4-e]indazol-3-amin;
142. 1-[1-(4-Methylphenyl)-1,6-dihydropyrazol[3,4-e]indazol-3-yl]ethanon;
143. 1-(4-Methoxyphenyl)-1,6-dihydropyrazol[3,4-e]indazol-3-carbonitril;
144. 1-(4-Methoxyphenyl)-1,6-dihydropyrazol[3,4-e]indazol-3-carbohydrazid;
145. 1-(4-Methoxyphenyl)-1,4,5,6-tetrahydropyrazol[3,4-e]indazol-3-carbohydrazid;
146. N'-Hydroxy-1-(4-methoxyphenyl)-1,6-dihydropyrazolo[3,4-e]indazol-3-carboximidamid;
147. 1-(4-Methoxyphenyl)-1,6-dihydropyrazolo[3,4-e] indazol-3-carbonsäure;
148. 1-(4-Bromphenyl)-1,4,5,6-tetrahydropyrazolo[3,4-e]indazol-3-carbonsäure;
149. 1-{4-[(Butylamino)sulfonyl]phenyl}-1,4,5,6-tetrahydropyrazol[3,4-e]indazol-3-carbonsäure;
150. 4,4-Dimethyl-1-(4-methylphenyl)-1,4,5,6-tetrahydropyrazol[3,4-e]indazol-3-carbonsäure;
151. N-Hydroxy-1-(4-methoxyphenyl)-1,6-dihydropyrazo[3,4-e]indazol-3-carboxamid;
152. N-(Allyloxy)-1-{4-[(butylamino)sulfonyl]phenyl}-1,4,5,6-tetrahydropyrazol[3,4-e]indazol-3-carboxamid;
153. N-{Allyloxy)-1-(4-methoxyphenyl}-1,4,5,6-tetrahydropyrazol[3,4-e]indazol-3-carboxamid;
154. N-Methyl-1-[4-(methylsulfony-1)phenyl]-1,4,5,6-tetrahydropyrazol[3,4-e]indazol-3-carboxamid;
155. 1-{4-(Aminosulfonyl)phenyl]-N-methyl-1,4,5,6-tetrahydropyrazol[3,4-e]indazol-3-carboxamid;
156. 1-{4-[(Butylamino)sulfonyl]phenyl}-N-methyl-1,4,5,6-tetrahydropyrazol[3,4-e]indazol-3-carboxamid;
157. 1-{4-[(Dimethylamino)sulfonyl]phenyl}-N-methyl-1,4,5,6-tetrahydropyrazol[3,4-e]indazol-3-carboxamid;
158. Ethyl 2-(3-Aminopropyl)-2,4,5,6-tetrahydropyrazol[3,4-e]indazol-3-carboxylathydrochlorid;
159. 4,5,7,8,9,10-Hexahydro[1,4]diazepino[1,2-b]pyrazol[3,4-g]indazol-6(3H)-on;
160. 5,5-Dimethyl-4,5,7,8,9,10-hexahydro[1,4]diazepino[1,2-b]pyrazol[3,4-g]indazol-6(3H)-on;
161. 5,5-Dimethyl-4,5,8,9-tetrahydro-3H-pyrazino[1,2-b]pyrazol[3,4-g]indazol-6(7H)-on;
162. 4,5,8,9-tetrahydro-3H-pyrazino[1,2-b]pyrazol[3,4-g] indazol-6(7H)-on;
163. 1-Anilino-4,5,8,9-tetrahydro-3H-pyrazino[1,2-b]pyrazol[3,4-g]indazol-6(7H)-on.

**13.** Ein Verfahren zur Herstellung einer Verbindung der Formel (1c) wie in Anspruch 9 definiert

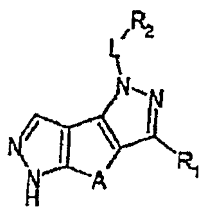

(1c)

worin L und $R_2$ wie in Anspruch 16 sind, $R_1$ ist eine Gruppe -COOEt oder $-CONH_2$, und A ist gewählt aus der Gruppe bestehend aus $-CH_2-$, $-CH_2-CH_2-$, -CH=CH- und $-CH_2-C(CH_3)_2-$, wobei das Verfahren folgendes umfasst:

a) Reagieren der Verbindung (10) mit Hydrazin-Dihydrochlorid, um die Verbindung (11) zu erhalten

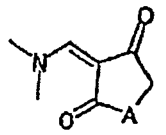

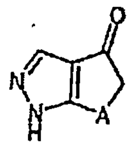

(10) (11)

worin A wie oben definiert, aber nicht -CH=CH- ist;

b) Reagieren der Verbindung (11) mit Tritylchlorid, um die Verbindung (12) zu erhalten

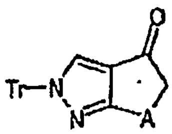

(12)

worin Tr für Trityl steht, und Kondensieren derselben mit Oxalylchlorid, um die Verbindung (13) zu erhalten

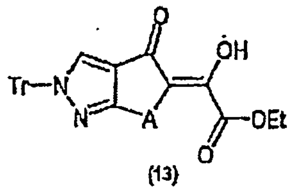

(13)

c) Reagieren der Verbindung (13) mit einem substituierten Hydrazin (8)

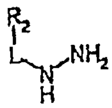

(8)

worin L und $R_2$ wie in Anspruch 16 definiert sind; um eine Verbindung der Formel (1c) zu erhalten, worin $R_1$ eine Gruppe -COOEt ist, und A ist wie oben definiert, außer -CH=CH-; und, wahlweise

d) Reagieren dieser letzteren mit Ammoniumhydroxid, um das entsprechende Derivat der Formel (1c) zu erhalten, worin $R_1$ -$CONH_2$ ist; und, wahlweise

e) Reagieren der Verbindung der Formel (1c), worin A -$CH_2$-$CH_2$- ist, wie in Schritten c) oder d) erhalten, mit einem geeigneten Oxidationsmittel, um das entsprechende Derivat der Formel (1c) zu erhalten, worin A -CH=CH- ist.

**14.** Das Verfahren von Anspruch 13, worin, in Schritt e) das Oxidationsmittel 2,3-Dichlor-5,6-dicyano-1,4-benzochinon ist.

**15.** Eine pharmazeutische Zusammensetzung, die eine wirksame Menge einer Verbindung der Formel (1) wie in Anspruch 9 definiert, und mindestens ein pharmazeutisch verträgliches Bindemittel, Träger oder Verdünnungsmittel umfasst.

**16.** Eine pharmazeutische Zusammensetzung gemäß Anspruch 15, die weiter einen oder mehrere chemotherapeutische Wirkstoffe umfasst, als ein Kombinationspräparat für die gleichzeitige, separate oder aufeinanderfolgende Verwendung in der Anti-Krebstherapie.

**17.** Ein Produkt oder Kit, das eine Verbindung von Anspruch 9 oder eine pharmazeutische Zusammensetzung davon, wie in Anspruch 15 definiert, und einen oder mehrere chemotherapeutische Wirkstoffe umfasst, als ein Kombinationspräparat für die gleichzeitige, separate oder aufeinanderfolgende Verwendung in der Anti-Krebstherapie.

**18.** Verwendung einer Verbindung der Formel (1c), (1e), (1f) oder (1g) oder eines pharmazeutisch verträglichen Salzes davon, wie in Anspruch 9 definiert, in der Herstellung eines Medikaments zur Behandlung von Krankheiten, die ausgelöst werden durch und/oder in Zusammenhang stehen mit einer veränderten Proteinkinase-Aktivität.

**19.** Verwendung gemäß Anspruch 1 zur Behandlung von Tumoren.

## Revendications

**1.** Utilisation, pour la production d'un médicament destiné au traitement de maladies provoquées par, et/ou associées à, une activité de protéine-kinase modifiée, par administration à un mammifère ayant besoin d'un tel traitement, d'une quantité efficace d'un composé de formule (Ic), (Ie), (If) ou (Ig)

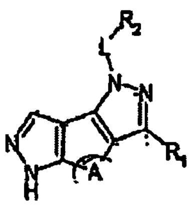
(Ic)

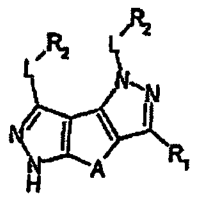
(Ie)

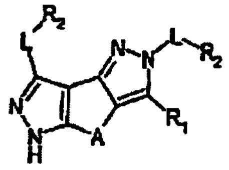
(If)

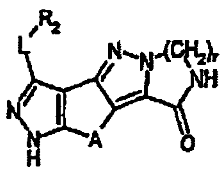
(Ig)

dans laquelle :

$R_1$ dans la formule (Ic) est choisi dans le groupe consistant en des groupes hydrido, alkyle inférieur, alkyle inférieur perfluoré, hétérocyclyle, CN, $CO_2R'$, $COCF_3$, COR', CONR'R", NR'R", C(=NR')NR'R", $CONHNH_2$, CONHOR', NHCOR', $CH_2NH_2$ et $CH_2NHCOR'$ ; ou
$R_1$ dans les formules (Ie) et (If) est choisi dans le groupe consistant en des groupes CN, $CO_2R'$, COR', CONR'R", NR'R", C(=NOH)NR'R", $CONHNH_2$, CONHOR' ;
R' et R" dans la formule (Ic) sont choisis, chacun indépendamment, dans le groupe consistant en des groupes hydrido, hydroxy, alkyle, hydroxyalkyle, alcényle, alcynyle, aryle, arylalkyle, hétérocyclyle ou hétérocyclylalkyle ; ou
R' et R" dans les formules (Ie) et (If) sont choisis, chacun indépendamment, dans le groupe consistant en des groupes hydrido et alkyle inférieur ;
A dans la formule (Ic) est choisi dans le groupe consistant en des groupes $-CH_2-$ ; $-CH_2-CH_2-$ ; -CH=CH- ; $(CH_2)-C(CH_3)_2$ ; ou
A dans les formules (Ie), (If) et (Ig) est choisi dans le groupe consistant en des groupes $-CH_2-CH_2-$ ; -CH=CH- ;

$(CH_2)-C(CH_3)_2$ ;
les symboles L représentent, chacun indépendamment, une liaison simple, un groupe alkylidène ou un groupe divalent choisi entre les groupes NH, NHCO, CONH, NHCONH, $SO_2NH$ et $NHSO_2$ ;
les groupes $R_2$ représentent, chacun indépendamment, un groupe hydrido, un groupe alkyle, un noyau penta- à dodécagonal mono- ou bicyclique comprenant 0 à 3 hétéroatomes choisis entre S, O et N, facultativement substitué avec un ou plusieurs groupes $-(CH_2)_q-R_3$ ; ou bien $R_2$ représente un groupe de formule

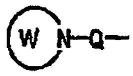

W représente un noyau tri- à heptagonal comprenant un hétéroatome de N lié directement à Q et 0 à 2 hétéroatomes supplémentaires choisis dans le groupe consistant en S, SO, $SO_2$, O, N et NR', dans lequel R' répond à la définition précitée ;
Q représente un groupe divalent choisi entre des groupes CO, $SO_2$ et $(CH_2)_n$ ;
les groupes $R_3$ sont choisis, chacun indépendamment, dans le groupe consistant en des groupes alkyle, halogéno, $CF_3$, $OCF_3$, $NO_2$, CN, C(=NR')NR'R", OR', SR', OCOR', OCONR'R", $COCF_3$, COR', $CO_2R'$, CONR'R", $SO_2R'$, $SO_2NR'R"$, NR'R", NR'COR', NR'COOR', NR'CONR'R", $NR'SO_2R'$, $NR'SO_2NR'R"$, dans lesquels R' et R" répondent aux définitions précitées ;
les indices n sont égaux, chacun indépendamment, à 0, 1 ou 2 ;
les indices q sont égaux, chacun indépendamment, à 0 ou à un nombre entier de 1 à 3 ;
r représente un nombre entier de 1 à 3 ;

ou de ses isomères, formes tautomères, supports, précurseurs médicamenteux et sels pharmaceutiquement acceptables.

**2.** Utilisation suivant la revendication 1, dans laquelle la maladie provoquée par, et/ou associée à, une activité de protéine-kinase modifiée est un trouble de la prolifération cellulaire choisi dans le groupe consistant en un cancer, la maladie d'Alzheimer, des infections virales, des maladies auto-immunes et des troubles neurodégénératifs.

**3.** Utilisation suivant la revendication 2, dans laquelle le cancer est choisi entre un carcinome, un carcinome à cellules squameuses, des tumeurs hématopoïétiques de la lignée lymphoïde ou myéloïde, des tumeurs d'origine mésenchymateuse, des tumeurs du système nerveux central et du système nerveux périphérique, un mélanome, un séminome, un tératocarcinome, un ostéosarcome, le xeroderma pigmentosum, le kératoxanthome, le cancer folliculaire de la thyroïde et le sarcome de Kaposi.

**4.** Utilisation suivant la revendication 1, dans laquelle le trouble de la prolifération cellulaire est choisi entre l'hyperplasie prostatique bénigne, l'adénomatose familiale, la polypose, la neurofibromatose, le psoriasis, la prolifération des cellules du tissu lisse vasculaire associée à l'athérosclérose, la fibrose pulmonaire, l'arthrite, la glomérulonéphrite et la sténose et la resténose postchirurgicales.

**5.** Utilisation suivant la revendication 1, qui provoque une inhibition de l'angiogenèse et de la métastase tumorales.

**6.** Utilisation suivant la revendication 1, dans laquelle ladite administration est associée à la mise en oeuvre chez le mammifère en ayant besoin d'un schéma de radiothérapie ou de chimiothérapie en association avec au moins un agent cytostatique ou cytotoxique.

**7.** Utilisation suivant la revendication 1, dans laquelle le mammifère en ayant besoin est un être humain.

**8.** Utilisation d'un composé de formule (Ic), (Ie), (If) ou (Ig) telle que définie dans la revendication 1, pour la production d'un médicament destiné à inhiber une activité de protéine-kinase, dans laquelle ledit composé est destiné à être mis en contact avec ladite kinase en une quantité efficace.

**9.** Composé représenté par la formule (Ic), (Ie), (If) ou (Ig)

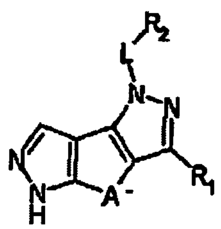

(Ic)

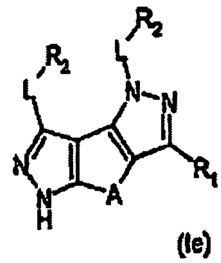

(Ie)

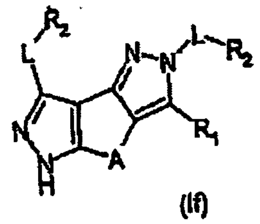

(If)

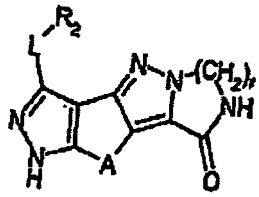

(Ig)

dans laquelle :

$R_1$ dans la formule (Ic) est choisi dans le groupe consistant en des groupes alkyle inférieur perfluoré, hétérocyclyle, CN, $CO_2R'$ $COCF_3$, COR', CONR'R", NR'R", C(=NR')NR'R", $CONHNH_2$, CONHOR', NHCOR', $CH_2NH_2$ et $CH_2NHCOR'$ ; ou

$R_1$ dans les formules (Ie) et (If) est choisi dans le groupe consistant en des groupes CN, $CO_2R'$, COR', CONR'R", NR'R", C(=NOH)NR'R", $CONHNH_2$, CONHOR' ;

R' et R" dans la formule (Ic) sont choisis, chacun indépendamment, dans le groupe consistant en des groupes hydrido, hydroxy, alkyle, hydroxyalkyle, alcényle, alcynyle, aryle, arylalkyle, hétérocyclyle ou hétérocyclylalkyle ; ou

R' et R" dans les formules (Ie) et (If) sont choisis, chacun indépendamment, dans le groupe consistant en des groupes hydrido et alkyle inférieur ;

A dans la formule (Ic) est choisi dans le groupe consistant en des groupes $-CH_2-$ ; $-CH_2-CH_2-$ ; -CH=CH- ; $(CH_2)-C(CH_3)_2$ ; ou

A dans les formules (Ie), (If) et (Ig) est choisi dans le groupe consistant en des groupes $-CH_2-CH_2-$ ; -CH=CH- ; $(CH_2)-C(CH_3)_2$ ;

les symboles L représentent, chacun indépendamment, une liaison simple, un groupe alkylidène ou un groupe divalent choisi entre les groupes NH, NHCO, CONH, NHCONH, $SO_2NH$ et $NHSO_2$;

les groupes $R_2$ représentent, chacun indépendamment, un groupe hydrido, un groupe alkyle, un noyau penta- à dodécagonal mono- ou bicyclique comprenant 0 à 3 hétéroatomes choisis entre S, O et N, facultativement substitué avec un ou plusieurs groupes $-(CH_2)_q-R_3$; ou bien $R_2$ représente un groupe de formule

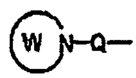

W représente un noyau tri- à heptagonal comprenant un hétéroatome de N lié directement à Q et 0 à 2 hétéroatomes supplémentaires choisis dans le groupe consistant en S, SO, $SO_2$, O, N et NR', dans lequel R' répond à la définition précitée ;

Q représente un groupe divalent choisi entre des groupes CO, $SO_2$ et $(CH_2)_n$ ;

les groupes $R_3$ sont choisis, chacun indépendamment, dans le groupe consistant en des groupes alkyle, halogéno, $CF_3$, $OCF_3$, $NO_2$, CN, C(=NR')NR'R", OR', SR', OCOR', OCONR'R", $COCF_3$, COR', $CO_2R'$, CONR'R", $SO_2R'$, $SO_2NR'R"$, NR'R", NR'COR', NR'COOR', NR'CONR'R", $NR'SO_2R'$, $NR'SO_2NR'R"$, dans lesquels R' répond à la définition précitée ;

les indices n sont égaux, chacun indépendamment, à 0, 1 ou 2 ;

les indices q sont égaux, chacun indépendamment, à 0 ou à un nombre entier de 1 à 3 ;

r représente un nombre entier de 1 à 3 ;

ou ses isomères, formes tautomères, supports, précurseurs médicamenteux et sels pharmaceutiquement acceptables.

**10.** Composé de formule (Ic) suivant la revendication 9, dans lequel chaque L est choisi indépendamment entre un groupe méthylène et une liaison simple, et chaque groupe $R_2$ est choisi indépendamment entre un groupe hydrido, un groupe phényle et un hétérocycle aromatique penta- ou hexagonal comprenant 1 ou 2 hétéroatomes choisis entre N, O et S.

**11.** Composé de formule (Ic) suivant la revendication 10, dans lequel $R_2$, qui est en outre facultativement substitué comme défini dans la revendication 9, est choisi dans le groupe consistant en des groupes hydrido, phényle, pyridyle, pyridazinyle et pyrimidinyle.

**12.** Composé de formule (Ic), (Ie), (If) ou (Ig) telle que définie dans la revendication 9, éventuellement sous forme d'un sel pharmaceutiquement acceptable, choisi dans le groupe consistant en :

1. 1-(4-méthoxyphényl)-1,4,5,6-tétrahydropyrazolo-[3,4-e]indazole-3-carboxylate d'éthyle ;
2. 1-[4-(aminosulfonyl)phényl]-1,4,5,6-tétrahydropyrazolo-[3,4-e]indazole-3-carboxylate d'éthyle ;
3. 1-{4-[(méthylamino)sulfonyl]phényl}-1,4,5,6-tétrahydropyrazolo[3,4-e]indazole-3-carboxylate d'éthyle ;
4. 1-{4-[(butylamino)sulfonyl]phényl}-1,4,5,6-tétrahydropyrazolo[3,4-e]indazole-3-carboxylate d'éthyle ;
5. 1-{4-[(diméthylamino)sulfonyl]phényl}-1,4,5,6-tétrahydropyrazolo[3,4-e]indazole-3-carboxylate d'éthyle ;
6. 1-{4-[(diprop-2-ynylamino)sulfonyl]phényl}-1,4,5,6-tétrahydropyrazolo[3,4-e]indazole-3-carboxylate d'éthyle ;
7. 1-[4-(anilinosulfonyl)phényl]-1,4,5,6-tétrahydropyrazolo[3,4-e]indazole-3-carboxylate d'éthyle ;
8. 1-[4-(méthylsulfonyl)phényl]-1,4,5,6-tétrahydropyrazolo-[3,4-e]indazole-3-carboxylate d'éthyle ;
9. 1-(4-{[(2-hydroxypropyl)amino]sulfonyl}phényl)-1,4,5,6-tétrahydropyrazolo[3,4-e]indazole-3-carboxylate d'éthyle ;
10. 1-[4-(aminocarbonyl)phényl]-1,4,5,6-tétrahydropyrazolo-[3,4-e]indazole-3-carboxylate d'éthyle ;
11. 1,4,5,6-tétrahydropyrazolo[3,4-e]indazole-3-carboxylate d'éthyle ;
12. 1-phényl-1,4,5,6-tétrahydropyrazolo[3,4-e]indazole-3-carboxylate d'éthyle ;
13. 1-(4-fluorophényl)-1,4,5,6-tétrahydropyrazolo[3,4-e]-indazole-3-carboxylate d'éthyle ;
14. 1-(4-bromophényl)-1,4,5,6-tétrahydropyrazolo[3,4-e]-indazole-3-carboxylate d'éthyle ;
15. 1-(4-méthylphényl)-1,4,5,6-tétrahydropyrazolo-[3,4-e]indazole-3-carboxylate d'éthyle ;
16. 1-(4-chlorophényl)-1,4,5,6-tétrahydropyrazolo-[3,4-e]indazole-3-carboxylate d'éthyle ;
17. 1-(4-cyanophényl)-1,4,5,6-tétrahydropyrazolo[3,4-e]-indazole-3-carboxylate d'éthyle ;
18. 1-(4-nitrophényl)-1,4,5,6-tétrahydropyrazolo[3,4-e]-indazole-3-carboxylate d'éthyle ;
19. 1-[4-(trifluorométhyl)phényl]-1,4,5,6-tétrahydropyrazolo[3,4-e]indazole-3-carboxylate d'éthyle ;
20. 1-benzyl-1,4,5,6-tétrahydropyrazolo[3,4-e]-indazole-3-carboxylate d'éthyle ;
21. 1-(3-hydroxybenzyl)-1,4,5,6-tétrahydropyrazolo-[3,4-e]indazole-3-carboxylate d'éthyle;
22. 1-pyridine-2-yl-1,4,5,6-tétrahydropyrazolo[3,4-e]-indazole-3-carboxylate d'éthyle ;
23. 1-(6-chloropyridazine-3-yl)-1,4,5,6-tétrahydropyrazolo[3,4-e]indazole-3-carboxylate d'éthyle ;
24. 1-[4-(trifluorométhyl) pyrimidine-2-yl]-1,4,5,6-tétrahydropyrazolo[3,4-e]indazole-3-carboxylate d'éthyle ;
25. 1-(3-méthylphényl)-1,4,5,6-tétrahydropyrazolo[3,4-e]-indazole-3-carboxylate d'éthyle ;
26. 1-(3-chlorophényl)-1,4,5,6-tétrahydropyrazolo[3,4-e]-indazole-3-carboxylate d'éthyle ;
27. 1-(3-fluorophényl)-1,4,5,6-tétrahydropyrazolo-[3,4-e]indazole-3-carboxylate d'éthyle ;
28. 4,4-diméthyl-1-(4-méthylphényl)-1,4,5,6-tétrahydropyrazolo[3,4-e]indazole-3-carboxylate d'éthyle ;
29. 1-pyridine-3-yl-1,4,5,6-tétrahydropyrazolo[3,4-e]-indazole-3-carboxylate d'éthyle ;
30. 1-[4-(acétylamino)phényl]-1,4,5,6-tétrahydropyrazolo[3,4-e]indazole-3-carboxylate d'éthyle ;
31. 1-{4-[(4-méthylpipérazine-1-yl)sulfonyl]phényl}-1,4,5,6-tétrahydropyrazolo[3,4-e]indazole-3-carboxylate d'éthyle ;
32. Acide 4-[3-(éthoxycarbonyl)-5,6-dihydropyrazolo-[3,4-e]indazole-1(4H)-yl]benzoïque ;
33. 1-[4-(trifluorométhoxy)phényl]-1,4,5,6-tétrahydropyrazolo[3,4-e]indazole-3-carboxylate d'éthyle ;
34. 1-butyl-1,4,5,6-tétrahydropyrazolo[3,4-e]indazole-3-carboxylate d'éthyle ;
35. 1-(2,5-diméthylphényl)-1,4,5,6-tétrahydropyrazolo-[3,4-e]indazole-3-carboxylate d'éthyle ;
36. Chlorhydrate de 1-{4-[amino(imino)méthyl]phényl}-1,4,5,6-tétrahydropyrazolo[3,4-e]indazole-3-carboxylate d'éthyle ;
37. Chlorhydrate de 1-[4-(1H-imidazole-2-yl)phényl]-1,4,5,6-tétrahydropyrazolo[3,4-e]indazole-3-carboxylate d'éthyle ;
38. 1-méthyl-1,4,5,6-tétrahydropyrazolo[3,4-e]indazole-3-carboxylate d'éthyle ;

39. 8-anilino-1-méthyl-1,4,5,6-tétrahydropyrazolo-[3,4-e]indazole-3-carboxylate d'éthyle ;
40. 8-anilino-1-(2,2,2-trifluorométhyl)-1,4,5,6-tétrahydropyrazolo[3,4-e]indazole-3-carboxylate d'éthyle ;
41. 8-anilino-2-{2-[(tertiobutoxycarbonyl)amino]éthyl}-2,4,5,6-tétrahydropyrazolo[3,4-e]indazole-3-carboxylate d'éthyle ;
42. 8-amino-1,4,5,6-tétrahydropyrazolo[3,4-e]indazole-3-carboxylate d'éthyle ;
43. 1-(4-méthoxyphényl)-1,4,5,6-tétrahydropyrazolo-[3,4-e]indazole-3-carboxamide ;
44. 1-[4-(aminosulfonyl) phényl]-1,4,5,6-tétrahydropyrazolo[3,4-e]indazole-3-carboxamide ;
45. 1-{4-[(méthylamino)sulfonyl]phényl}-1,4,5,6-tétrahydropyrazolo[3,4-e]indazole-3-carboxamide ;
46. 1-{4-[(butylamino)sulfonyl]phényl}-1,4,5,6-tétrahydropyrazolo[3,4-e]indazole-3-carboxamide ;
47. 1-{4-[(diméthylamino)sulfonyl]phényl}-1,4,5,6-tétrahydropyrazolo[3,4-e]indazole-3-carboxamide ;
48. 1-{4-[(diprop-2-ylamino)sulfonyl]phényl}-1,4,5,6-tétrahydropyrazolo[3,4-e]indazole-3-carboxamide ;
49. 1-[4-(anilinosulfonyl)phényl]-1,4,5,6-tétrahydropyrazolo[3,4-e]indazole-3-carboxamide ;
50. 1-[4-(méthylsulfonyl)phényl]-1,4,5,6-tétrahydropyrazolo[3,4-e]indazole-3-carboxamide ;
51. 1-[4-(aminocarbonyl)phényl]-1,4,5,6-tétrahydropyrazolo[3,4-e]indazole-3-carboxamide ;
52. 1-(4-{[(2-hydroxypropyl)amino]sulfonyl}phényl)-1,4,5,6-tétrahydropyrazolo[3,4-e]indazole-3-carboxamide ;
53. 1,4,5,6-tétrahydropyrazolo[3,4-e]indazole-3-carboxamide ;
54. 1-phényl-1,4,5,6-tétrahydropyrazolo[3,4-e]-indazole-3-carboxamide ;
55. 1-(4-fluorophényl)-1,4,5,6-tétrahydropyrazolo-[3,4-e]indazole-3-carboxamide ;
56. 1-(4-bromophényl)-1,4,5,6-tétrahydropyrazolo-[3,4-e]indazole-3-carboxamide ;
57. 1-(4-nitrophényl)-1,4,5,6-tétrahydropyrazolo-[3,4-e]indazole-3-carboxamide ;
58. 1-(4-méthylphényl)-1,4,5,6-tétrahydropyrazolo-[3,4-e]indazole-3-carboxamide ;
59. 1-(4-chlorophényl)-1,4,5,6-tétrahydropyrazolo-[3,4-e]indazole-3-carboxamide ;
60. 1-(4-cyanophényl)-1,4,5,6-tétrahydropyrazolo[3,4-e]-indazole-3-carboxamide ;
61. 1-[4-(trifluorométhyl)phényl]-1,4,5,6-tétrahydropyrazolo[3,4-e]indazole-3-carboxamide ;
62. 1-benzyl-1,4,5,6-tétrahydropyrazolo[3,4-e]indazole-3-carboxamide ;
63. 1-(3-hydroxybenzyl)-1,4,5,6-tétrahydropyrazolo[3,4-e]-indazole-3-carboxamide ;
64. 1-pyridine-2-yl-1,4,5,6-tétrahydropyrazolo-[3,4-e]indazole-3-carboxamide ;
65. 1-(3-méthylphényl)-1,4,5,6-tétrahydropyrazolo[3,4-e]-indazole-3-carboxamide ;
66. 1-(3-chlorophényl)-1,4,5,6-tétrahydropyrazolo[3,4-e]-indazole-3-carboxamide ;
67. 1-(3-fluorophényl)-1,4,5,6-tétrahydropyrazolo[3,4-e]-indazole-3-carboxamide ;
68. 1-(6-chloropyridazine-3-yl)-1,4,5,6-tétrahydropyrazolo[3,4-e]indazole-3-carboxamide ;
69. 4,4-diméthyl-1-(4-méthylphényl)-1,4,5,6-tétrahydropyrazolo[3,4-e]indazole-3-carboxamide ;
70. 1-pyridine-3-yl-1,4,5,6-tétrahydropyrazolo[3,4-e]-indazole-3-carboxamide ;
71. 1-[4-(acétylamino)phényl]-1,4,5,6-tétrahydropyrazolo[3,4-e]indazole-3-carboxamide ;
72. 1-(4-aminophényl)-1,4,5,6-tétrahydropyrazolo[3,4-e]-indazole-3-carboxamide ;
73. 1-{4-[(4-méthylpipérazine-1-yl)sulfonyl]phényl}-1,4,5,6-tétrahydropyrazolo[3,4-e]indazole-3-carboxamide ;
74. Acide 4-[3-(aminocarbonyl)-5,6-dihydropyrazolo-[3,4-e]indazole-1(4H)-yl]benzoïque ;
75. 1-(4-morpholine-4-ylphényl)-1,4,5,6-tétrahydropyrazolo[3,4-e]indazole-3-carboxamide ;
76. 1-[4-(trifluorométhoxy)phényl]-1,4,5,6-tétrahydropyrazolo[3,4-e]indazole-3-carboxamide ;
77. 1-butyl-1,4,5,6-tétrahydropyrazolo[3,4-e]indazole-3-carboxamide ;
78. 1-(2-hydroxyéthyl)-1,4,5,6-tétrahydropyrazolo[3,4-e]-indazole-3-carboxamide ;
79. 1-(2,5-diméthylphényl)-1,4,5,6-tétrahydropyrazolo-[3,4-e] indazole-3-carboxamide;
80. 1-(2,2,2-trifluoréthyl)-1,4,5,6-tétrahydropyrazolo-[3,4-e]indazole-3-carboxamide;
81. 1-(2-amino-2-oxoéthyl)-1,4,5,6-tétrahydropyrazolo-[3,4-e]indazole-3-carboxamide ;
82. 1-[4-(1H-imidazole-2-yl)phényl]-1,4,5,6-tétrahydropyrazolo[3,4-e]indazole-3-carboxamide ;
83. 4,4-diméthyl-1-(2,2,2-trifluoréthyl)-1,4,5,6-tétrahydropyrazolo[3,4-e]indazole-3-carboxamide ;
84. 1-méthyl-1,4,5,6-tétrahydropyrazolo[3,4-e]indazole-3-carboxamide ;
85. 2-(2-hydroxyéthyl)-2,4,5,6-tétrahydropyrazolo[3,4-e]-indazole-3-carboxamide ;
86. 8-anilino-1-méthyl-1,4,5,6-tétrahydropyrazolo[3,4-e]-indazole-3-carboxamide ;
87. 8-anilino-1-(2,2,2-trifluoréthyl)-1,4,5,6-tétrahydropyrazolo[3,4-e]indazole-3-carboxamide ;
88. 8-amido-1,4,5,6-tétrahydropyrazolo[3,4-e]indazole-3-carboxamide ;
89. 1-[4-méthoxyphényl]-1,6-dihydropyrazolo[3,4-e]-indazole-3-carboxamide ;
90. 1-[4-(aminosulfonyl)phényl]-1,6-dihydropyrazolo-[3,4-e]indazole-3-carboxamide ;
91. 1-{4-[(méthylamino)sulfonyl]phényl}-1,6-dihydropyrazolo[3,4-e]indazole-3-carboxamide ;
92. 1-{4-[(butylamino)sulfonyl]phényl}-1,6-dihydropyrazolo[3,4-e]indazole-3-carboxamide ;
93. 1-{4-[(diméthylamino) sulfonyl]phényl}-1,6-dihydropyrazolo[3,4-e]indazole-3-carboxamide ;
94. 1-{4-[(diprop-2-ynylamino)sulfonyl]phényl}-1,6-dihydropyrazolo[3,4-e]indazole-3-carboxamide ;
95. 1-[4-(anilinosulfonyl)phényl]-1,6-dihydropyrazolo[3,4-e]indazole-3-carboxamide ;

96. 1-(4-{[(2-hydroxypropyl)amino]sulfonyl}phényl)-1,6-dihydropyrazolo[3,4-e]indazole-3-carboxamide ;
97. 1-[4-(méthylsulfonyl)phényl]-1,6-dihydropyrazolo-[3,4-e]indazole-3-carboxamide;
98. 1,6-dihydropyrazolo[3,4-e]indazole-3-carboxamide ;
99. 1-phényl-1,6-dihydropyrazolo[3,4-e]indazole-3-carboxamide ;
100. 1-(4-fluorophényl)-1,6-dihydropyrazolo[3,4-e]-indazole-3-carboxamide ;
101. 1-(4-méthylphényl)-1,6-dihydropyrazolo[3,4-e]-indazole-3-carboxamide ;
102. 1-(4-cyanophényl)-1,6-dihydropyrazolo[3,4-e]indazole-3-carboxamide ;
103. 1-[4-trifluorométhyl)phényl]-1,6-dihydropyrazolo-[3,4-e]indazole-3-carboxamide ;
104. 1-(4-chlorophényl)-1,6-dihydropyrazolo[3,4-e]indazole-3-carboxamide ;
105. 1-(4-bromophényl)-1,6-dihydropyrazolo[3,4-e]indazole-3-carboxamide ;
106. 1-(4-nitrophényl)-1,6-dihydropyrazolo[3,4-e]indazole-3-carboxamide ;
107. 1-benzyl-1,6-dihydropyrazolo[3,4-e]indazole-3-carboxamide ;
108. 1-(3-hydroxybenzyl)-1,6-dihydropyrazolo[3,4-e]indazole-3-carboxamide ;
109. 1-pyridine-2-yl-1,6-dihydropyrazolo[3,4-e]indazole-3-carboxamide ;
110. 1-(3-chlorophényl)-1,6-dihydropyrazolo[3,4-e]indazole-3-carboxamide;
111. 1-(3-méthylphényl)-1,6-dihydropyrazolo[3,4-e]indazole-3-carboxamide ;
112. 1-(3-fluorophényl)-1,6-dihydropyrazolo[3,4-e]indazole-3-carboxamide;
113. 1-(6-chloropyridazine-3-yl)-1,6-dihydropyrazolo-[3,4-e]indazole-3-carboxamide ;
114. Acide 1-(4-méthoxyphényl)-1,6-dihydropyrazolo-[3,4-e]indazole-3-carboxylique ;
115. 1-phényl-1,6-dihydropyrazolo[3,4-e]indazole-3-carboxylate d'éthyle ;
116. 1-(4-méthoxyphényl)-1,6-dihydropyrazolo[3,4-e]-indazole-3-carboxylate d'éthyle ;
117. N-méthyl-1-[4-(aminosulfonyl)phényl]-1,6-dihydropyrazolo[3,4-e]indazole-3-carboxamide ;
118. N-méthyl-1-(4-[(butylamino) sulfonyl]phényl)-1,6-dihydropyrazolo[3,4-e]indazole-3-carboxamide ;
119. N-méthyl-1-{4-[(diméthylamino)sulfonyl]phényl}-1,6-dihydropyrazolo[3,4-e]indazole-3-carboxamide ;
120. N-méthyl-1-[4-(méthylsulfonyl)phényl]-1,6-dihydropyrazolo[3,4-e]indazole-3-carboxamide ;
121. N-(allyloxy)-1-{4-[(butylamino)sulfonyl]phényl}-1,6-dihydropyrazolo[3,4-e]indazole-3-carboxamide ;
122. 7,8,9,10-tétrahydro[1,4]diazépino[1,2-b]pyrazolo-[3,4-g]indazole-6(3H)-one ;
123. 1-pyridine-3-yl-1,6-dihydropyrazolo[3,4-e]indazole-3-carboxamide ;
124. 1-[4-(acétylamino)phényl]-1,6-dihydropyrazolo-[3,4-e]indazole-3-carboxamide;
125. Acide 4-[3-(aminocarbonyl)pyrazolo[3,4-e]indazole-1(6H)-yl]benzoïque;
126. 1-{4-[(4-méthylpipérazine-1-yl)sulfonyl]phényl}-1,6-dihydropyrazolo[3,4-e]indazole-3-carboxamide ;
127. 1-[4-(trifluorométhoxy)phényl]-1,6-dihydropyrazolo[3,4-e]indazole-3-carboxamide ;
128. Acide 4-[3-(éthoxycarbonyl)pyrazolo[3,4-e]indazole-1(6H)-yl]benzoïque ;
129. 1-(4-morpholine-4-ylphényl)-1,6-dihydropyrazolo[3,4-e]-indazole-3-carboxamide ;
130. 1-(2-hydroxyéthyl)-1,6-dihydropyrazolo[3,4-e]indazole-3-carboxamide ;
131. 1-(2,5-diméthylphényl)-1,6-dihydropyrazolo[3,4-e]-indazole-3-carboxamide ;
132. Chlorhydrate de 1-(2-aminoéthyl)-1,6-dihydropyrazolo[3,4-e]indazole-3-carboxamide ;
133. 1-(2,2,2-trifluorométhyl)-1,6-dihydropyrazolo[3,4-e]-indazole-3-carboxamide ;
134. 1-[4-(1H-imidazole-2-yl)phényl]-1,6-dihydropyrazolo[3,4-e]indazole-3-carboxamide ;
135. 1-méthyl-1,6-dihydropyrazolo[3,4-e]indazole-3-carboxamide ;
136. 8,9-dihydro-3H-pyrazino[1,2-b]pyrazolo[3,4-g]-indazole-6(7H)-one ;
137. Chlorhydrate de 2-(2-aminoéthyl)-2,6-dihydropyrazolo[3,4-e]indazole-3-carboxamide ;
138. 2-(2-hydroxyéthyl)-2,6-dihydropyrazolo[3,4-e]indazole-3-carboxamide ;
139. 2-méthyl-2,6-dihydropyrazolo[3,4-e] indazole-3-carboxamide ;
140. 1-anilino-8,9-dihydro-3H-pyrazino[1,2-b]-pyrazolo-[3,4-g]indazole-6(7H)-one ;
141. 1-(4-méthoxy-phényl)-1,6-dihydropyrazolo[3,4-e]-indazole-3-amine ;
142. 1-[1-(4-méthylphényl)-1,6-dihydropyrazolo[3,4-e]-indazole-3-yl]éthanone ;
143. 1-(4-méthoxyphényl)-1,6-dihydropyrazolo[3,4-e]-indazole-3-carbonitrile ;
144. 1-(4-méthoxyphényl)-1,6-dihydropyrazolo[3,4-e]-indazole-3-carbohydrazide ;
145. 1-(4-méthoxyphényl)-1,4,5,6-tétrahydropyrazolo-[3,4-e]indazole-3-carbohydrazide ;
146. N'-hydroxy-1-(4-méthoxyphényl)-1,6-dihydropyrazolo[3,4-e]indazole-3-carboximidamide ;
147. Acide 1-(4-méthoxyphényl)-1,6-dihydropyrazolo[3,4-e]-indazole-3-carboxylique;
148. Acide 1-(4-bromophényl)-1,4,5,6-tétrahydropyrazolo-[3,4-e]indazole-3-carboxylique ;
149. Acide 1-{4-[(butylamino)sulfonyl]phényl}-1,4,5,6-tétrahydropyrazolo[3,4-e]indazole-3-carboxylique;
150. Acide 4,4-diméthyl-1-(4-méthylphényl)-1,4,5,6-tétrahydropyrazolo[3,4-e]indazole-3-carboxylique ;
151. N-hydroxy-1-(4-méthoxyphényl)-1,6-dihydropyrazolo[3,4-e]-indazole-3-carboxamide ;
152. N-(allyloxy)-1-{4-[(butylamino)sulfonyl]phényl}-1,4,5,6-tétrahydropyrazolo[3,4-e]indazole-3-carboxamide ;

153. N-(allyloxy)-1-(4-méthoxyphényl)-1,4,5,6-tétrahydropyrazolo[3,4-e]indazole-3-carboxamide ;
154. N-méthyl-1-[4-(méthylsulfonyl)phényl]-1,4,5,6-tétrahydropyrazolo[3,4-e]indazole-3-carboxamide ;
155. 1-[4-(aminosulfonyl)phényl]-N-méthyl-1,4,5,6-tétrahydropyrazolo[3,4-e]indazole-3-carboxamide ;
156. 1-{4-[(butylamino)sulfonyl]phényl}-N-méthyl-1,4,5,6-tétrahydropyrazolo[3,4-e]indazole-3-carboxamide ;
157. 1-{4-[(diméthylamino)sulfonyl]phényl}-N-méthyl-1,4,5,6-tétrahydropyrazolo[3,4-e]indazole-3-carboxamide ;
158. Chlorhydrate de 2-(3-aminopropyl)-2,4,5,6-tétrahydropyrazolo[3,4-e]indazole-3-carboxylate d'éthyle ;
159. 4,5,7,8,9,10-hexahydro[1,4]diazépino[1,2-b]pyrazolo-[3,4-g]indazole-6(3H)one ;
160. 5,5-diméthyl-4,5,7,8,9,10-hexahydro[1,4]diazépino-[1,2-b]pyrazolo[3,4-g]indazole-6(3H)one ;
161. 5,5-diméthyl-4,5,8,9-tétrahydro-3H-pyrazino[1,2-b]-pyrazolo[3,4-g]indazole-6(7H)one ;
162. 4,5,8,9-tétrahydro-3H-pyrazino[1,2-b]-pyrazolo[3,4-g]-indazole-6(7H)one ;
163. 1-anilino-4,5,8,9-tétrahydro-3H-pyrazino[1,2-b]-pyrazolo[3,4-g]indazole-6(7H)one.

**13.** Procédé pour la préparation d'un composé de formule (Ic) telle que définie dans la revendication 9

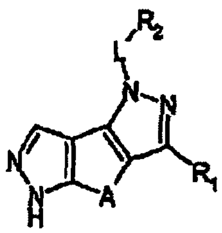

(Ic)

dans laquelle L et $R_2$ sont tels que définis dans la revendication 16, $R_1$ représente un groupe -COOEt ou -$CONH_2$, et A est choisi dans le groupe consistant en des groupes -$CH_2$-, -$CH_2$-$CH_2$-, -CH=CH- et -$CH_2$-$C(CH_3)_2$-, procédé qui comprend :

a) la réaction du composé (10) avec du dichlorhydrate d'hydrazine, de manière à obtenir le composé (11)

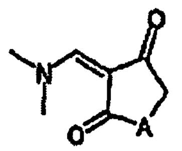

(10)

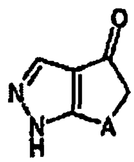

(11)

composé dans lequel A répond à la définition précitée, hormis un groupe -CH=CH- ;
b) la réaction du composé (11) avec du chlorure de trityle, de manière à obtenir le composé (12)

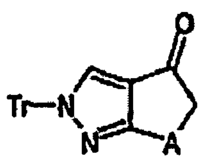

(12)

dans lequel Tr représente un groupe trityle, et la condensation de ce composé avec du chlorure d'oxalyle de manière à obtenir le composé (13)

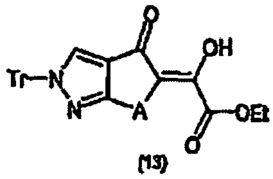

(13)

c) la réaction du composé (13) avec une hydrazine substituée (8)

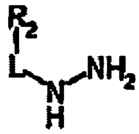

(8)

dans laquelle L et $R_2$ sont tels que définis dans la revendication 16, de manière à obtenir un composé de formule (Ic) dans laquelle $R_1$ représente un groupe -COOEt et A répond à la définition précitée, hormis le groupe -CH=CH- ; et, facultativement

d) la réaction de ce dernier composé avec de l'hydroxyde d'ammonium de manière à obtenir le dérivé correspondant de formule (Ic) dans laquelle $R_1$ représente un groupe $-CONH_2$; et, facultativement

e) la réaction du composé de formule (Ic) dans laquelle A représente un groupe $-CH_2-CH_2-$, obtenu dans l'étape c) ou d), avec un agent oxydant convenable de manière à obtenir le dérivé correspondant de formule (Ic) dans laquelle A représente un groupe -CH=CH-.

**14.** Procédé suivant la revendication 13, dans lequel, dans l'étape e), l'agent oxydant est la 2,3-dichloro-5,6-dicyano-1,4-benzoquinone.

**15.** Composition pharmaceutique comprenant une quantité efficace d'un composé de formule (I) telle que définie dans la revendication 9 et, au moins, un excipient, support ou diluant pharmaceutiquement acceptable.

**16.** Composition pharmaceutique suivant la revendication 15, comprenant en outre un ou plusieurs agents chimiothérapeutiques, sous forme d'une préparation combinée pour une utilisation simultanée, séparée ou séquentielle en thérapie anticancéreuse.

**17.** Produit ou kit comprenant un composé de la revendication 9 ou une composition pharmaceutique le contenant, répondant à la définition figurant dans la revendication 15, et un ou plusieurs agents chimiothérapeutiques, sous forme d'une préparation combinée pour une utilisation simultanée, séparée ou séquentielle en thérapie anticancéreuse.

**18.** Utilisation d'un composé de formule (Ic), (Ie), (If) ou (Ig) ou d'un de ses sels pharmaceutiquement acceptables, répondant à la définition figurant dans la revendication 9, dans la production d'un médicament destiné au traitement de maladies provoquées par, et/ou associées à, une activité de protéine-kinase modifiée.

**19.** Utilisation suivant la revendication 1, pour le traitement de tumeurs.